(19) Europäisches Patentamt
European Patent Office
Office européen des brevets

(11) EP 4 353 231 A1

(12) EUROPEAN PATENT APPLICATION
published in accordance with Art. 153(4) EPC

(43) Date of publication:
17.04.2024 Bulletin 2024/16

(21) Application number: 22819599.6

(22) Date of filing: 09.06.2022

(51) International Patent Classification (IPC):
*A61K 31/495 (2006.01)* *A61K 31/519 (2006.01)*
*C07D 487/14 (2006.01)* *A61P 1/00 (2006.01)*
*A61P 35/00 (2006.01)*

(52) Cooperative Patent Classification (CPC):
**A61K 31/495; A61K 31/519; A61P 1/00; A61P 35/00; C07D 487/14**

(86) International application number:
**PCT/CN2022/097793**

(87) International publication number:
**WO 2022/258002 (15.12.2022 Gazette 2022/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.06.2021 CN 202110649083**

(71) Applicant: **OnQuality Pharmaceuticals China Ltd.**
**Baoshan District**
**Shanghai 200431 (CN)**

(72) Inventors:
- **LI, Wenxi**
  **Shanghai 201112 (CN)**
- **LIU, Shilan**
  **Shanghai 201112 (CN)**
- **WU, Zhaoyu**
  **Shanghai 201112 (CN)**
- **ZHANG, Shiyi**
  **Shanghai 201112 (CN)**

(74) Representative: **AWA Denmark A/S**
**Strandgade 56**
**1401 Copenhagen K (DK)**

# (54) COMPOUND AND METHOD FOR TREATING CHEMOTHERAPY-ASSOCIATED GASTROINTESTINAL SIDE EFFECTS

(57) A compound and method for treating chemotherapy-associated gastrointestinal side effects. The present application relates to a use of a compound represented by Formula I or a pharmaceutically acceptable salt thereof in the preparation of a medicament,

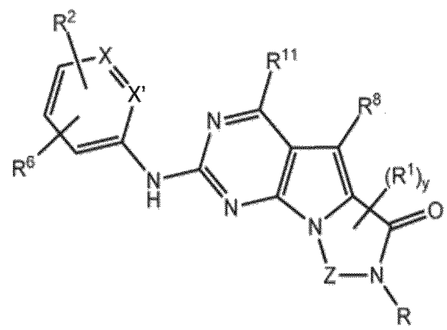

Formula I,

the medicament being used for preventing, alleviating and/or treating gastrointestinal side effects associated with chemotherapy in a subject. Also provided a method for using the compound to treat chemotherapy-associated gastrointestinal side effects.

EP 4 353 231 A1

## Description

## TECHNICAL FIELD

The present application relates to the field of biomedicine, and particularly to a method of preventing, relieving and/or treating chemotherapy-associated gastrointestinal side effects.

## BACKGROUND

Tumor chemotherapy is one of the most commonly used means for treating tumors clinically, which mainly utilizes a chemical medicament (a chemotherapeutic agent) to prevent the proliferation, infiltration, and metastasis of cancer cells, eventually killing cancer cells. However, the administration of a chemotherapeutic agent can not only kill rapidly growing and isolated tumor cells, it may also kill normal cells and immune cells at the same time, thus causing severe side effects, including nausea, vomiting, anemia, inflammation, etc. Such side effects may result in withdrawal of the chemotherapeutic agent or dosage reduction, greatly affecting the therapeutic effect on the tumor of patients, and impairing the quality of life for patients, and endanging the patient's life in severe cases.

No successful therapeutic regimen has been available yet in the prior art for controlling the side effects associated with the administration of a chemotherapeutic agent. Therefore, there is an urgent need for therapeutic regimens that can successfully control these side effects.

## SUMMARY OF THE INVENTION

The present application provides a method of preventing, relieving and/or treating chemotherapy-associated gastrointestinal side effects (e.g., diarrhea, enteritis or constipation) in a subject, comprising administering a compound as shown in Formula I or a pharmaceutically acceptable salt thereof to the subject. The compound as shown in Formula I of the present application or the pharmaceutically acceptable salt thereof is capable of effectively preventing, relieving and/or treating chemotherapy-associated gastrointestinal side effects.

In one aspect, the present application provides a use of a compound as shown in Formula I or a pharmaceutically acceptable salt thereof in preparing a medicament, which is used for preventing, relieving and/or treating chemotherapy-associated gastrointestinal side effects in a subj ect:

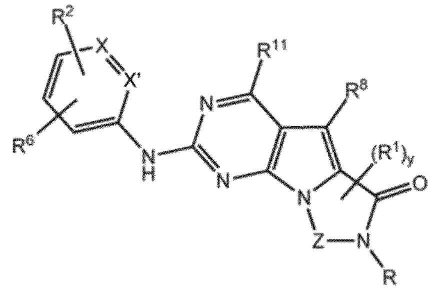

Formula I,

wherein,

- Z is $-(CH_2)_X-$, wherein X is 1, 2, 3 or 4, or $-O-(CH_2)_Z-$, wherein z is 2, 3 or 4;
- X and X' are each independently CH or N;
- R, $R^8$ and $R^{11}$ are each independently H, $C_1$-$C_3$ alkyl or haloalkyl, cycloalkyl or cycloalkyl containing one or more heteroatoms selected from N, O or S, -(alkylene)$_m$-$C_3$-$C_8$ cycloalkyl, -(alkylene)$_m$-aryl, -(alkylene)$_m$-heterocyclyl, -(alkylene)$_m$-heteroaryl, -(alkylene)$_m$-$NR^3R^4$, -(alkylene)$_m$-C(O)-$NR^3R^4$, -(alkylene)$_m$-O-$R^5$, -(alkylene)$_m$-$S(O)_n$-$R^5$, or -(alkylene)$_m$-$S(O)_n$-$NR^3R^4$, any of which may be optionally independently substituted with one or more R groups as allowed by valance, and wherein two $R^x$ groups bound to the same or adjacent atoms may optionally combine to form a ring, and wherein m is 0 or 1, n is 0, 1 or 2;
- $R^1$ is independently aryl, alkyl, cycloalkyl or haloalkyl, wherein each of the alkyl, cycloalkyl and haloalkyl optionally comprises O or N heteroatoms in place of a carbon in the chain and two $R^1$'s on adjacent ring atoms or on the same ring atom together with the ring atom(s) to which they are attached optionally form a 3-8-membered cycle;
- y is 0, 1, 2, 3 or 4;
- $R^2$ is -(alkylene)$_m$-heterocyclyl, -(alkylene)$_m$-heteroaryl, -(alkylene)$_m$-$NR^3R^4$, -(alkylene)$_m$-C(O)-$NR^3R^4$, -(alkylene)$_m$-C(O)-O-alkyl; -(alkylene)$_m$-O-$R^5$, -(alkylene)$_m$-$S(O)_n$-$R^5$ or -(alkylene)$_m$-$S(O)_n$-$NR^3R^4$, any of which may

be optionally independently substituted with one or more $R^x$ groups as allowed by valance, and wherein two $R^x$ groups bound to the same or adjacent atoms may optionally combine to form a ring, and wherein m is 0 or 1, n is 0, 1 or 2;

$R^3$ and $R^4$ at each occurrence are independently:

(i) hydrogen or

(ii) alkyl, cycloalkyl, heterocyclyl, aryl, heteroaryl, cycloalkylalkyl, heterocycloalkyl, arylalkyl or heteroarylalkyl, any of which may be optionally independently substituted with one or more $R^x$ groups as allowed by valance, and wherein two $R^x$ groups bound to the same or adjacent atoms may optionally combine to form a ring; or $R^3$ and $R^4$ together with the nitrogen atom to which they are attached may combine to form a heterocyclic ring optionally independently substituted with one or more $R^x$ groups as allowed by valance, and wherein two $R^x$ groups bound to the same or adjacent atoms may optionally combine to form a ring;

$R^5$ and $R^{5*}$ at each occurrence is:

(i) hydrogen or

(ii) alkyl, alkenyl, alkynyl, cycloalkyl, heterocyclyl, aryl, heteroaryl, cycloalkylalkyl, heterocycloalkyl, arylalkyl or heteroarylalkyl, any of which may be optionally independently substituted with one or more $R^x$ groups as allowed by valance;

$R^x$ at each occurrence is independently halo, cyano, nitro, oxo, alkyl, haloalkyl, alkenyl, alkynyl, cycloalkyl, cycloalkenyl, heterocyclyl, aryl, heteroaryl, arylalkyl, heteroarylalkyl, cycloalkylalkyl, heterocycloalkyl, -(alkylene)$_m$-OR$^5$, -(alkylene)$_m$-O-alkylene-OR$^5$, -(alkylene)$_m$-S(O)$_n$-R$^5$, -(alkylene)$_m$-NR$^3$R$^4$, -(alkylene)$_m$-CN, -(alkylene)$_m$-C(O)-R$^5$, -(alkylene)$_m$-C(S)-R$^5$, -(alkylene)$_m$-C(O)-OR$^5$, -(alkylene)$_m$-O-C(O)-R$^5$, -(alkylene)$_m$-C(S)-OR$^5$, -(alkylene)$_m$-C(O)-(alkylene)$_m$-NR$^3$R$^4$, -(alkylene)$_m$-C(S)-NR$^3$R$^4$, -(alkylene)$_m$-N(R$^3$)-C(O)-NR$^3$R$^4$, -(alkylene)$_m$-N(R$^3$)-C(S)-NR$^3$R$^4$, -(alkylene)$_m$-N(R$^3$)-C(O)-R$^5$, -(alkylene)$_m$-N(R$^3$)-C(S)-R$^5$, -(alkylene)$_m$-O-C(O)-NR$^3$R$^4$, -(alkylene)$_m$-O-C(S)-NR$^3$R$^4$, -(alkylene)$_m$-SO$_2$-NR$^3$R$^4$, -(alkylene)$_m$-N(R$^3$)-SO$_2$-R$^5$, -(alkylene)$_m$-N(R$^3$)-SO$_2$-NR$^3$R$^4$, -(alkylene)$_m$-N(R$^3$)-C(O)-OR$^5$, -(alkylene)$_m$-N(R$^3$)-C(S)-OR$^5$ or -(alkylene)$_m$-N(R$^3$)-SO$_2$-R$^5$; wherein:

the alkyl, haloalkyl, alkenyl, alkynyl, cycloalkyl, cycloalkenyl, heterocyclyl, aryl, heteroaryl, arylalkyl, heteroarylalkyl, cycloalkylalkyl and heterocycloalkyl may be further independently substituted with one or more of the following groups:

-(alkylene)$_m$-CN, -(alkylene)$_m$-OR$^{5*}$, -(alkylene)$_m$-S(O)$_n$-R$^{5*}$, -(alkylene)$_m$-NR$^{3*}$R$^{4*}$, -(alkylene)$_m$-C(O)-R$^{5*}$, -(alkylene)$_m$-C( = S)R$^{5*}$, -(alkylene)$_m$-C( = O)OR$^{5*}$, -(alkylene)$_m$-OC( = O)R$^{5*}$, -(alkylene)$_m$-C(S)-OR$^{5*}$, -(alkylene)$_m$-C(O)-NR$^{3*}$R$^{4*}$, -(alkylene)$_m$-C(S)-NR$^{3*}$R$^{4*}$, -(alkylene)$_m$-N(R$^{3*}$)-C(O)-NR$^{3*}$R$^{4*}$, -(alkylene)$_m$-N(R$^{3*}$)-C(S)-NR$^{3*}$R$^{4*}$, -(alkylene)$_m$-N(R$^{3*}$)-C(O)-R$^{5*}$, -(alkylene)$_m$-N(R$^{3*}$)-C(S)-R$^{5*}$, -(alkylene)$_m$-O-C(O)-NR$^{3*}$R$^{4*}$, -(alkylene)$_m$-O-C(S)-NR$^{3*}$R$^{4*}$, -(alkylene)$_m$-SO$_2$-NR$^{3*}$R$^{4*}$, -(alkylene)$_m$-N(R$^{3*}$)-SO$_2$-R$^{5*}$, -(alkylene)$_m$-N(R$^{3*}$)-SO$_2$-NR$^{3*}$R$^{4*}$, -(alkylene)$_m$-N(R$^{3*}$)-C(O)-OR$^{5*}$, -(alkylene)$_m$-N(R$^{3*}$)-C(S)-OR$^{5*}$ or -(alkylene)$_m$-N(R$^{3*}$)-SO$_2$-R$^{5*}$,

n is 0, 1 or 2, and m is 0 or 1;

$R^{3*}$ and $R^{4*}$ at each occurrence are independently:

(i) hydrogen or

(ii) alkyl, alkenyl, alkynyl, cycloalkyl, heterocyclyl, aryl, heteroaryl, cycloalkylalkyl, heterocycloalkyl, arylalkyl or heteroarylalkyl, any of which may be optionally independently substituted with one or more $R^x$ groups as allowed by valance; or $R^{3*}$ and $R^{4*}$ together with the nitrogen atom to which they are attached may combine to form a heterocyclic ring optionally independently substituted with one or more $R^x$ groups as allowed by valance; and

$R^6$ is H or lower alkyl, -(alkylene)$_m$-heterocyclyl, -(alkylene)$_m$-heteroaryl, -(alkylene)$_m$-NR$^3$R$^4$, -(alkylene)$_m$-C(O)-NR$^3$R$^4$; -(alkylene)$_m$-O-R$^5$, -(alkylene)$_m$-S(O)$_n$-R$^5$ or -(alkylene)$_m$-S(O)$_n$-NR$^3$R$^4$, any of which may be optionally independently substituted with one or more $R^x$ groups as allowed by valance, and wherein two $R^x$ groups bound to the same or adjacent atoms may optionally combine to form a ring;

or a pharmaceutically acceptable salt, prodrug, isotopic variant (e.g., completely or partially deuterated form) or solvate thereof.

In some embodiments, the pharmaceutically acceptable salt of the compound as shown in Formula I is hydrochloride.

In some embodiments, the compound has a structure of Formula I and $R^6$ is absent.

In some embodiments, the $R^x$ in the Formula I is not further substituted.

In some embodiments, the $R^8$ in the Formula I is hydrogen or $C_1$-$C_3$ alkyl.

In some embodiments, the $R^{11}$ in the Formula I is hydrogen or $C_1$-$C_3$ alkyl.

In some embodiments, the compound comprises a compound as shown in Formula Ia:

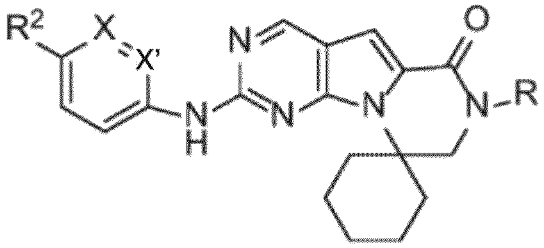

Formula Ia.

In some embodiments, the X in the Formula I or Formula Ia is C.

In some embodiments, the X' in the Formula I or Formula Ia is N.

In some embodiments, the R in the Formula I or Formula Ia is hydrogen or $C_1$-$C_3$ alkyl.

In some embodiments, the $R^2$ in the Formula I or Formula Ia is -(alkylene)$_m$-heterocyclyl, -(alkylene)$_m$-$NR^3R^4$, -(alkylene)$_m$-C(O)-$NR^3R^4$, -(alkylene)$_m$-C(O)-O-alkyl or -(alkylene)$_m$-$OR^5$, any of which may be optionally independently substituted with one or more $R^x$ groups as allowed by valance, and wherein two $R^x$ groups bound to the same or adjacent atoms may optionally combine to form a ring, and wherein m is 0 or 1.

In some embodiments, the $R^2$ in the Formula I or Formula Ia is -(alkylene)$_m$-heterocyclyl, -(alkylene)$_m$-$NR^3R^4$, -(alkylene)$_m$-C(O)-$NR^3R^4$, -(alkylene)$_m$-C(O)-O-alkyl or -(alkylene)$_m$-$OR^5$, which is not further substituted.

In some embodiments, m in the $R^2$ in the Formula I or Formula Ia is 1.

In some embodiments, alkylene in the $R^2$ in the Formula I or Formula Ia is methylene.

In some embodiments, the $R^2$ in the Formula I or Formula Ia is

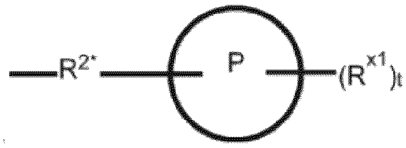

,

wherein, $R^{2*}$ is a bond, alkylene, -(alkylene)$_m$-O-(alkylene)$_m$-, -(alkylene)$_m$-C(O)-(alkylene)$_m$-, -(alkylene)$_m$-S(O)$_2$-(alkylene)$_m$- and -(alkylene)$_m$-NH-(alkylene)$_m$-, wherein each m is independently 0 or 1;

P is a 4- to 8-membered mono- or bicyclic saturated heterocyclyl group;

each $R^{x1}$ is independently -(alkylene)$_m$-(C(O))$_m$-(alkylene)$_m$-(N($R^N$))$_m$-(alkyl)$_m$, wherein each m is independently 0 or 1 provided at least one m is 1; -(C(O))-O-alkyl; -(alkylene)$_m$-cycloalkyl wherein m is 0 or 1; -N($R^N$)-cycloalkyl; -C(O)-cycloalkyl; -(alkylene)$_m$-heterocyclyl wherein m is 0 or 1; or -N($R^N$)-heterocyclyl; -C(O)-heterocyclyl; -S(O)$_2$-(alkylene)$_m$ wherein m is 1 or 2, wherein:

$R^N$ is H, $C_1$ to $C_4$ alkyl or $C_1$ to $C_6$ heteroalkyl, and

wherein two $R^{x1}$ can, together with the atoms to which they attach on P, which may be the same atom, form a ring; and

t is 0, 1 or 2.

In some embodiments, the each $R^{x1}$ in the $R^2$ is optionally substituted by unsubstituted alkyl, halogen or hydroxyl.

In some embodiments, $R^{x1}$ in the $R^2$ is hydrogen or unsubstituted $C_1$-$C_4$alkyl.

In some embodiments, the at least one $R^{x1}$ in the $R^2$ is -(alkylene)$_m$-heterocyclyl, wherein m is 0 or 1.

In some embodiments, the $R^2$ in the Formula I or Formula Ia is

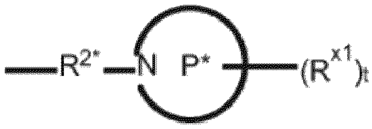,

wherein P* is a 4- to 8-membered mono- or bicyclic saturated heterocyclyl group.

In some embodiments, the $R^2$ in the Formula I or Formula Ia is

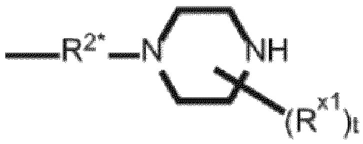.

In some embodiments, the $R^2$ in the Formula I or Formula Ia is

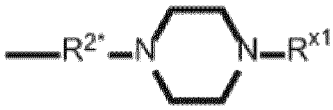.

In some embodiments, the $R^2$ in the Formula I or Formula Ia is

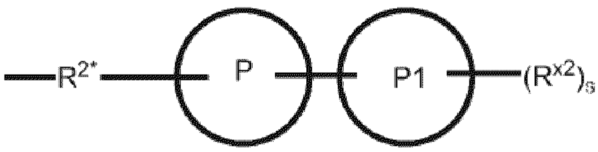,

wherein, $R^{2*}$ is a bond, alkylene, -(alkylene)$_m$-O-(alkylene)$_m$-, -(alkylene)$_m$-C(O)-(alkylene)$_m$-, -(alkylene)$_m$-S(O)$_2$-(alkylene)$_m$- and -(alkylene)$_m$-NH-(alkylene)$_m$-, wherein each m is independently 0 or 1;

P is a 4- to 8-membered mono- or bicyclic saturated heterocyclyl group;
P1 is a 4- to 6-membered monocyclic saturated heterocyclyl group; and
each $R^{X2}$ is independently hydrogen or alkyl; and s is 0, 1 or 2.

In some embodiments, the $R^2$ in the Formula I or Formula Ia is

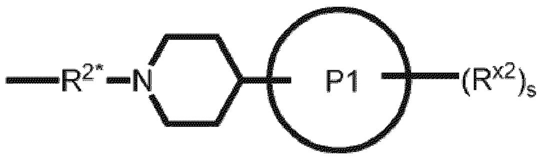

In some embodiments, P1 in the $R^2$ comprises at least one nitrogen.

In some embodiments, any alkylene in $R^{2*}$ of any one embodiment above is not further substituted.

In some embodiments, the $R^{2*}$ in the $R^2$ is a bond.

In some embodiments, the $R^{x1}$ in the $R^2$ is hydrogen, methyl or propyl.

In some embodiments, the $R^2$ is

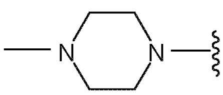, 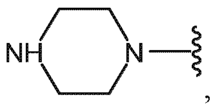, 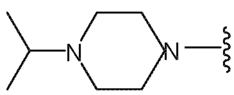

or

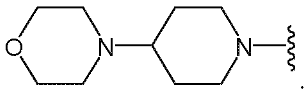

In some embodiments, the compound is selected from the following group:

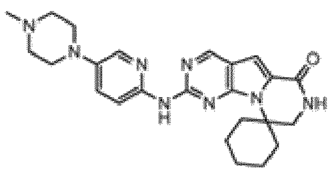

Compound I

,

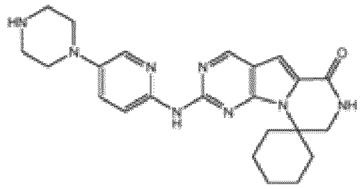

Compound II

,

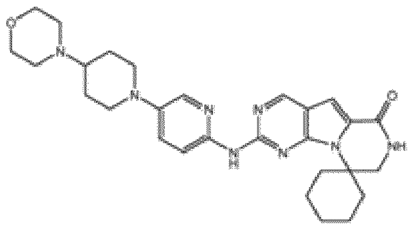

Compound III

and

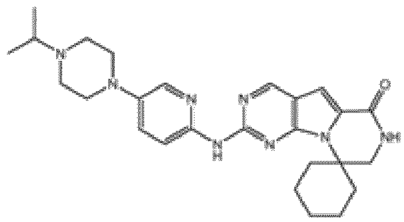

Compound IV

.

In some embodiments, the compound is

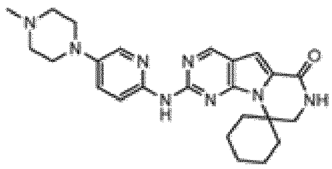

Compound I

,

and its hydrochloride form.

In some embodiments, the compound is

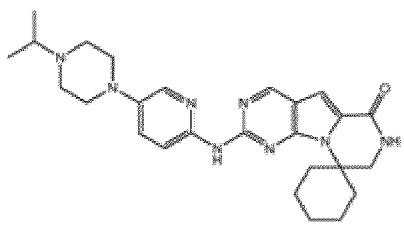

Compound IV

, and its hydrochloride form.

In some embodiments, the chemotherapy comprises administration of a chemotherapeutic agent.

In some embodiments, the chemotherapeutic agent is a cytotoxic agent.

In some embodiments, the chemotherapeutic agent is selected from one or more of the following group: a DNA synthesis inhibitor, an RNA synthesis inhibitor, a protein synthesis inhibitor, a cell division inhibitor, a DNA base analogue, a topoisomerase inhibitor and/or a telomerase synthesis inhibitor.

In some embodiments, the chemotherapy is administered in combination with one or more other therapies.

In some embodiments, the chemotherapeutic agent is selected from fluorouracil, oxaliplatin, irinotecan, topotecan, etoposide, cisplatin, docetaxel, gemcitabine, carboplatin, methotrexate, doxorubicin, cytarabine, vinorelbine and capecitabine, and a combination thereof.

In some embodiments, the chemotherapeutic agent is selected from 5-fluorouracil, irinotecan and oxaliplatin, and a combination thereof.

In some embodiments, the chemotherapy-associated gastrointestinal side effects comprise gastrointestinal side effects caused by the chemotherapeutic agent.

In some embodiments, the chemotherapy-associated gastrointestinal side effects comprise gastrointestinal adverse events that occur or deteriorate after the administration of the chemotherapeutic agent.

In some embodiments, the gastrointestinal adverse events will occur or deteriorate about 1 hr, about 2 hrs, about 3 hrs, about 4 hrs, about 5 hrs, about 6 hrs, about 7 hrs, about 8 hrs, about 9 hrs, about 10 hrs, about 11 hrs, about 12 hrs, about 1 day, about 2 days, about 4 days, about 7 days, about 2 weeks, about 3 weeks, about 1 month, about 2 months or longer after the administration of the chemotherapeutic agent, in the absence of prevention or treatment.

In some embodiments, the gastrointestinal side effects comprise gastric mucosal injury diseases and/or intestinal mucosal injury diseases.

In some embodiments, the gastrointestinal side effects comprise diarrhea, abdominal pain, nausea, vomiting, mucositis, loss of appetite, gastric ulcer, gastritis, constipation, enteritis, intestinal perforation, intestinal bleeding, ulcer and/or intestinal necrosis.

In some embodiments, the gastrointestinal side effects comprise diarrhea, constipation and/or enteritis.

In some embodiments, the severity of the gastrointestinal side effects is Grade 1 or above, Grade 2 or above, Grade 3 or above, Grade 4 or above, or Grade 5, as evaluated in accordance with NCI-CTCAE.

In some embodiments, the subject comprises a cancer patient.

In some embodiments, the medicament does not substantially affect the therapeutic effect of the chemotherapeutic agent.

In some embodiments, the medicament is administered about 0.5 hrs, about 1 hr, about 2 hrs, about 3 hrs, about 4 hrs, about 5 hrs, about 6 hrs, about 7 hrs, about 8 hrs, about 9 hrs, about 10 hrs, about 11 hrs, about 12 hrs, about 13 hrs, about 14 hrs, about 15 hrs, about 16 hrs, about 17 hrs, about 18 hrs, about 19 hrs, about 20 hrs or longer before the administration of the chemotherapy.

In some embodiments, the medicament is administered about 0.5~12 hrs before the administration of the chemotherapy.

In some embodiments, the medicament is prepared for oral administration.

In some embodiments, the medicament is prepared for injection administration.

In some embodiments, the medicament is prepared for administration through subcutaneous injection, intramuscular injection, intraperitoneal injection and/or intravenous injection.

In some embodiments, the medicament further comprises one or more other active ingredients.

In another aspect, the present application provides a method of preventing, relieving and/or treating chemotherapy-associated gastrointestinal side effects, comprising administering to a subject in need thereof the compound as shown in Formula I or a pharmaceutically acceptable salt thereof.

In another aspect, the present application provides the compound as shown in Formula I or a pharmaceutically acceptable salt thereof, which is used for preventing, relieving and/or treating chemotherapy-associated gastrointestinal side effects in a subject.

In another aspect, the present application provides a pharmaceutical combination, comprising the compound as shown in Formula I or a pharmaceutically acceptable salt thereof and a chemotherapeutic agent.

In another aspect, the present application provides a kit, comprising the compound as shown in Formula I or a pharmaceutically acceptable salt thereof and a chemotherapeutic agent.

Other aspects and advantages of the present application will be readily apparent to those skilled in the art from the following detailed description. Only the exemplary embodiments of the present application are shown and described in the following detailed description. As will be appreciated by those skilled in the art, the disclosure of the present application allows persons skilled in the art to modify the disclosed embodiments without departing from the spirit and scope of the invention involved in the present application. Accordingly, the drawings and the description in the specification of the present application are merely exemplary, and not restrictive.

## BRIEF DESCRIPTION OF THE DRAWINGS

Specific features of the invention involved in the present application are set forth in the appended claims. The features and advantages of the invention involved in the present application can be better understood by referring to the exemplary embodiments detailed hereinafter and the accompanying drawings. A brief description of the drawings

is as follows:

FIG. 1 shows the diarrhea photographs of typical mice in the chemotherapeutic agent groups of examples 1-201.
FIG. 2 shows partial results of diarrhea grading in the control group, the chemotherapeutic agent group, and the group of the compound as shown in Formula I of examples 1-201.
FIG. 3 shows the photographs showing the number and shape of faeces in the control group, the chemotherapeutic agent group, and the group of the compound as shown in Formula I of examples 202-364.
FIG. 4 shows partial results of feces reduction rate in the chemotherapeutic agent group, and the group of the compound as shown in Formula I of examples 202-364.
FIG. 5 shows partial results of diarrhea grading in the control group, the chemotherapeutic agent group, the group of the compound as shown in Formula I of examples 365-384.
FIG. 6 shows partial results of feces reduction rate in the control group, and the group of the compound as shown in Formula I of examples 385-405.

## DETAILED DESCRIPTION

The embodiments of the present invention are described below by way of specific examples, and persons skilled in the art can readily appreciate other advantages and effects of the present invention from the disclosure of the present specification.

### Term Definition

In the present application, the term "alkyl", either alone or within other terms such as "haloalkyl" and "alkylamino", generally refers to linear or branched radicals having one to about twelve carbon atoms. "Lower alkyl" radicals generally have one to about six carbon atoms. Examples of such radicals include methyl, ethyl, n-propyl, isopropyl, n-butyl, isobutyl, sec-butyl, tert-butyl, pentyl, isopentyl, hexyl, and the like. The term "alkylene" embraces bridging divalent linear or branched alkyl radicals. Examples may include methylene, ethylene, propylene, isopropylene and the like.

In the present application, the term "alkenyl" generally refers to linear or branched radicals having at least one carbon-carbon double bond and two to about twelve carbon atoms. "Lower alkenyl" radicals have two to about six carbon atoms. Examples of alkenyl radicals include vinyl, propenyl, allyl, propenyl, butenyl and 4-methylbutenyl. The terms "alkenyl" and "lower alkenyl" embrace radicals having "cis" and "trans" orientations or alternatively, "E" and "Z" orientations.

In the present application, the term "alkynyl" generally refers to linear or branched radicals having at least one carbon-carbon triple bond and having two to about twelve carbon atoms. "Lower alkynyl" radicals have two to about six carbon atoms. Examples of such radicals include propargyl, butynyl, and the like. Alkyl, alkenyl and alkynyl radicals may be optionally substituted with one or more functional groups such as halo, hydroxyl, nitro, amino, cyano, haloalkyl, aryl, heteroaryl, heterocyclyl and the like.

In the present application, the term "alkylamino" generally embraces "N-alkylamino" and "N,N-dialkylamino", where amino groups are independently substituted with one alkyl radical and two alkyl radicals, respectively. "Lower alkylamino" radicals generally have one or two alkyl radicals of one to six carbon atoms attached to a nitrogen atom. Suitable alkylamino radicals may be monoalkylamino or dialkylamino, such as N-methylamino, N-ethylamino, N,N-dimethylamino or N,N-diethylamino.

In the present application, the term "halo" generally refers to halogen, such as fluorine, chlorine, bromine or iodine atoms.

In the present application, the term "haloalkyl" generally refers to radicals wherein any one or more of the alkyl carbon atoms is substituted with one or more halo. Examples may include monohaloalkyl, dihaloalkyl and plyhaloalkyl radicals including perhaloalkyl. For example, a monohaloalkyl radical may have an iodo, bromo, chloro or fluoro atom within the radical. Dihaloalkyl and polyhaloalkyl radicals may have two or more of the same halo atoms or a combination of different halo radicals. "Lower haloalkyl" may embrace radicals having 1-6 carbon atoms. Examples of haloalkyl radicals may include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl. "Perfluoroalkyl" generally refers to an alkyl radical having all hydrogen atoms replaced with fluoro atoms. Examples may include trifluoromethyl and pentafluoroethyl.

In the present application, the term "aryl", alone or in combination with other terms, generally refers to a carbocyclic aromatic system containing one or two rings, wherein such rings may be attached together in a fused manner. The term "aryl" generally refers to aromatic radicals, such as phenyl, naphthyl, indenyl, tetrahydronaphthyl and indanyl. More preferred aryl is phenyl. Said "aryl" group may have 1 or more substituents, such as lower alkyl, hydroxyl, halo, haloalkyl, nitro, amino, alkoxy, lower alkylamino and the like. An aryl group may be optionally substituted with one or

more functional groups such as halo, hydroxyl, nitro, amino, cyano, haloalkyl, aryl, heteroaryl, heterocyclyl and the like.

In the present application, the term "heterocyclyl" (or "heterocyclic ring") generally refers to saturated and partially saturated heteroatom-containing ring radicals, where the heteroatoms may be selected from nitrogen, sulfur and oxygen. Heterocyclic rings comprise monocyclc 6-8-membered rings and 5-16-membered bicyclic ring systems (which can include bridged fused and spiro-fused bicyclic ring systems). It does not include rings containing -O-O-, -O-S- or -S-S- portions. Said "heterocyclyl" radical may have 1 to 3 substituents such as hydroxyl, Boc, halo, haloalkyl, amino, lower alkyl, lower aralkyl, oxo, lower alkoxy, amino, lower alkylamino and the like.

Examples of saturated heterocyclyl radicals include saturated 3- to 6-membered heteromonocyclic groups containing1 to 4 nitrogen atoms (e.g., pyrrolidinyl, imidazolidinyl, piperidinyl, pyrrolinyl, piperazinyl); saturated 3- to 6-membered heteromonocyclic groups containing1 to 2 oxygen atoms and 1 to 3 nitrogen atoms (e.g., morpholinyl); saturated 3 to 6-membered heteromonocyclic groups containing 1 to 2 sulfur atoms and 1 to 3 nitrogen atoms (e.g., thiazolidinyl). Examples of partially saturated heterocyclyl radicals include dihydrothienyl, dihydropyranyl, dihydrofuryl, dihydrothiazolyl, and the like.

Particular Examples of partially saturated and saturated heterocyclyl radicals may include pyrrolidinyl, imidazolidinyl, piperidinyl, pyrrolinyl, pyrazolidinyl, piperazinyl, morpholinyl, tetrahydropyranyl, thiazolidinyl, dihydrothienyl, 2,3-dihydro-benzo[1,4]dioxanyl, indolinyl, isoindolinyl, dihydrobenzothienyl, dihydrobenzofuryl, isochromanyl, chromanyl, 1,2-dihydroquinolyl, 1,2,3,4-tetrahydro-isoquinolyl, 1,2,3,4-tetrahydro-quinolyl, 2,3,4,4a,9,9a-hexahydro-1H-3-aza-fluorenyl, 5,6,7-trihydro-1,2,4-triazolo[3,4-a]isoquinolyl, 3,4-dihydro-2H-benzo[1,4]oxazinyl, benzo[1,4]dioxanyl, 2,3-dihydro-1H-λ'benzo[d]isothiazol-6-yl, dihydropyranyl, dihydrofuryl and dihydrothiazolyl and the like.

Heterocyclo groups also include radicals where heterocyclic radicals are fused/condensed with aryl radicals: condensed heterocyclic groups containing 1 to 5 nitrogen atoms, e.g., indolyl, isoindolyl, indolizinyl, benzimidazolyl, quinolyl, isoquinolyl, indazolyl, benzotrizaolyl, tetrahydropyridazinyl (e.g., tetrazolo[1,5-b]pyridazinyl); unsaturated condensed heterocyclic groups containing 1 to 2 oxygen atoms and 1 to 3 nitrogen atoms (e.g., benzoxazolyl, benzoxadiazolyl); unsaturated condensed heterocyclic groups containing 1 to 2 sulfur atoms and 1 to 3 nitrogen atoms (e.g., benzothiazolyl, benzothiadiazolyl); and saturated, partially unsaturated and unsaturated condensed heterocyclic groups containing 1 to 2 oxygen or sulfur atoms (e.g., benzofuryl, benzothienyl, 2,3-dihydrobenzo[1,4]dioxanyl and dihydrobenzofuryl).

In the present application, the term "heteroaryl" generally refers to aryl ring systems containing one or more heteroatoms selected from the group consisting of O, N and S, wherein the ring nitrogen and sulfur atom(s) are optionally oxidized, and nitrogen atom(s) are optionally quarternized. Examples include unsaturated 5- to 6-membered heteromonocyclic groups containing 1 to 4 nitrogen atoms, e.g., pyrrolyl, imidazolyl, pyrazolyl, 2-pyridyl, 3-pyridyl, 4-pyridyl, pyrimidyl, pyrazinyl, pyridazinyl, triazolyl (e.g., 4H-1,2,4-triazolyl, 1H-1,2,3-triazolyl, 2H-1,2,3-triazolyl); unsaturated 5- to 6-membered heteromonocyclic groups containing an oxygen atom, e.g., pyranyl, 2-furyl, 3-furyl, etc.; unsaturated 5- to 6-membered heteromonocyclic groups containing a sulfur atom, e.g., 2-thienyl, 3-thienyl, etc.; unsaturated 5- to 6-membered heteromonocyclic groups containing 1 to 2 oxygen atoms and 1 to 3 nitrogen atoms, e.g., oxazolyl, isoxazolyl, oxadiazolyl (e.g., 1,2,4-oxadiazolyl, 1,3,4-oxadiazolyl, 1,2,5-oxadiazolyl); unsaturated 5- to 6-membered heteromonocyclic groups containing 1 to 2 sulfur atoms and 1 to 3 nitrogen atoms, e.g., thiazolyl, thiadiazolyl (e.g., 1,2,4-thiadiazolyl, 1,3,4-thiadiazolyl, 1,2,5-thiazdiaolyl).

In the present application, the term "heteroarylalkyl" generally refers to alkyl radicals substituted with a heteroaryl group. Examples include pyridylmethyl and thienylethyl.

In the present application, the term "sulfonyl", whether used alone or together with other terms such as alkylsulfonyl, denotes divalent radicals $-SO_2-$ respectively.

In the present application, the term "carboxy" or "carboxyl", whether used alone or together with other terms such as "carboxyalkyl", denotes -C(O)-OH.

In the present application, the term "carbonyl", whether used alone or together with other terms such as "aminocarbonyl", denotes -C(O)-.

In the present application, the term "aminocarbonyl" generally refers to an amido group of Formula $-C(O)-NH_2$.

In the present application, the term "heterocycloalkyl" generally refers to heterocyclic-substituted alkyl radicals. Examples include piperidinylmethyl and morpholinylethyl.

In the present application, the term "arylalkyl" generally refers to aryl-substituted alkyl radicals. Examples include benzyl, diphenylmethyl and phenylethyl. The aryl in said arylalkyl may be additionally substituted with halo, alkyl, alkoxy, haloalkyl and haloalkoxy.

In the present application, the term "cycloalkyl" generally refers to saturated carbocyclic groups of 3 to 10 carbons. Lower cycloalkyl groups include $C_3$-$C_6$ rings. Examples include cyclopentyl, cyclopropyl and cyclohexyl. Cycloalkyl groups may be optionally substituted with one or more functional groups such as halo, hydroxyl, nitro, amino, cyano, haloalkyl, aryl, heteroaryl, heterocyclyl and the like.

In the present application, the term "cycloalkylalkyl" generally refers to cycloalkyl-substituted alkyl radicals. "Lower cycloalkylalkyl" radicals are cycloalkyl radicals attached to alkyl radicals having one to six carbon atoms. Examples

include cyclohexylmethyl. The cycloalkyl in said radicals may be additionally substituted with halo, alkyl, alkoxy and hydroxyl.

In the present application, the term "cycloalkenyl" includes carbocyclic groups having one or more carbon-carbon double bonds, including "cycloalkyldienyl" compounds. Examples include cyclopentenyl, cyclopentadienyl, cyclohexenyl and cycloheptadienyl.

In the present application, the term "oxo" generally refers to an oxygen atom attached with a double bond.

In the present application, the term "nitro" generally refers to $-NO_2$.

In the present application, the term "cyano" generally refers to -CN.

In the present application, the term "prodrug" generally refers to a compound which when administered to a host in vivo is converted into the parent drug. As used herein, the term "parent drug" generally refers to any of the presently described chemical compounds that are useful to treat any of the disorders described herein, or to control or improve the underlying cause or symptoms associated with any physiological or pathological disorder described herein in a host (e.g., a human). Prodrugs can be used to achieve any desired effect, including to enhance properties of the parent drug or to improve the pharmaceutic or pharmacokinetic properties of the parent drug. Prodrug strategies exist which provide choices in modulating the conditions for in vivo generation of the parent drug, all of which are deemed included herein. Nonlimiting examples of prodrug strategies include the following covalent attachments: removable groups or removable portions of groups, for example, but not limited to acylation, phosphorylation, phosphonylation, phosphoramidate derivatives, amidation, reduction, oxidation, esterification, alkylation, other carboxy derivatives, sulfoxy or sulfone derivatives, carbonylation or anhydride, among others.

In the present application, the term "chemotherapy" generally refers to a therapy that uses a chemotherapeutic agent to treat tumors, which can lead to the death of cancer cells, or interfere with the division, repair, growth and/or function of cancer cells. The chemotherapeutic agent comprises a chemical or biological substance which can cause the death of cancer cells or interfere with the growth, division, repair and/or function of cancer cells. For example, the chemotherapeutic agent may comprise cytotoxic agens, cell inhibitors and antitumor agents which can kill tumor cells, inhibit the growth or metastasis of tumor cells or disrupt the cell cycle of rapidly proliferating cells. The chemotherapeutic agent comprises natural compounds found in animals and plants, or artificial chemical substances. In the present application, the chemotherapeutic agents have low selectivity on tumor cells and normal cells, so they may also interfere with the growth, division, repair and/or function of normal cells (e.g., bone marrow cells, hair follicle cells or digestive tract cells), finally resulting in the death of normal cells. The chemotherapeutic agents in the present application may not include tumor-targeted medicaments, i.e., the chemotherapeutic agents in the present application do not include substances which can specifically recognize tumor cells, tumor tissues, tumor organs or proteins specifically expressed by tumor cells (e.g., tumor-associated antigens or tumor specific antigens). Because of genetic mutation or other factors, some kinases or phosphoric acid kinases are activated, causing the reproduction of cancer cells. The chemotherapeutic agents of the present application do not include those kinase inhibitors which prevent the division of cancer cells by inhibiting abnormally activated kinases, either. The chemotherapeutic agents of the present application do not include antibodies and/or angiogenesis inhibitors, either.

In the present application, examples of chemotherapy may include, but not limited to, alkylating agents, e.g., mechlorethamine, ethyleneimine compounds, alkyl sulfonates and other compounds having alkylating effects, e.g. nitrosourea, cisplatin and dacarbazine; antimetabolites, e.g., folic acid, purine or pyrimidine antagonists; mitotic inhibitors, e.g. vinca alkaloid and podophyllotoxin derivatives; cytotoxic antibiotics and camptothecin derivatives. The chemotherapy may also include amifostine cisplatin, dacarbazine (DTIC), dactinomycin, streptomycin, cyclophosphamide, carmustine (BCNU), lomustine (CCNU), doxorubicin, doxorubicin liposome, gemcitabine, erythromycin, daunorubicin liposome procarbazine, mitomycin, cytarabine, etoposide, methotrexate, 5-fluorouracil (5-FU), vinblastine, vincristine, bleomycin, paclitaxel, Docetaxel, Aldesleukin, Asparaginase, busulfan, carboplatin, cladribine, camptothecin, irinotecan, 10-hydroxyl-7-ethyl-camptothecin (SN38), floxuridine, fludarabine, hydroxyurea, ifosfamide, idarubicin, Mesna, interferon $\alpha$, interferon $\beta$, mitoxantrone, topotecan, Lupron, megestrol, Melphalan, mercaptopurine, plicamycin, mitotane, Oncaspar, pegaspargase, pentostatin, pipobroman, plicamycin, streptozocin, tamoxifen, teniposide, testosterone, thioguanine, thiotepa, uramustine, vinorelbine, chlorambucil aromatase inhibitor and a combination thereof.

In the present application, the term "cytotoxic agent" generally refers to a reagent for inhibiting the biological process of cells or reducing the viability or proliferative potential of cells. The cytotoxic agent may act in various ways, for example, but not limited to, through inducing DNA damages, inducing cell cycle arrest, inhibiting DNA synthesis, inhibiting transcription, inhibiting translation or protein synthesis, inhibiting cell division or inducing apoptosis.

In the present application, the term "continuous administration" generally refers to repeated administration of the same medicament every day. Repeated administration every day may be once a day, twice a day, 3 or more times a day. For example, continuous administration may be administration once a day for at least more than 2 days. For example, a continuous administration time of 2 days may mean administration once a day (twice, 3 or more times) continuously for 2 days. For example, a continuous administration time of 3 days may mean administration once a day (twice, 3 or more times) continuously for 3 days. For example, a continuous administration time of 5 days may mean

administration once a day (twice, 3 or more times) continuously for 5 days. For example, a continuous administration time of 7 days may mean administration once a day (twice, 3 or more times) continuously for 7 days. For example, a continuous administration time of 10 days may mean administration once a day (twice, 3 or more times) continuously for 10 days. For example, a continuous administration time of 14 days may mean administration once a day (twice, 3 or more times) continuously for 14 days. For example, a continuous administration time of more than 7 days may mean administration once a day (twice, 3 or more times) continuously for more than 7 days.

In the present application, the term "noncontinuous administration" generally refers to an administration mode in which the same medicament of the same type administered repeatedly is not administered every day in an administration cycle, also known as intermittent administration and/or pulsed administration. Noncontinuous administration may be regular or irregular. In noncontinuous administration, in an administration cycle, for any continuous period of time, if the administration is performed every day and continuously, it also belongs to the case of continuous administration in this period of time.

In the present application, the term "does not substantially affect" generally refers to that as compared to the therapeutic effect of using the chemotherapy alone, the use of the combination of the medicament and the chemotherapy of the present application has a comparable therapeutic effect, or does not produce a significant disadvantage. For example, for any subject, as compared to the therapeutic effect of using the chemotherapy alone, the use of the combination of the medicament and the chemotherapy causes the same degree of tumor size reduction, or the reduction degree is not less than about 5%, not less than about 4%, not less than about 3%, not less than about 2%, not less than about 1%, not less than about 0.5%, not less than about 0.1%, not less than about 0.01%, not less than about 0.001% or smaller.

In the present application, the term "gastrointestinal adverse events" generally refers to harmful and undesirable reactions, effects, actions, consequences, results or influences associated with the gastrointestinal tract or displayed in the gastrointestinal tract area, which are caused by a certain medicament or other medical treatments such as chemotherapy or surgery. They are also known as gastrointestinal adverse effects, gastrointestinal adverse influences or gastrointestinal adverse consequences. The gastrointestinal adverse events comprise adverse events in various parts (e.g., digestive tracts and glands) of the digestive system. For example, gastrointestinal adverse events may include, but not limited to, abdominal distension, abdominal pain, anal fissure, anal fistula, anal bleeding, anal mucositis, anal necrosis, anal pain, anal stenosis, anal ulcer, ascites, hiccup, cecal bleeding, cheilitis, chylous ascites, colitis, colonic fistula, colonorrhagia, colonic obstruction, colonic perforation, colonic stricture, colonic ulcer, constipation, dental caries, diarrhea, xerostomia, duodenal fistula, duodenal bleeding, duodenal obstruction, duodenuml perforation, duodenal stenosis, duodenal ulcer, dyspepsia, dysphagia, enterocolitis, intestinal fistula, esophageal fistula, esophageal bleeding, esophageal necrosis, esophageal obstruction, esophageal pain, esophageal perforation, esophageal stenosis, esophageal ulcer, esophageal variceal bleeding, oesophagitis, fecal incontinence, flatus, gastric fistula, gastrorrhagia, gastric necrosis, gastric perforation, gastric stenosis, gastric ulcer, gastritis, gastroesophageal reflux disease, gastrointestinal diseases, gastrointestinal fistula, gastrointestinal pain, gastroparesis, gum pain, hemorrhoidal bleeding, haemorrhoids, ileal fistula, ileal bleeding, ileocleisis, ileal perforation, ileal stenosis, ileal ulcer, ileus, intraperitoneal hemorrhage, jejunal fistula, jejunal bleeding, jejunal obstruction, jejunal perforation, jejunal stenosis, jejunal ulcer, chilalgia, lower gastrointestinal hemorrhage, malabsorption, oral mucositis, nausea, gastric obstruction, oral fistula, oral dysaesthesia, oral hemorrhage, oral pain, pancreatic-duct stenosis, pancreatic fistula, pancreatic bleeding, pancreatic necrosis, pancreatitis, periodontal diseases, peritoneal necrosis, rectitis, rectal rupture, rectofistula, rectal bleeding, rectal mucositis, rectal necrosis, rectal obstruction, rectal pain, rectal perforation, rectostenosis, rectal ulcer, retroperitoneal hemorrhage, salivary duct inflammation, salivary gland fistula, small bowel mucositis, small bowel obstruction, small bowel perforation, small bowel stenosis, small bowel ulcer, diarrhea, dental developmental disturbance, dental stain, toothache, appendicitis, upper gastrointestinal hemorrhage, visceral artery ischemia, vomiting.

In the present application, the term "gastrointestinal side effects" generally refers to harmful and adverse effects associated with the gastrointestinal tract or displayed in the gastrointestinal tract area, which are caused by a preventive medicine or a therapeutic medicine. Adverse effects are always undesirable, but undesirable effects are not necessarily adverse. The adverse effects of the preventive medicine or the therapeutic medicine may be harmful, unsuitable or dangerous. Gastrointestinal side effects include side effects in various parts (e.g., digestive tract and gland) of the digestive system. Chemotherapy-associated gastrointestinal side effects may refer to any abnormal clinical manifestations of gastrointestinal tract associated with the time of using the chemotherapeutic agent, but there may be no causal relationship between these abnormal manifestations and the administration of the chemotherapeutic agent.

In the present application, the term "gastric mucosal injury diseases" generally refers to the diseases or disorders with symptoms of gastric mucosal injury, which can include abnormal color, bleeding spots, congestion and erosion of the gastric mucosa.

In the present application, the term "intestinal mucosal injury diseases" generally refers to the diseases or disorders with symptoms of intestinal mucosal injury, which can include abnormal color, bleeding spots, congestion and erosion of the intestinal mucosa.

In the present application, the term "diarrhea" generally refers to a disease or disorder with the features of increased peristalsis frenquency and/or relaxation or watery peristalsis. In the present application, the diarrhea may occur or deteriorate after administration of the chemotherapy. In some cases, the description of the standardized definition of diarrhea can refer to various versions of Common Terminology Criteria for Adverse Events (CTCAE) issued by National Cancer Institute (NCI). In some cases, diarrhea can also be evaluated referring to animal experiments: for example, the severity of diarrhea is scored in accordance with the method of Akinobu Kurita (Cancer Chemother Pharmacol 2000; 46:211-20.), wherein, Grade 0: normal feces; Grade 1: mild diarrhea, the stools is slightly wet and soft; Grade 2: moderate diarrhea, loose stools and mild perianal contamination; Grade 3: severe diarrhea, watery stools and accompanied by severe perianal staining.

In the present application, the term "constipation" generally refers to a disease or disorder with the features of irregular defecation, infrequent defecation or difficult defecation of intestines. In the present application, the constipation may occur or deteriorate after administration of the chemotherapy. The description of the standardized definition of constipation can refer to various versions of Common Terminology Criteria for Adverse Events (CTCAE) issued by National Cancer Institute (NCI).

In the present application, the term "enteritis" generally refers to inflammation of the intestines, including colitis, enteritis, enterocolitis, duodenitis, ileitis, appendicitis, rectitis and/or jeiunitis. In the present application, the enteritis may occur or deteriorate after administration of the chemotherapy. The description of the standardized definition of enteritis can refer to various versions of Common Terminology Criteria for Adverse Events (CTCAE) issued by National Cancer Institute (NCI).

In the present application, the term "in the absence of prevention or treatment" generally refers to a condition without performing prevention or treatment on gastrointestinal adverse events, and without taking measures to make the gastrointestinal adverse events to be weakened or disappear. The measures to make the gastrointestinal adverse events to be weakened or disappear may be, for example, administration of a medicament or other means to prevent or treat gastrointestinal adverse events, stopping the administration of medicaments or other means that cause gastrointestinal adverse events, including administration of the compound as shown in Formula I or the medicament of the present application. In the present application, after administering the chemotherapeutic agent of the present application, if no measures are taken to make the gastrointestinal adverse events to be weakened or disappear, the gastrointestinal adverse events will occur or deteriorate 1 hr, 2 hrs, 3 hrs, 4 hrs, 5 hrs, 6 hrs, 7 hrs, 8 hrs, 9 hrs, 10 hrs, 11 hrs, 12 hrs, 1 day, 2 days, 4 days, 7 days, 2 weeks, 3 weeks, 1 month, 2 months or longer after the administration of the chemotherapy. If the compound as shown in Formula I or the medicament of the present application is administered, the gastrointestinal adverse events may be weakened or disappear.

In the present application, the term "NCI-CTCAE" generally refers to a standardized definition of adverse events issued by National Cancer Institute (NCI)-Common Terminology Criteria for Adverse Events (CTCAE), which is used to describe the severity of organ toxicity in patients treated for cancer. With the development of scientific evidence, the criteria can be updated continuously. In the present application, "NCI-CTCAE" may comprise any version of "NCI-CTCAE". In CTCAE V5.0, constipation and diarrhea are defined into 5 grades respectively, as shown in Table 1 and Table 2.

Table 1 Standardized definition on the grading of constipation in CTCAE V5.0

| CTCAE Grading | Criteria |
|---|---|
| Grade 1 | Incidental or intermittent symptoms; occasionally employing stool softener, laxatives, antisecosis or enteroclysis |
| Grade 2 | persistent symptoms in need of long-term use of laxatives or enteroclysis; limited instrumental activities of daily living (ADL) |
| Grade 3 | Constipation with manual evacuation indications: limited self-care |
| Grade 4 | Life-threatening consequences; indicating emergency interventions |
| Grade 5 | Death |

Table 2 Standardized definition on the grading of diarrhea in CTCAE V5.0

| CTCAE Grading | Criteria |
|---|---|
| Grade 1 | An increasement of < 4 times of defecation compared with the basic standard every day; compared with the baseline, the output of neostomy increases slightly |

(continued)

| CTCAE Grading | Criteria |
|---|---|
| Grade 2 | An increasement of 4-6 times of defecation compared with the basic standard every day; compared with the baseline, the output of neostomy increases moderately; limited instrumental ADL |
| Grade 3 | An increasement of >= 7 times of defecation compared with the basic standard every day; indicating hospitalization; compared with the baseline, the output of neostomy increases significantly; limited self-care |
| Grade 4 | Life-threatening consequences; indicating emergency interventions |
| Grade 5 | Death |

In the present application, the term "cancer" generally refers to any medical conditions mediated by the growth, proliferation or metastasis of tumors or malignant cells and causing solid tumors and nonsolid tumors (e.g., leukemia). The cancer in the present application may include, but not limited to, malignant epithelial tumors (epithelium-derived cancers), lung cancer (e.g., non-small cell lung cancer), breast cancer, skin cancer, bladder cancer, colon cancer, gastrointestinal (GI) cancer, prostatic cancer, pancreatic cancer, uterine cancer, cervical cancer, ovarian cancer, esophagus cancer, head and neck cancer, gastric cancer and throat cancer.

**Detailed description of the invention**

**Compound as shown in Formula I**

In one aspect, the present application provides a use of a compound as shown in Formula I in preparing a medicament, which can be used for preventing, relieving and/or treating chemotherapy-associated gastrointestinal side effects in a subject:

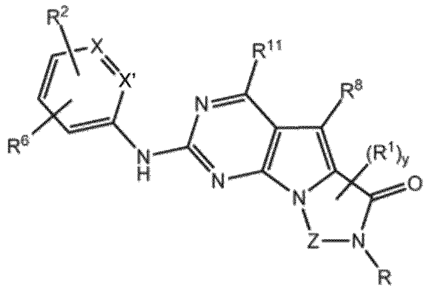

Formula I,

wherein,

Z is $-(CH_2)_X-$, wherein X is 1, 2, 3 or 4, or $-O-(CH_2)_Z-$, wherein z is 2, 3 or 4;
X and X' are each independently CH or N;
R, $R^8$ and $R^{11}$ are each independently H, $C_1$-$C_3$ alkyl or haloalkyl, cycloalkyl or cycloalkyl containing one or more heteroatoms selected from N, O or S, -(alkylene)$_m$-$C_3$-$C_8$ cycloalkyl, -(alkylene)$_m$-aryl, -(alkylene)$_m$-heterocyclyl, -(alkylene)$_m$-heteroaryl, -(alkylene)$_m$-$NR^3R^4$, -(alkylene)$_m$-C(O)-$NR^3R^4$, -(alkylene)$_m$-O-$R^5$, -(alkylene)$_m$-$S(O)_n$-$R^5$, or -(alkylene)$_m$-$S(O)_n$-$NR^3R^4$, any of which may be optionally independently substituted with one or more R groups as allowed by valance, and wherein two $R^x$ groups bound to the same or adjacent atoms may optionally combine to form a ring, and wherein m is 0 or 1, n is 0, 1 or 2;
$R^1$ is independently aryl, alkyl, cycloalkyl or haloalkyl, wherein each of the alkyl, cycloalkyl and haloalkyl optionally comprises O or N heteroatoms in place of a carbon in the chain, and two $R^1$'s on adjacent ring atoms or on the same ring atom together with the ring atom(s) to which they are attached optionally form a 3-8-membered cycle;
y is 0, 1, 2, 3 or 4;
$R^2$ is -(alkylene)$_m$-heterocyclyl, -(alkylene)$_m$-heteroaryl, -(alkylene)$_m$-$NR^3R^4$, -(alkylene)$_m$-C(O)-$NR^3R^4$, -(alkylene)$_m$-C(O)-O-alkyl; -(alkylene)$_m$-O-$R^5$, -(alkylene)$_m$-$S(O)_n$-$R^5$ or -(alkylene)$_m$-$S(O)_n$-$NR^3R^4$, any of which may be optionally independently substituted with one or more $R^x$ groups as allowed by valance, and wherein two $R^x$ groups bound to the same or adjacent atoms may optionally combine to form a ring, and wherein m is 0 or 1, n is

0, 1 or 2;

$R^3$ and $R^4$ at each occurrence are independently:

(i) hydrogen or

(ii) alkyl, cycloalkyl, heterocyclyl, aryl, heteroaryl, cycloalkylalkyl, heterocycloalkyl, arylalkyl or heteroarylalkyl, any of which may be optionally independently substituted with one or more $R^x$ groups as allowed by valance, and wherein two $R^x$ groups bound to the same or adjacent atoms may optionally combine to form a ring; or $R^3$ and $R^4$ together with the nitrogen atom to which they are attached may combine to form a heterocyclic ring optionally independently substituted with one or more $R^x$ groups as allowed by valance, and wherein two $R^x$ groups bound to the same or adjacent atoms may optionally combine to form a ring;

$R^5$ and $R^{5*}$ at each occurrence are:

(i) hydrogen or

(ii) alkyl, alkenyl, alkynyl, cycloalkyl, heterocyclyl, aryl, heteroaryl, cycloalkylalkyl, heterocycloalkyl, arylalkyl or heteroarylalkyl, any of which may be optionally independently substituted with one or more $R^x$ groups as allowed by valance;

$R^x$ at each occurrence is independently halo, cyano, nitro, oxo, alkyl, haloalkyl, alkenyl, alkynyl, cycloalkyl, cycloalkenyl, heterocyclyl, aryl, heteroaryl, arylalkyl, heteroarylalkyl, cycloalkylalkyl, heterocycloalkyl, $-(alkylene)_m-OR^5$, $-(alkylene)_m-O-alkylene-OR^5$, $-(alkylene)_m-S(O)_n-R^5$, $-(alkylene)_m-NR^3R^4$, $-(alkylene)_m-CN$, $-(alkylene)_m-C(O)-R^5$, $-(alkylene)_m-C(S)-R^5$, $-(alkylene)_m-C(O)-OR^5$, $-(alkylene)_m-O-C(O)-R^5$, $-(alkylene)_m-C(S)-OR^5$, $-(alkylene)_m-C(O)-(alkylene)_m-NR^3R^4$, $-(alkylene)_m-C(S)-NR^3R^4$, $-(alkylene)_m-N(R^3)-C(O)-NR^3R^4$, $-(alkylene)_m-N(R^3)-C(S)-NR^3R^4$, $-(alkylene)_m-N(R^3)-C(O)-R^5$, $-(alkylene)_m-N(R^3)-C(S)-R^5$, $-(alkylene)_m-O-C(O)-NR^3R^4$, $-(alkylene)_m-O-C(S)-NR^3R^4$, $-(alkylene)_m-SO_2-NR^3R^4$, $-(alkylene)_m-N(R^3)-SO_2-R^5$, $-(alkylene)_m-N(R^3)-SO_2-NR^3R^4$, $-(alkylene)_m-N(R^3)-C(O)-OR^5$, $-(alkylene)_m-N(R^3)-C(S)-OR^5$ or $-(alkylene)_m-N(R^3)-SO_2-R^5$; wherein:

the alkyl, haloalkyl, alkenyl, alkynyl, cycloalkyl, cycloalkenyl, heterocyclyl, aryl, heteroaryl, arylalkyl, heteroarylalkyl, cycloalkylalkyl and heterocycloalkyl may be further independently substituted with one or more of the following groups:

$-(alkylene)_m-CN$, $-(alkylene)_m-OR^{5*}$, $-(alkylene)_m-S(O)_n-R^{5*}$, $-(alkylene)_m-NR^{3*}R^{4*}$, $-(alkylene)_m-C(O)-R^{5*}$, $-(alkylene)_m-C(=S)R^{5*}$, $-(alkylene)_m-C(=O)OR^{5*}$, $-(alkylene)_m-OC(=O)R^{5*}$, $-(alkylene)_m-C(S)-OR^{5*}$, $-(alkylene)_m-C(O)-NR^{3*}R^{4*}$, $-(alkylene)_m-C(S)-NR^{3*}R^{4*}$, $-(alkylene)_m-N(R^{3*})-C(O)-NR^{3*}R^{4*}$, $-(alkylene)_m-N(R^{3*})-C(S)-NR^{3*}R^{4*}$, $-(alkylene)_m-N(R^{3*})-C(O)-R^{5*}$, $-(alkylene)_m-N(R^{3*})-C(S)-R^{5*}$, $-(alkylene)_m-O-C(O)-NR^{3*}R^{4*}$, $-(alkylene)_m-O-C(S)-NR^{3*}R^{4*}$, $-(alkylene)_m-SO_2-NR^{3*}R^{4*}$, $-(alkylene)_m-N(R^{3*})-SO_2-R^{5*}$, $-(alkylene)_m-N(R^{3*})-SO_2-NR^{3*}R^{4*}$, $-(alkylene)_m-N(R^{3*})-C(O)-OR^{5*}$, $-(alkylene)_m-N(R^{3*})-C(S)-OR^{5*}$ or $-(alkylene)_m-N(R^{3*})-SO_2-R^{5*}$,

n is 0, 1 or 2, and m is 0 or 1;

$R^{3*}$ and $R^{4*}$ at each occurrence are independently:

(i) hydrogen or

(ii) alkyl, alkenyl, alkynyl, cycloalkyl, heterocyclyl, aryl, heteroaryl, cycloalkylalkyl, heterocycloalkyl, arylalkyl or heteroarylalkyl, any of which may be optionally independently substituted with one or more $R^x$ groups as allowed by valance; or $R^{3*}$ and $R^{4*}$ together with the nitrogen atom to which they are attached may combine to form a heterocyclic ring optionally independently substituted with one or more $R^x$ groups as allowed by valance; and

$R^6$ is H or lower alkyl, $-(alkylene)_m-heterocyclyl$, $-(alkylene)_m-heteroaryl$, $-(alkylene)_m-NR^3R^4$, $-(alkylene)_m-C(O)-NR^3R^4$; $-(alkylene)_m-O-R^5$, $-(alkylene)_m-S(O)_n-R^5$ or $-(alkylene)_m-S(O)_n-NR^3R^4$, any of which may be optionally independently substituted with one or more $R^x$ groups as allowed by valance, and wherein two $R^x$ groups bound to the same or adjacent atoms may optionally combine to form a ring;

or a pharmaceutically acceptable salt, prodrug, isotopic variant (e.g., completely or partially deuterated form) or solvate thereof.

In another aspect, the present application provides a method of preventing, relieving and/or treating chemotherapy-associated gastrointestinal side effects in a subject, comprising administering the compound as shown in Formula I.

In the present application, the term "a compound as shown in Formula I" may be known as "a compound of Formula I", "Formula I". Such a term is also defined as comprising all the forms of the compound of Formula I, including hydrate, solvate, isomer, crystalline and non-crystalline form, isomorphism, polymorphism, and metabolite. For example, the compound of Formula I or a pharmaceutically acceptable salt thereof may be present in an unsolvated or solvated form. When the binding force of solvent or water thereto is relatively strong, the coordination compound has a definite stoichiometry that is not affected by humidity. However, if the binding force of solvent or water thereto is relatively weak, e. g., in a channel solvate and a hygroscopic compound, the content of water/solvent will depend on the humidity and drying conditions. In this case, non-stoichiometry is normal.

The "compound of Formula I" may comprise chiral carbon atom(s). In the present application, the carbon-carbon bonds in the compound of Formula I may be represented by solid lines, solid wedges, or dot wedges. Depicting the bond to a chiral carbon atom with solid lines means that all the potential stereoisomers (e. g., specific enantiomers, racemates, etc.) on the carbon atom are comprised. The compound of the present application may comprise more than one chiral carbon atoms. In these compounds, depicting the bond to a chiral carbon atom with solid lines is intended to indicate that all the potential stereoisomers should be encompassed. For example, unless otherwise indicated, the compound of Formula I may present as enantiomers, diastereomers, or racemates and mixtures thereof. Depicting the bond to one or more chiral carbon atoms in the compound of Formula I with solid lines and depicting the bond to another chiral carbon atom in the same compound with solid or dot wedges indicate the presence of a mixture of diastereomers.

The compound of the present application may be present as inclusion compounds or other coordination compounds. The present application encompasses complexes, e.g., inclusion compounds, drug-host inclusion complex, wherein in contrast to the aforesaid solvates, drug and host are present in stoichiometric or non-stoichiometric amount. The present application further comprises a coordination compound of Formula I comprising two or more organic and/or inorganic components that may be stoichiometric or non-stoichiometric. The resultant complex may be ionized, partially ionized, or unionized.

The stereoisomer of Formula I comprises cis- and trans-isomers, optical isomers, e. g., R and S enantiomers, diastereomers, geometrical isomers, rotamers, conformational isomers and tautomers, the compound of Formula I, including the compounds exhibiting one or more of the aforesaid types of isomerisms, and mixtures thereof (e.g., racemate and diastereomer pair). The stereoisomer further comprises acid or base addition salts in which the counter ions have optical activity, e.g., D-lactate or L-lysine, or racemates, e.g., DL-tartrate or DL-arginine.

When any racemate crystallizes, there may be two different types of crystals. The first type is the racemic compounds (true racemates) as described above, wherein a homogeneous form of crystal is produced and comprises two enantiomers in equimolar amounts. The second type is a racemic mixture or agglomerate, wherein two forms of crystals are produced in equimolar amounts, each comprising a single enantiomer.

The compound of Formula I can exhibit tautomerism and structural isomerism. For example, the compound of Formula I may be present in several tautomeric forms, comprising enol and imine forms, and ketone and enamine forms; as well as geometric isomers and their mixtures. All these tautomeric forms are encompassed within the scope of the compound of Formula I. Tautomers are present in solution as a mixture of tautomers. In the solid form, one tautomer generally dominates. Even if one tautomer may be described, the present invention also comprises all the tautomers of the compound of Formula I.

The present application may further comprise isotope-labeled compounds that are the same as that of Formula I except that one or more atoms thereof are replaced with atoms having atomic mass or mass number different from those found in nature. Isotopes which may be incorporated into the compound of Formula I comprise isotopes of hydrogen, carbon, nitrogen, oxygen, phosphorus, fluorine and chlorine, e. g., but are not limited to: $^{2}H$, $^{3}H$, $^{13}C$, $^{14}C$, $^{15}N$, $^{18}O$, $^{17}O$, $^{31}P$, $^{32}P$, $^{35}S$, $^{18}F$, and $^{36}Cl$. Some isotope-labeled compounds of Formula I, e. g., into which radioisotopes (e. g., $^{3}H$ and $^{14}C$) are incorporated, may be used for measuring the distribution of drugs and/or substrate tissues due to their easy preparation and detectability. Heavier isotopes, such as, $^{2}H$, can provide certain therapeutic advantages due to its greater metabolic stability, e. g., longer half-life in the body or lower dose requirements. The isotope-labeled compounds of Formula I generally may be prepared with isotope-labeled reagents in stead of non-isotope-labeled reagents.

The compound of the present application may be used as salts derived from inorganic or organic acids. Some compounds have advantages, such as, enhanced drug stability at different temperatures and humidities, or desired solubilities in water/oil due to the physical properties of one or more salts. In some cases, the salts of the compound can also be adjuvants for use in the separation, purification, and/or resolution of the compound.

In the present application, the compound has a structure of Formula I and $R^6$ may be absent.

In the present application, the $R^x$ in the Formula I may be not further substituted.

In the present application, the $R^8$ in the Formula I may be hydrogen or $C_1$-$C_3$ alkyl.

In the present application, the $R^{11}$ in the Formula I may be hydrogen or $C_1$-$C_3$ alkyl.

In the present application, the compound may comprise a compound as shown in Formula Ia:

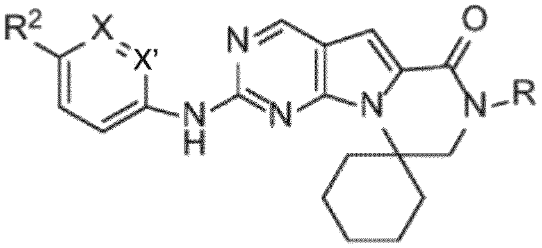

Formula Ia.

In the present application, the X in the Formula I or Formula Ia may be C.

In the present application, the X' in the Formula I or Formula Ia may be N.

In the present application, the R in the Formula I or Formula Ia may be hydrogen or $C_1$-$C_3$ alkyl.

In the present application, the $R^2$ in the Formula I or Formula Ia may be -(alkylene)$_m$-heterocyclyl, -(alkylene)$_m$-$NR^3R^4$, -(alkylene)$_m$-C(O)-$NR^3R^4$, -(alkylene)$_m$-C(O)-O-alkyl or -(alkylene)$_m$-$OR^5$, any of which may be optionally independently substituted with one or more $R^x$ groups as allowed by valance, and wherein two $R^x$ groups bound to the same or adjacent atoms may optionally combine to form a ring, and wherein m is 0 or 1.

In the present application, the $R^2$ in the Formula I or Formula Ia may be -(alkylene)$_m$-heterocyclyl, -(alkylene)$_m$-$NR^3R^4$, -(alkylene)$_m$-C(O)-$NR^3R^4$, -(alkylene)$_m$-C(O)-O-alkyl or -(alkylene)$_m$-$OR^5$, which is not further substituted.

In the present application, m in the $R^2$ in the Formula I or Formula Ia may be 1.

In the present application, the alkylene in the $R^2$ in the Formula I or Formula Ia may be methylene.

In the present application, the $R^2$ in the Formula I or Formula Ia may be

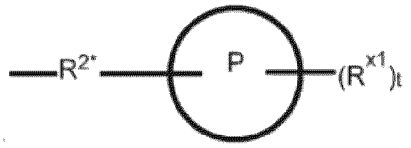

,

wherein, $R^{2*}$ may be a bond, alkylene, -(alkylene)$_m$-O-(alkylene)$_m$-, -(alkylene)$_m$-C(O)-(alkylene)$_m$-, -(alkylene)$_m$-S(O)$_2$-(alkylene)$_m$- and -(alkylene)$_m$-NH-(alkylene)$_m$-, wherein each m may be independently 0 or 1;

P may be a 4- to 8-membered mono- or bicyclic saturated heterocyclyl group;
each $R^{x1}$ may be independently -(alkylene)$_m$-(C(O))$_m$-(alkylene)$_m$-(N($R^N$))$_m$-(alkyl)$_m$, wherein each m may be independently 0 or 1 provided at least one m may be 1; -(C(O))-O-alkyl; -(alkylene)$_m$-cycloalkyl, wherein m may be 0 or 1; -N($R^N$)-cycloalkyl; -C(O)-cycloalkyl; -(alkylene)$_m$-heterocyclyl, wherein m may be 0 or 1; or -N($R^N$)-heterocyclyl; -C(O)-heterocyclyl; -S(O)$_2$-(alkylene)$_m$, wherein m may be 1 or 2, wherein:

$R^N$ may be H, $C_1$ to $C_4$ alkyl or $C_1$ to $C_6$ heteroalkyl, and
wherein two $R^{x1}$ can, together with the atoms to which they attach on P, which may be the same atom, form a ring; and
t is 0, 1 or 2.

In the present application, the each $R^{x1}$ in the $R^2$ may be optionally substituted by unsubstituted alkyl, halogen or hydroxyl.

In the present application, $R^{x1}$ in the $R^2$ may be hydrogen or unsubstituted $C_1$-$C_4$ alkyl.

In the present application, the at least one $R^{x1}$ in the $R^2$ may be -(alkylene)$_m$-heterocyclyl, wherein m is 0 or 1.

In the present application, the $R^2$ in the Formula I or Formula Ia is

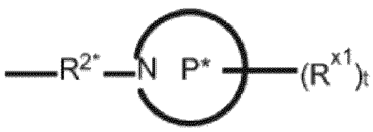

,

wherein P* may be a 4- to 8-membered mono- or bicyclic saturated heterocyclyl group.

In the present application, the $R^2$ in the Formula I or Formula Ia may be

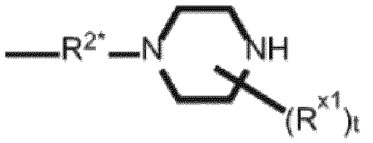

In the present application, the $R^2$ in the Formula I or Formula Ia may be

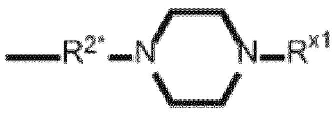

In the present application, the $R^2$ in the Formula I or Formula Ia may be

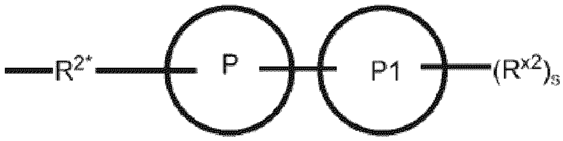

,

wherein, $R^{2*}$ is a bond, alkylene, -(alkylene)$_m$-O-(alkylene)$_m$-, -(alkylene)$_m$-C(O)-(alkylene)$_m$-, -(alkylene)$_m$-S(O)$_2$-(alkylene)$_m$- and -(alkylene)$_m$-NH-(alkylene)$_m$-, wherein each m is independently 0 or 1, s is 0, 1 or 2;

P is a 4- to 8-membered mono- or bicyclic saturated heterocyclyl group;
P1 is a 4- to 6-membered monocyclic saturated heterocyclyl group; and
each $R^{X2}$ may be independently hydrogen or alkyl; and s is 0, 1 or 2.

In the present application, the $R^2$ in the Formula I or Formula Ia may be

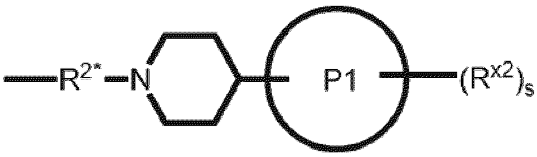

In the present application, P1 in the $R^2$ may comprise at least one nitrogen.
In the present application, any alkylene in $R^{2*}$ in any one of the above embodiments may be not further substituted.
In the present application, the $R^{2*}$ in the $R^2$ may be a bond.
In the present application, the $R^{x1}$ in the $R^2$ may be hydrogen, methyl or propyl.
For example, the $R^2$ may be

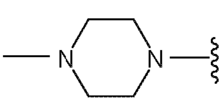, 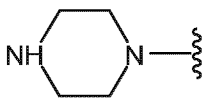, 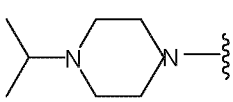 or

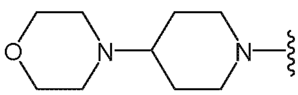.

For example, the compound as shown in Formula I may be selected from the following

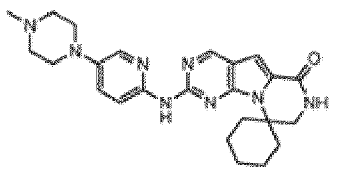

Compound I

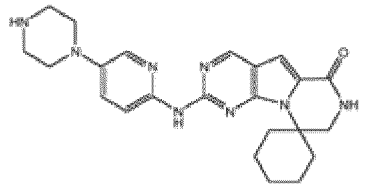

Compound II

group:

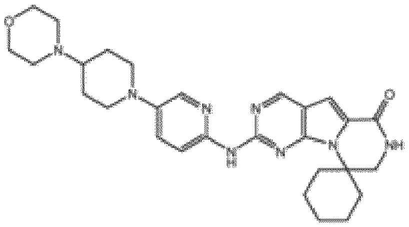

Compound III

and

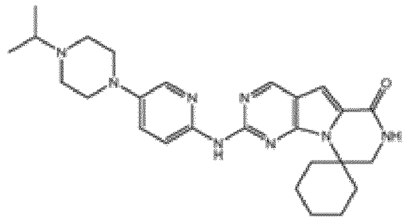

Compound IV

.

For example, the compound as shown in Formula I may be

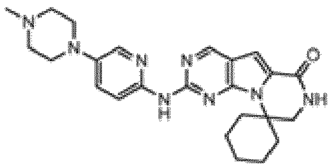

Compound I

. For example, the compound as shown in Formula I may be the hydrochloride form of

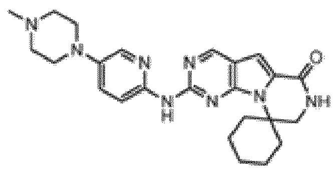

Compound I

.

For example, the compound as shown in Formula I may be

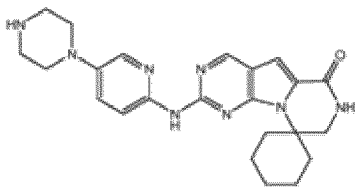

Compound II

. For example, the compound as shown in Formula I may be the hydrochloride form of

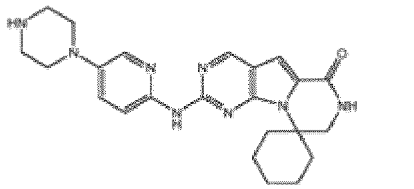

Compound II

.

For example, the compound as shown in Formula I may be

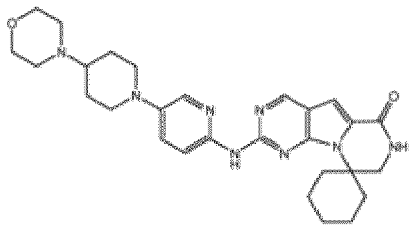

Compound III

. For example, the compound as shown in Formula I may be the hydrochloride form of

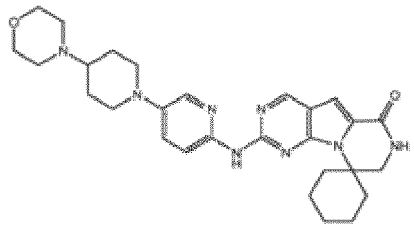

Compound III

.

For example, the compound as shown in Formula I may be

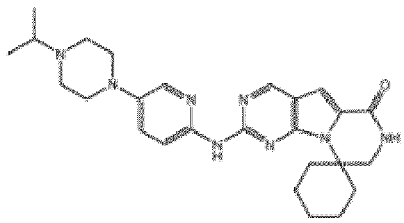

Compound IV

. For example, the compound as shown in Formula I may be the hydrochloride form of

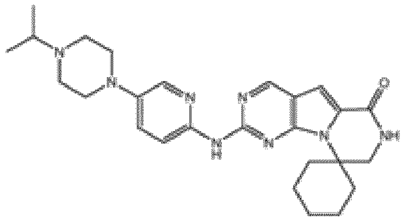

Compound IV

## Chemotherapy

In the present application, the chemotherapeutic agent used in the chemotherapy may be any one kind of compound or medicament used for preventing, relieving and/or treating cancers selected from those known to persons skilled in the art. In terms of the mechanism of action, the chemotherapy may comprise a DNA synthesis inhibitor, an RNA synthesis inhibitor, a protein synthesis inhibitor, a cell division inhibitor, a DNA base analogue, a topoisomerase inhibitor and/or a telomerase synthesis inhibitor.

In the present application, the chemotherapeutic agent may be toxic to immunologic effector cells. In the present application, the chemotherapeutic agent may inhibit cell growth. In the present application, the administered chemotherapeutic agent may be a chemotherapeutic agent for DNA damages. In the present application, the chemotherapeutic agent is a protein synthesis inhibitor, a DNA-damage chemotherapeutic agent, an alkylating agent, a topoisomerase inhibitor, an RNA synthesis inhibitor, a DNA composite binding agent, a thiolate alkylating agent, a guanine alkylating agent, a tubulin binding agent, a DNA polymerase inhibitor, an anti-cancer enzyme, a RAC1 inhibitor, a thymidylic acid synthetase inhibitor, an oxazophosphorine compound, an integrin inhibitor, such as cilengitide, camptothecin or homocamptothecin, an antifolate, a folic acid antimetabolite, a telomerase inhibitor and/or a telomere DNA-binding compound.

For example, the alkylating agent may comprise alkyl sulfonates, such as busulfan, improsulfan and piposulfan; aziridines, such as benzodizepa, carboquone, meturedepa and uredepa; ethylene imines and methyl melamines, such as altretamine, triethylene melamine, triethylene phosphamide, triethylene thiophosphamide and trimethylol melamine; mechlorethamines, such as chlorambucil, chlornaphazine, cyclophosphamide, estramustine, dichloromethyl diethylamine, mechlorethamine oxide hydrochloride, Melphalan, novembichine, phenesterine, prednimustine, trofosfamide and uracil mustard; and nitrosoureas, such as carmustine, chlorozotocin, fotemustine, lomustine, nimustine and ranimustine. Other chemotherapeutic agents may comprise daunorubicin, doxorubicin, idarubicin, epirubicin, mitomycin and streptozotocin. Antimetabolites for the chemotherapy may comprise gemcitabine, mercaptopurine, thioguanine, cladribine, fludarabine phosphate, fluorouracil (5-FU), floxuridine, cytarabine, pentostatin, methotrexate, azathioprine, acyclovir, adenine β-1-D-arabinoside, amethopterin, aminopterin, 2-aminopurine, aphidicolin, 8-azaguanine, azaserine, 6-azauracil, 2'-azido-2'-deoxynucleoside, 5-bromodeoxycytidine, cytosine β-1-D-arabinoside, diazoxonorleucine, dideoxynucleoside, 5-fluorodeoxycytidine, 5-fluorodeoxyuridine and hydroxyurea.

For example, the protein synthesis inhibitor may comprise abrin, aurin tricarboxylic acid, chloramphenicol, colicin E3, cycloheximide, diphtheria toxin, edeine A, emetine, erythromycin, ethionine, fluoride, 5-fluorotryptophan, fusidic acid, guanylyl methylene bisphosphonate and guanylyl imino diphosphate, kanamycin, kasugamycin, kirromycin and O-methylthreonine. Other protein synthesis inhibitors comprise modeccin, neomycin, norvaline, pactamycin, paromomycine, puromycin, ricin, Shiga toxin, showdomycin, sparsomycin, spectinomycin, streptomycin, tetracycline, thiostreptone and trimethoprim.

For example, the DNA synthesis inhibitor may include an alkylating agent, such as dimethyl sulfate, mechlorethamine and sulfur mustard; an intercalating agent, such as acridine dyes, actinomycin, anthracenes, benzopyrene, ethidium bromide, propidium diiodide-intertwining; a topoisomerase inhibitor, such as irinotecan, teniposide, coumermycin, nalidixic acid, novobiocin and oxolinic acid; a cell division inhibitor, including demecolcine, mitoxantrone, colchicine, vinblastine and vincristine; and other medicaments, such as distamycin and netropsin.

In the present application, the chemotherapeutic agent may be a DNA composite binding agent, e.g., camptothecin or etoposide; a thiolate alkylating agent, e.g. nitrosourea, BCNU, CCNU, ACNU or fotesmustine; a guanine alkylating agent, e.g., temozolomide; a tubulin binding agent, such as vinblastine, vincristine, vinorelbine, vinflunine, cryptophycin 52, halichondrin such as halichondrin B, aplysiatoxin such as aplysiatoxin 10 and aplysiatoxin 15, hemiasterlins (such as hemiasterlin A and hemiasterlin B), colchicine, combrestatins, 2-methoxy estradiol, E7010, paclitaxel, Docetaxel, epothilone, discodermolide; a DNA polymerase inhibitor, e.g., cytarabine; anti-cancer enzyme, e.g. asparaginase; a RAC1 inhibitor, e.g., 6-thioguanine; a thymidylic acid synthetase inhibitor, e.g., capecitabine or 5-FU; an oxazophosphorine compound, e.g., Endoxan; an integrin inhibitor, e.g., cilengitide; an antifolate, e.g., pralatrexate; a folic acid antimetabolite, e.g., pemetrexed; or camptothecin or homocamptothecin, e.g., diflomotecan.

In the present application, the compound as shown in Formula I may be used for preventing, relieving and/or

treating the above one or more chemotherapy-associated gastrointestinal side effects.

In the present application, the compound as shown in Formula I may be used for preventing, relieving and/or treating gastrointestinal side effects associated with the administration of the following chemotherapeutic agents: fluorouracil, oxaliplatin, irinotecan, topotecan, etoposide, cisplatin, docetaxel, gemcitabine, carboplatin, methotrexate, doxorubicin, cytarabine, vinorelbine and capecitabine, and a combination thereof.

In the present application, the compound as shown in Formula I may be used for preventing, relieving and/or treating gastrointestinal side effects associated with the administration of the following chemotherapeutic agents: fluorouracil, oxaliplatin, irinotecan (CPT-11), tetrahydrofolic acid, and a combination thereof. In the present application compound as shown in Formula I may be used for preventing, relieving and/or treating gastrointestinal side effects associated with the following chemotherapies: 5-fluorouracil, irinotecan and oxaliplatin, and a combination thereof.

In the present application, the chemotherapeutic agents may not include tumor-targeted medicaments. In the present application, the chemotherapeutic agents may not include those kinase inhibitors which prevent the division of cancer cells by inhibiting abnormally activated kinase. In the present application, the chemotherapeutic agents may not include an antibody and/or an angiogenesis inhibitor. For example, the chemotherapeutic agent may not include a tyrosine kinase inhibitor.

In the present application, the chemotherapeutic agent may be administered continuously. For example, the chemotherapy may be administered continuously for 2 days, 3 days, 4 days, 5 days, 6 days, 7 days, 8 days, 9 days or more days. In some embodiments, the days for the continuous administration of the chemotherapy are no more than 7 days, for example, the chemotherapy may be administered continuously for 2 days, 3 days, 4 days, 5 days, 6 days or 7 days. In the present application, the administration frequency of the chemotherapy may be once a day, twice a day, 3 times a day or others.

In the present application, the chemotherapeutic agent may be administered noncontinuously. In the present application, the administration frequency of the chemotherapy may be once every two days, once every three days, twice every three days or others.

For example, the continuous administration time of the chemotherapy may be no more than 7 days. For example, the continuous administration time of the chemotherapy may be less than 7 days (e.g., administration continuously for 2 days, 3 days, 4 days, 5 days or 6 days).

For example, the administration cycle of the chemotherapy may be 3 days, 5 days, 7 days, 2 weeks, 20 days, 1 month, 2 months or longer. The administration mode, administration route and/or administration time of the chemotherapeutic agent may refer to the package insert of the chemotherapeutic agent.

In the present application, one or more different chemotherapies may be used. In some embodiments, the compound as shown in Formula I may be used in combination with one or more other cancer therapies. The other cancer therapies may be common methods used for treating cancers in the art, e.g., cytotoxic anticancer agent, immunotherapeutic anticancer agent or hormonotherapeutic anticancer agent. In accordance with the present application, the medicament used for treating a cancer may also be used in combination with radiotherapy or surgery. In some embodiments, in the case of the combination use of the compound as shown in Formula I and other anticancer agents, they can be administered to the subject at the same time, or separately administered at a certain interval.

## Gastrointestinal side effects

In the present application, the compound as shown in Formula I can prevent, relieve and/or treat gastrointestinal side effects associated with the administration of chemotherapy in a subject. In the present application, chemotherapy-associated gastrointestinal side effects may mean that the gastrointestinal side effects are caused by the administration of the chemotherapy and will occur or deteriorate after the administration of the chemotherapy. In the absence of prevention or treatment, the gastrointestinal side effects will occur or deteriorate about 1 hr, about 2 hrs, about 3 hrs, about 4 hrs, about 5 hrs, about 6 hrs, about 7 hrs, about 8 hrs, about 9 hrs, about 10 hrs, about 11 hrs, about 12 hrs, about 1 day, about 2 days, about 4 days, about 7 days, about 2 weeks, about 3 weeks, about 1 month, about 2 months or longer after the administration of the chemotherapy.

In some embodiments, before administering the chemotherapy to the subject, the subject does not develop the gastrointestinal side effects; after administering the chemotherapy to the subject, the subject develops the gastrointestinal side effects.

In some embodiments, before administering the chemotherapy to the subject, the subject has developed the gastrointestinal side effects; after administering the chemotherapy to the subject, the severity of the gastrointestinal side effects in the subject increases. In the present application, after the administration of the chemotherapy, the symptoms of gastrointestinal side effects (e.g., diarrhea, enteritis or constipation) in the subject may deteriorate by at least about 10%, e.g., by about 15%, about 20%, about 25%, about 30%, about 35%, about 40%, about 45%, about 50%, about 55%, about 60%, about 65%, about 70%, about 75%, about 80%, about 85%, about 90%, about 95%, about 99% or higher. For example, in accordance with the criteria of NCI-CTCAE, after administering the chemotherapy to the subject,

the severity of the gastrointestinal side effects (e.g., diarrhea, enteritis or constipation) in the subject increases from Grade 1 to Grade 2, from Grade 1 to Grade 3, from Grade 1 to Grade 4, from Grade 1 to Grade 5, from Grade 2 to Grade 3, from Grade 2 to Grade 4, from Grade 2 to Grade 5, from Grade 3 to Grade 4, from Grade 3 to Grade 5 or from Grade 4 to Grade 5. For example, in accordance with the method of Akinobu Kurita, after administering the chemotherapy to the subject, the scoring of constipation in the subject increases from Grade 0 to Grade 1, from Grade 0 to Grade 2, from Grade 0 to Grade 3, from Grade 1 to Grade 2, from Grade 1 to Grade 3 or from Grade 2 to Grade 3.

In some embodiments, the gastrointestinal side effects comprise gastric mucosal injury diseases and/or intestinal mucosal injury diseases.

In some embodiments, the gastrointestinal side effects comprise diarrhea, abdominal pain, nausea, vomiting, mucositis, loss of appetite, gastric ulcer, gastritis, constipation, enteritis, intestinal perforation, intestinal bleeding, ulcer and/or intestinal necrosis. In some embodiments, the gastrointestinal side effects comprise abnormal defecation. In some embodiments, the gastrointestinal side effects comprise diarrhea and/or constipation.

In some embodiments, the chemotherapy-associated gastrointestinal side effects comprise chemotherapy-associated gastric mucosal injury diseases and/or chemotherapy-associated intestinal mucosal injury diseases.

In some embodiments, the chemotherapy-associated gastrointestinal side effects comprise chemotherapy-associated diarrhea, chemotherapy-associated abdominal pain, chemotherapy-associated nausea, chemotherapy-associated vomiting, chemotherapy-associated mucositis, chemotherapy-associated loss of appetite, chemotherapy-associated gastric ulcer, chemotherapy-associated gastritis, chemotherapy-associated constipation, chemotherapy-associated enteritis, chemotherapy-associated intestinal perforation, chemotherapy-associated intestinal bleeding, chemotherapy-associated ulcer and/or chemotherapy-associated intestinal necrosis. In some embodiments, the chemotherapy-associated gastrointestinal side effects comprise chemotherapy-associated abnormal defecation. In some embodiments, the chemotherapy-associated gastrointestinal side effects comprise chemotherapy-associated diarrhea and/or chemotherapy-associated constipation.

In the present application, after administration of the compound as shown in Formula I of the present application, the severity of chemotherapy-associated gastrointestinal side effects in the subject is relieved. In the present application, the relief may generally mean that the occurrence or development of the gastrointestinal side effects in the subject is delayed. In the present application, after administration of the compound as shown in Formula I, the symptoms of gastrointestinal side effects in the subject may be ameliorated.

In the present application, after administration of the compound as shown in Formula I, the symptoms of gastrointestinal side effects in the subject may be ameliorated by at least about 10%, e.g., by about 15%, about 20%, about 25%, about 30%, about 35%, about 40%, about 45%, about 50%, about 55%, about 60%, about 65%, about 70%, about 75%, about 80%, about 85%, about 90%, about 95%, about 99% or more.

For example, in accordance with the criteria of NCI-CTCAE, after administration of the compound as shown in Formula I, the symptoms of gastrointestinal side effects (e.g., diarrhea, enteritis and/or constipation) in the subject may decrease from Grade 5 to Grade 4, from Grade 5 to Grade 3, from Grade 5 to Grade 2, from Grade 5 to Grade 1, from Grade 4 to Grade 3, from Grade 4 to Grade 2, from Grade 4 to Grade 1, from Grade 3 to Grade 2, from Grade 3 to Grade 1 or from Grade 2 to Grade 1.

Alternatively, in accordance with the method of Akinobu Kurita, after administration of the compound as shown in Formula I, the scoring of diarrhea in the subject may decrease from Grade 3 to Grade 2, from Grade 3 to Grade 1, from Grade 3 to Grade 0, from Grade 2 to Grade 1, from Grade 2 to Grade 0 or from Grade 1 to Grade 0.

In the present application, after administration of the compound as shown in Formula I, the symptoms of gastrointestinal side effects in the subject may be eliminated. However, the case that the gastrointestinal side effects recur or deteriorate again after stopping the administration of the compound as shown in Formula I may not be excluded.

## Therapeutic method

The term "prevention" as used herein generally refers to the prevention of occurrence, recurrence, or spread of diseases or one or more symptoms thereof. In the context of the present application, the "prevention" may be interchangeably used with the "preventive treatment". In some embodiments, the "prevention" generally refers to the treatment of providing a patient suffering from the diseases or disorders as described in the present application with the medicament in accordance with the present application with or without administration of other medicaments as described in the present application prior to the occurrence of any symptoms. In some embodiments, patients with a family history of a particular disease can be the candidates of a prevention program. In some embodiments, patients with a history of recurring symptoms are also potential objects of prevention.

The term "treatment" as used herein generally refers to eliminating or improving diseases or one or more symptoms associated with the diseases. In some embodiments, the treatment generally refers to eliminating or ameliorating a disease by administering one or more therapeutic agents to the patients suffering from the disease. In some embodiments, the "treatment" may be administering a medicament in the presence or absence of other therapeutic

agent(s) after the occurrence of symptoms of a particular disease.

The term "subject" as used herein generally refers to a human or non-human animal (including mammals) in need of diagnosing, prognosing, improving, preventing, relieving and/or treating diseases, especially those in need of treatment, relief or prevention by using the compound as shown in Formula I. In some embodiments, the subject may comprise a cancer patient. For example, the cancer patient may have been, is being, and/or will be administered with the chemotherapy.

In some embodiments, the subject may be a human or a non-human mammal. The non-human mammals may include any mammalian species other than human, e.g., livestocks (e.g., cow, pig, sheep, chick, rabbit or horse), or rodents (e.g., rat and mouse), or primates (e.g., gorilla and monkey), or domestic animals (e.g., dog and cat). The "subject" may be male or female, and also may be at different ages.

The term "effective amount" as used herein generally refers to an amount of medicament capable of ameliorating or eliminating diseases or symptoms of the subject, or preventively inhibiting or prohibiting the occurrence of diseases or symptoms. The effective amount may be an amount of medicament capable of ameliorating one or more diseases or symptoms to some extent in the subject; an amount of medicament capable of partially or completely restoring one or more physiological or biochemical parameters associated with the causes of diseases or symptoms to normal; and/or an amount of medicament capable of reducing the possibility of the occurrence of diseases or symptoms.

In the present application, after the administration of the chemotherapy, for example, about 0.5 hrs, about 1 hr, about 2 hrs, about 3 hrs, about 4 hrs, about 5 hrs, about 6 hrs, about 7 hrs, about 8 hrs, about 9 hrs, about 10 hrs, about 11 hrs, about 12 hrs, about 13 hrs, about 14 hrs, about 15 hrs, about 16 hrs, about 17 hrs, about 18 hrs, about 19 hrs, about 20 hrs or longer before the administration of the chemotherapy, the medicament or the compound as shown in Formula I can be administered to prevent, relieve and/or treat the occurrence or deterioration of the gastrointestinal side effects. For example, the medicament or the compound as shown in Formula I is administered 0.5-12 hrs before the administration of the chemotherapy to prevent, relieve and/or treat the occurrence or deterioration of the gastrointestinal side effects.

In the present application, the administration site of the compound as shown in Formula I may be or not the occurrence site of cancer or the potential metastatic site of cancer. For example, the administration site may be or not the primary site of cancer. As another example, the administration site may be or not the metastasis site of cancer. For example, the metastasis site may comprise the occurrence site of cancer metastasis caused by lymphatic metastasis, vascular metastasis and/or implantation metastasis. In some embodiments, the metastasis site may comprise bone, brain, liver, stomach and/or lung. As another example, the administration site may be or not the recurrence site of cancer.

The compound as shown in Formula I of the present application may be administered in a manner known in the art, e.g., by injection (e.g., subcutaneous, intraperitoneal, intraarticular, intraarterial, intrathecal, intrasternal, intrathecal, intralesional, intracranial, intramuscular, intracutaneous and intravenous injection or infusion) or non-injection (e.g., oral, nasal, sublingual, vaginal, rectal, or topical administration). The compound as shown in Formula I of the present application may be administered in a form of a pharmaceutical combination or a kit. In some embodiments, the compound as shown in Formula I of the present application may be administered in the same administration route as that of the chemotherapy or in a different route.

In the present application, the medicament and/or the compound as shown in Formula I may be prepared for oral administration. For example, the medicament and/or the compound as shown in Formula I may be prepared as powders, tablets, granules, capsules, solution, emulsion and/or suspension.

In the present application, the medicament and/or the compound as shown in Formula I may be prepared for injection administration, e.g. administration through subcutaneous injection, intramuscular injection, intraperitoneal injection and/or intravenous injection. For example, the medicament and/or the compound as shown in Formula I may be prepared as injections.

In the present application, the dosage of the compound as shown in Formula I may be about 0.01-1000 mg/kg, for example, may be about 0.01-800 mg/kg, about 0.01-900 mg/kg, about 0.01-800 mg/kg, about 0.01-700 mg/kg, about 0.01-600 mg/kg, about 0.01-500 mg/kg, about 0.01-400 mg/kg, about 0.01-300 mg/kg, about 0.01-200 mg/kg, about 0.01-100 mg/kg, about 0.1-1000 mg/kg, about 1-1000 mg/kg, about 10-1000 mg/kg, about 50-1000 mg/kg, about 100-1000 mg/kg, about 0.1-800 mg/kg, about 1-600 mg/kg, about 10-500 mg/kg, about 10-400 mg/kg, about 15-300 mg/kg, about 50-250 mg/kg or about 50-200 mg/kg.

For example, the dosage of the compound as shown in Formula I in oral administration may be about 0.01-1000 mg/kg, for example, may be about 0.01-800 mg/kg, about 0.01-900 mg/kg, about 0.01-800 mg/kg, about 0.01-700 mg/kg, about 0.01-600 mg/kg, about 0.01-500 mg/kg, about 0.01-400 mg/kg, about 0.01-300 mg/kg, about 0.01-200 mg/kg, about 0.01-100 mg/kg, about 0.1-1000 mg/kg, about 1-1000 mg/kg, about 10-1000 mg/kg, about 50-1000 mg/kg, about 100-1000 mg/kg, about 0.1-800 mg/kg, about 1-600 mg/kg, about 10-500 mg/kg, about 10-400 mg/kg, about 15-300 mg/kg, about 50-250 mg/kg or about 50-200 mg/kg.

For example, the dosage of the compound as shown in Formula I in injection administration may be about 0.01-1000 mg/kg, for example, may be about 0.01-800 mg/kg, about 0.01-900 mg/kg, about 0.01-800 mg/kg, about

0.01-700 mg/kg, about 0.01-600 mg/kg, about 0.01-500 mg/kg, about 0.01-400 mg/kg, about 0.01-300 mg/kg, about 0.01-200 mg/kg, about 0.01-100 mg/kg, about 0.1-1000 mg/kg, about 1-1000 mg/kg, about 10-1000 mg/kg, about 50-1000 mg/kg, about 100-1000 mg/kg, about 0.1-800 mg/kg, about 1-600 mg/kg, about 10-500 mg/kg, about 10-400 mg/kg, about 15-300 mg/kg, about 50-250 mg/kg or about 50-200 mg/kg.

For example, the compound as shown in Formula I may be administered 0.5-24 h before the administration of the chemotherapeutic agent, may be administered orally, and the administration dosage may be about 50-150 mg/kg, the administration frequency may be once a week to once every 2 weeks (e.g., once every 7 days, once every 8 days, once every 9 days, once every 10 days, once every 11 days, once every 12 days, once every 13 days or once every 14 days).

For example, the compound as shown in Formula I may be administered 0.5-12 h before the administration of the chemotherapeutic agent, may be administered orally, and the administration dosage may be about 50-150 mg/kg, the administration cycle may be once a week to once every 2 weeks (e.g., once every 7 days, once every 8 days, once every 9 days, once every 10 days, once every 11 days, once every 12 days, once every 13 days or once every 14 days).

For example, the compound as shown in Formula I may be administered 0.5-24 h before the administration of the chemotherapeutic agent, may be administered by injection, and the administration dosage may be about 50-150 mg/kg, the administration frequency may be once a week to once every 2 weeks (e.g., once every 7 days, once every 8 days, once every 9 days, once every 10 days, once every 11 days, once every 12 days, once every 13 days or once every 14 days).

For example, the compound as shown in Formula I may be administered 0.5-12 h before the administration of the chemotherapeutic agent, may be administered by injection, and the administration dosage may be about 50-150 mg/kg, the administration cycle may be once a week to once every 2 weeks (e.g., once every 7 days, once every 8 days, once every 9 days, once every 10 days, once every 11 days, once every 12 days, once every 13 days or once every 14 days).

In the present application, the compound I and the pharmaceutically acceptable salt thereof (e.g., hydrochloride) can prevent, relieve and/or treat gastrointestinal side effects associated with fluorouracil in a subject. For example, the compound I and the pharmaceutically acceptable salt thereof may be administered 0.5-24 h before the administration of fluorouracil, the compound I and the pharmaceutically acceptable salt thereof may be administered by injection or orally, and the dosage of the compound I and the pharmaceutically acceptable salt thereof may be about 100 mg/kg-about 150 mg/kg.

For example, the compound I and the pharmaceutically acceptable salt thereof (e.g., hydrochloride) may be administered 0.5-12 h before the administration of fluorouracil, the compound I and the pharmaceutically acceptable salt thereof may be administered by intravenous injection, and the dosage of the compound I and the pharmaceutically acceptable salt thereof may be about 100 mg/kg-about 150 mg/kg.

For example, the compound I and the pharmaceutically acceptable salt thereof (e.g., hydrochloride) may be administered 0.5-12 h before the administration of fluorouracil, the compound I and the pharmaceutically acceptable salt thereof may be administered by intraperitoneal injection, and the dosage of the compound I and the pharmaceutically acceptable salt thereof may be about 100 mg/kg-about 150 mg/kg.

For example, the compound I and the pharmaceutically acceptable salt thereof (e.g., hydrochloride) may be administered 0.5-12 h before the administration of fluorouracil, the compound I and the pharmaceutically acceptable salt thereof may be administered by intramuscular injection, and the dosage of the compound I and the pharmaceutically acceptable salt thereof may be about 100 mg/kg-about 150 mg/kg.

For example, the compound I and the pharmaceutically acceptable salt thereof (e.g., hydrochloride) may be administered 0.5-12 h before the administration of fluorouracil, the compound I and the pharmaceutically acceptable salt thereof may be administered orally, and the dosage of the compound I and the pharmaceutically acceptable salt thereof may be about 100 mg/kg-about 150 mg/kg.

In the present application, the compound I and the pharmaceutically acceptable salt thereof (e.g., hydrochloride) can prevent, relieve and/or treat gastrointestinal side effects associated with oxaliplatin in a subject. For example, the compound I and the pharmaceutically acceptable salt thereof may be administered 0.5-24 h before the administration of oxaliplatin, the compound I and the pharmaceutically acceptable salt thereof may be administered by injection or orally, and the dosage of the compound I and the pharmaceutically acceptable salt thereof may be about 100 mg/kg-about 150 mg/kg.

For example, the compound I and the pharmaceutically acceptable salt thereof (e.g., hydrochloride) may be administered 0.5-12 h before the administration of oxaliplatin, the compound I and the pharmaceutically acceptable salt thereof may be administered by injection (e.g., intravenous injection or intraperitoneal injection), and the dosage of the compound I and the pharmaceutically acceptable salt thereof may be about 100 mg/kg-about 150 mg/kg.

In the present application, the compound I and the pharmaceutically acceptable salt thereof (e.g., hydrochloride) can prevent, relieve and/or treat gastrointestinal side effects associated with irinotecan in a subject. For example, the compound I and the pharmaceutically acceptable salt thereof may be administered 0.5-12 h before the administration

of irinotecan, the compound I and the pharmaceutically acceptable salt thereof may be administered by injection or orally, and the dosage of the compound I and the pharmaceutically acceptable salt thereof may be about 100 mg/kg-about 150 mg/kg.

In the present application, the compound I and the pharmaceutically acceptable salt thereof (e.g., hydrochloride) can prevent, relieve and/or treat gastrointestinal side effects associated with the combined administration of fluorouracil and irinotecan in a subject. For example, the compound I and the pharmaceutically acceptable salt thereof may be administered 0.5-12 h before the administration of fluorouracil and irinotecan, the compound I and the pharmaceutically acceptable salt thereof may be administered by injection or orally, and the dosage of the compound I and the pharmaceutically acceptable salt thereof may be about 100 mg/kg-about 150 mg/kg.

In the present application, the compound I and the pharmaceutically acceptable salt thereof (e.g., hydrochloride) can prevent, relieve and/or treat gastrointestinal side effects associated with the combined administration of fluorouracil, oxaliplatin, irinotecan and tetrahydrofolic acid in a subject. For example, the compound I and the pharmaceutically acceptable salt thereof may be administered 0.5-12 h before the administration of fluorouracil, oxaliplatin, irinotecan and tetrahydrofolic acid, the compound I and the pharmaceutically acceptable salt thereof may be administered by injection or orally, and the dosage of the compound I and the pharmaceutically acceptable salt thereof may be about 100 mg/kg-about 150 mg/kg.

In the present application, the compound I and the pharmaceutically acceptable salt thereof can prevent, relieve and/or treat gastrointestinal side effects associated with gemcitabine in a subject.

In the present application, the compound I and the pharmaceutically acceptable salt thereof can prevent, relieve and/or treat gastrointestinal side effects associated with the combined administration of gemcitabine and carboplatin in a subject.

In the present application, the compound IV and the pharmaceutically acceptable salt thereof (e.g., hydrochloride) can prevent, relieve and/or treat gastrointestinal side effects associated with irinotecan in a subject. For example, the compound IV and the pharmaceutically acceptable salt thereof may be administered 0.5-12 h before the administration of irinotecan, the compound IV and the pharmaceutically acceptable salt thereof may be administered by injection, and the dosage of the compound I and the pharmaceutically acceptable salt thereof may be about 100 mg/kg-about 150 mg/kg.

In the present application, the compound IV and the pharmaceutically acceptable salt thereof (e.g., hydrochloride) can prevent, relieve and/or treat gastrointestinal side effects associated with fluorouracil in a subject. For example, the compound IV and the pharmaceutically acceptable salt thereof may be administered 0.5-12 h before the administration of fluorouracil, the compound IV and the pharmaceutically acceptable salt thereof may be administered by injection or orally, and the dosage of the compound IV and the pharmaceutically acceptable salt thereof may be about 100 mg/kg-about 150 mg/kg.

In the present application, the compound IV and the pharmaceutically acceptable salt thereof (e.g., hydrochloride) can prevent, relieve and/or treat gastrointestinal side effects associated with the combined administration of fluorouracil and irinotecan in a subject. For example, the compound IV and the pharmaceutically acceptable salt thereof may be administered 0.5-12 h before the administration of fluorouracil and irinotecan, the compound IV and the pharmaceutically acceptable salt thereof may be administered by injection or orally, and the dosage of the compound IV and the pharmaceutically acceptable salt thereof may be about 100 mg/kg-about 150 mg/kg.

In the present application, the compound IV and the pharmaceutically acceptable salt thereof (e.g., hydrochloride) can prevent, relieve and/or treat gastrointestinal side effects associated with the combined administration of fluorouracil, oxaliplatin, irinotecan and tetrahydrofolic acid in a subject. For example, the compound IV and the pharmaceutically acceptable salt thereof may be administered 0.5-24 h before the administration of fluorouracil, oxaliplatin, irinotecan and tetrahydrofolic acid, the compound IV and the pharmaceutically acceptable salt thereof may be administered by injection or orally, and the dosage of the compound IV and the pharmaceutically acceptable salt thereof may be about 100 mg/kg-about 150 mg/kg.

In the present application, the compound IV and the pharmaceutically acceptable salt thereof can prevent, relieve and/or treat gastrointestinal side effects associated with topotecan in a subject.

In the present application, the compound IV and the pharmaceutically acceptable salt thereof can prevent, relieve and/or treat gastrointestinal side effects associated with the combined administration of gemcitabine and carboplatin in a subject.

In the present application, the compound II and the pharmaceutically acceptable salt thereof can prevent, relieve and/or treat gastrointestinal side effects associated with fluorouracil in a subject. For example, the compound II and the pharmaceutically acceptable salt thereof may be administered 0.5-24 h before the administration of fluorouracil, the compound II and the pharmaceutically acceptable salt thereof may be administered by injection (e.g., intraperitoneal injection or intramuscular injection), and the dosage of the compound II and the pharmaceutically acceptable salt thereof may be about 50 mg/kg-about 150 mg/kg.

In the present application, the compound II and the pharmaceutically acceptable salt thereof can prevent, relieve and/or treat gastrointestinal side effects associated with the combined administration of fluorouracil, oxaliplatin, irinotecan

and tetrahydrofolic acid in a subject. For example, the compound II and the pharmaceutically acceptable salt thereof may be administered 0.5-24 h before the combined administration of fluorouracil, oxaliplatin, irinotecan and tetrahydrofolic acid, the compound II and the pharmaceutically acceptable salt thereof may be administered orally or by injection, and the dosage of the compound II and the pharmaceutically acceptable salt thereof may be about 100 mg/kg-about 150 mg/kg.

In the present application, the compound II and the pharmaceutically acceptable salt thereof can prevent, relieve and/or treat gastrointestinal side effects associated with oxaliplatin in a subject. For example, the compound II and the pharmaceutically acceptable salt thereof may be administered 0.5-12 h before the administration of oxaliplatin, the compound II and the pharmaceutically acceptable salt thereof may be administered by injection, and the dosage of the compound II and the pharmaceutically acceptable salt thereof may be about 100 mg/kg-about 150 mg/kg.

In the present application, the compound II and the pharmaceutically acceptable salt thereof can prevent, relieve and/or treat gastrointestinal side effects associated with the combined administration of fluorouracil, oxaliplatin, irinotecan and tetrahydrofolic acid in a subject. For example, the compound II and the pharmaceutically acceptable salt thereof may be administered 0.5-24 h before the combined administration of fluorouracil, oxaliplatin, irinotecan and tetrahydrofolic acid, the compound II and the pharmaceutically acceptable salt thereof may be administered orally or by injection, and the dosage of the compound II and the pharmaceutically acceptable salt thereof may be about 100 mg/kg-about 150 mg/kg.

In the present application, the compound III and the pharmaceutically acceptable salt thereof can prevent, relieve and/or treat gastrointestinal side effects associated with oxaliplatin in a subject. For example, the compound III and the pharmaceutically acceptable salt thereof may be administered 0.5-12 h before the administration of oxaliplatin, the compound III and the pharmaceutically acceptable salt thereof may be administered orally or by injection, and the dosage of the compound III and the pharmaceutically acceptable salt thereof may be about 100 mg/kg-about 150 mg/kg.

In the present application, the compound III and the pharmaceutically acceptable salt thereof can prevent, relieve and/or treat gastrointestinal side effects associated with the combined administration of fluorouracil, oxaliplatin, irinotecan and tetrahydrofolic acid in a subject. For example, the compound III and the pharmaceutically acceptable salt thereof may be administered 0.5-24 h before the combined administration of fluorouracil, oxaliplatin, irinotecan and tetrahydrofolic acid, the compound III and the pharmaceutically acceptable salt thereof may be administered orally or by injection, and the dosage of the compound III and the pharmaceutically acceptable salt thereof may be about 150 mg/kg-about 200 mg/kg.

In the present application, the compound III and the pharmaceutically acceptable salt thereof can prevent, relieve and/or treat gastrointestinal side effects associated with fluorouracil in a subject. For example, the compound III and the pharmaceutically acceptable salt thereof may be administered 0.5-12 h before the administration of fluorouracil, the compound III and the pharmaceutically acceptable salt thereof may be administered orally or by injection, and the dosage of the compound III and the pharmaceutically acceptable salt thereof may be about 100 mg/kg-about 150 mg/kg.

In the present application, the medicament may further include one or more pharmaceutically acceptable carriers. The pharmaceutically acceptable carriers may include, but not limited to, e.g., pharmaceutically acceptable liquid, gel or solid carriers, aqueous medium, non-aqueous medium, antimicrobial substances, isotonic substances, buffer solution, antioxidants, anaesthetics, suspending agents/dispersing agents, chelating agents, emulsifiers, diluents, adjuvants, ingredients, non-toxic auxiliary substances, fillers, binders, disintegrants, buffer solution, preservatives, lubricants, flavoring agents, thickening agents, colorants, emulsifiers, other components well known in the art or various combinations of the above.

In another aspect, the present application provides a method of preventing, relieving and/or treating chemotherapy-associated gastrointestinal side effects with

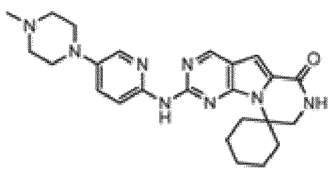

Compound I

or a pharmaceutically acceptable salt thereof. In some embodiments, the chemotherapy is fluorouracil, oxaliplatin, irinotecan (CPT-11), tetrahydrofolic acid, and a combination thereof. In some embodiments, the compound I is administered orally or by injection.

In another aspect, the present application provides a method of preventing, relieving and/or treating chemotherapy-associated gastrointestinal side effects with

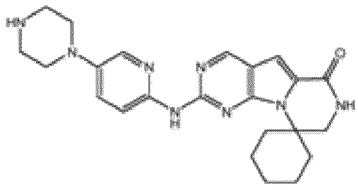

Compound II

or a pharmaceutically acceptable salt thereof. In some embodiments, the chemotherapy is fluorouracil, oxaliplatin, irinotecan (CPT-11), tetrahydrofolic acid, and a combination thereof. In some embodiments, the compound II is administered orally or by injection.

In another aspect, the present application provides a method of preventing, relieving and/or treating chemotherapy-associated gastrointestinal side effects with

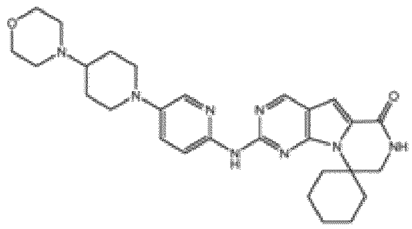

Compound III

or a pharmaceutically acceptable salt thereof. In some embodiments, the chemotherapy is fluorouracil, oxaliplatin, irinotecan (CPT-11), tetrahydrofolic acid, and a combination thereof. In some embodiments, the compound III is administered orally or by injection.

In another aspect, the present application provides a method of preventing, relieving and/or treating chemotherapy-associated gastrointestinal side effects with

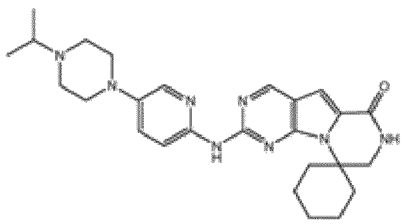

Compound IV

or a pharmaceutically acceptable salt thereof. In some embodiments, the chemotherapy is fluorouracil, oxaliplatin, irinotecan (CPT-11), tetrahydrofolic acid, and a combination thereof. In some embodiments, the compound IV is administered orally or by injection.

In another aspect, the present application provides a method of preventing, relieving and/or treating chemotherapy-associated gastrointestinal side effects with the compound as shown in Formula I. In another aspect, the present application provides a use of the compound as shown in Formula I in preparing a medicament for preventing, relieving and/or treating chemotherapy-associated gastrointestinal side effects. In another aspect, the present application provides the compound as shown in Formula I, which is used for preventing, relieving and/or treating chemotherapy-associated gastrointestinal side effects. In some embodiments, the continuous administration time of the chemotherapy is less than 7 days. In some embodiments, the chemotherapy is administered orally or by injection.

In another aspect, the present application provides a method of preventing, relieving and/or treating chemotherapy-associated diarrhea with the compound as shown in Formula I. In another aspect, the present application provides a use of the compound as shown in Formula I in preparing a medicament for preventing, relieving and/or treating chemotherapy-associated diarrhea. In another aspect, the present application provides the compound as shown in Formula I, which is used for preventing, relieving and/or treating chemotherapy-associated diarrhea. In some embodiments, the continuous administration time of the chemotherapy is less than 7 days. In some embodiments, the chemotherapy is administered orally or by injection.

In another aspect, the present application provides a method of preventing, relieving and/or treating chemotherapy-associated constipation with the compound as shown in Formula I. In another aspect, the present application provides a use of the compound as shown in Formula I in preparing a medicament for preventing, relieving and/or treating chemotherapy-associated constipation. In another aspect, the present application provides the compound as shown in Formula I, which is used for preventing, relieving and/or treating chemotherapy-associated constipation. In some embodiments, the continuous administration time of the chemotherapy is less than 7 days. In some embodiments, the chemotherapy is administered orally or by injection.

Without being limited by any theory, the following examples are only for the purpose of illustrating the compounds, preparation methods and uses of the present application, and not intended to limit the invention scope of the present application.

**Examples**

**Examples 1-201: Relieving effect of the compound as shown in Formula I on diarrhea caused by a chemotherapeutic agent**

Construction of mouse animal models: Chemotherapeutic agent-caused diarrhea models of Balb/c mice were constructed according to the administration mode and frequency of the chemotherapeutic agent as shown in Table 3. After administration for several days, mice developed different extents of diarrhea symptoms (as shown in FIG. 1), which were similar to the conditions in human. Thus, chemotherapeutic agent-caused diarrhea models of mice are very good models to mimic diarrhea in human caused by the chemotherapeutic agent.

Balb/c mice were fed for 1 week, and then divided into groups. The experiment was divided into a control group, a chemotherapeutic agent group, and a group of the compound as shown in Formula I, each of which comprised 10 mice, to perform the administration experiment, for which the administration dosage, mode, time and frequency were shown in Table 3. The control group: being injected or intragastrically administered with the same solvent as that used in the group of the compound as shown in Formula I (the injection mode and time were shown in Table 3), and then being injected or intragastrically administered with the same solvent as that used in the chemotherapeutic agent group (the administration mode was shown in Table 3); the chemotherapeutic agent group: being injected or intragastrically administered with the same solvent as that used in the group of the compound as shown in Formula I (the administration mode and time were shown in Table 3), and then being injected or intragastrically administered with the chemotherapeutic agent (the type, administration mode and dosage were shown in Table 3); the group of the compound as shown in Formula I: being injected or intragastrically administered with the compound as shown in Formula I (the administration mode and time were shown in Table 3), and then being administered with the chemotherapeutic agent (the administration mode and dosage were shown in Table 3).

Diarrhea observation

The scoring of diarrhea can refer to the method of Akinobu Kurita (Cancer Chemother Pharmacol 2000; 46:211-20.), Grade 0: normal feces; Grade 1: mild diarrhea, the stools is slightly wet and soft; Grade 2: moderate diarrhea, loose stools and mild perianal contamination; Grade 3: severe diarrhea, watery stools and accompanied by severe perianal staining (see FIG. 1). It is regarded as effective remission when the diarrhea grading of mice in the group of the compound as shown in Formula I is reduced compared with the average diarrhea grading of mice in the chemotherapeutic agent group.

The diarrhea grading of mice was observed and recorded every day. At the end of the experiment, the number of mice in the group of the compound as shown in Formula I whose diarrhea grading was lower than that of mice in the chemotherapeutic agent group was recorded. Table 3 lists various animal experiment combinations of chemotherapeutic agents and the compound as shown in Formula I, as well as the corresponding experimental results. (Relative remission rate%= the number of mice in the group of the compound as shown in Formula I whose diarrhea has been effectively relieved/the total number of mice in the group of the compound as shown in Formula I * 100%. The establishment rate of diarrhea model in the chemotherapeutic agent group was 50%-70%, that is, about 5-7 of 10 mice developed diarrhea during the experiment, and there were cases of individual mice death or not developing diarrhea.)

Table 3: Experimental Conditions and Results of Examples 1-201

| Exa mple No. | Chemoth erapeutic agent | Admi nistrat ion mode | Dosage (mg/kg) | Freq uenc y | Compo und as shown in Formul a I | Adminis tration time (before the chemoth erapeuti c agent) | Ad mini strat ion mod e | Dosa ge (mg/k g) | Freq uenc y | Expe rimen tal cycle (days ) | Relati ve remis sion rate (%) |
|---|---|---|---|---|---|---|---|---|---|---|---|
| 1 | Fluorour acil (5-Fu) | Intrap eriton eal injecti on | 125 | Singl e | Compo und I (Trilaci clib) | 0.5 h | Intra ven ous inje ctio n | 100 | Singl e | 10 | 70 |
| 2 | | | | | | 1 h | | | | | 70 |
| 3 | | | | | | 2 h | | | | | 60 |
| 4 | | | | | | 3 h | | | | | 60 |
| 5 | | | | | | 4 h | | | | | 60 |
| 6 | | | | | | 5 h | | | | | 40 |
| 7 | | | | | | 6 h | | | | | 40 |
| 8 | | | | | | 7 h | | | | | 40 |
| 9 | | | | | | 8 h | | | | | 50 |
| 10 | | | | | | 9 h | | | | | 40 |
| 11 | | | | | | 10 h | | | | | 50 |
| 12 | | | | | | 11 h | | | | | 40 |
| 13 | | | | | | 12 h | | 12.5 | | | 30 |
| 14 | | | | | | | | 25 | | | 40 |

(continued)

| Exa mple No. | Chemoth erapeutic agent | Admi nistrat ion mode | Dosage (mg/kg) | Freq uenc y | Compo und as shown in Formul a I | Administration time (before the chemoth erapeuti c agent) | Ad mini strat ion mod e | Dosa ge (mg/k g) | Freq uenc y | Expe rimen tal cycle (days ) | Relati ve remis sion rate (%) |
|---|---|---|---|---|---|---|---|---|---|---|---|
| 15 | | | | | | | | 50 | | | 60 |
| 16 | | | | | | | | 100 | | | 70 |
| 17 | | | | | | | | 150 | | | 50 |
| 18 | | | | | | | | 240 | | | 50 |
| 19 | | | | | | 13 h | | 100 | | | 60 |
| 20 | | | | | | 14 h | | | | | 60 |
| 21 | | | | | | 16 h | | | | | 60 |
| 22 | | | | | | 18 h | | | | | 50 |
| 23 | | | | | | 20 h | | | | | 50 |
| 24 | | | | | | 22 h | | | | | 40 |
| 25 | | | | | | 24 h | | | | | 40 |
| 26 | | | | | | 48 h | | | | | 0 |
| 27 | Fluorour acil (5-Fu) | Intrap eriton eal injecti on | 125 | Singl e | Compo und I hydroc hloride (Trilaci clib dihydr ochlori de) | 0.5 h | Intra ven ous inje ctio n | 100 | Singl e | 10 | 50 |
| 28 | | | | | | 2 h | | | | | 70 |
| 29 | | | | | | 4 h | | | | | 70 |
| 30 | | | | | | 12 h | | | | | 60 |
| 31 | Fluorour acil (5-Fu) | Intrap eriton eal injecti on | 125 | Singl e | Compo und II | 0.5 h | Intra perit onea l inje ctio n | 50 | Singl e | 10 | 30 |
| 32 | | | | | | 4 h | | 100 | | | 60 |
| 33 | | | | | | 12 h | | 150 | | | 40 |
| 34 | Fluorour | Intrav | 175 | Singl | compo | 0.5 h | Intra | 100 | Singl | 10 | 40 |

(continued)

| Exa mple No. | Chemoth erapeutic agent | Admi nistrat ion mode | Dosage (mg/kg) | Freq uenc y | Compo und as shown in Formul a I | Administration time (before the chemoth erapeuti c agent) | Ad mini strat ion mod e | Dosa ge (mg/k g) | Freq uenc y | Expe rimen tal cycle (days ) | Relati ve remis sion rate (%) |
|---|---|---|---|---|---|---|---|---|---|---|---|
| 35 | acil (5-Fu) | enous injecti on | | e | und I | 1 h | perit onea l inje ctio n | | e | | 40 |
| 36 | | | | | | 2 h | | | | | 30 |
| 37 | | | | | | 4 h | | | | | 30 |
| 38 | | | | | | 6 h | | | | | 50 |
| 39 | | | | | | 8 h | | | | | 60 |
| 40 | | | | | | 10 h | | | | | 50 |
| 41 | | | | | | 12 h | | | | | 40 |
| 42 | | | | | | 14 h | | | | | 30 |
| 43 | | | | | | 16 h | | | | | 40 |
| 44 | | | | | | 18 h | | | | | 50 |
| 45 | | | | | | 20 h | | | | | 50 |
| 46 | | | | | | 24 h | | | | | 40 |
| 47 | | | | | | 48 h | | | | | 0 |
| 48 | Fluorour acil (5-Fu) | Intrap eriton eal injecti on | 125 | Singl e | Compo und I hydroc hloride | 0.5 h | Intra perit onea l inje ctio n | 150 | Singl e | 10 | 30 |
| 49 | | | | | | 2 h | | | | | 50 |
| 50 | | | | | | 4 h | | | | | 60 |
| 51 | | | | | | 12 h | | | | | 40 |
| 52 | Fluorour acil (5-Fu) | Intrav enous injecti on | 175 | Singl e | Compo und I | 0.5 h | Oral adm inist ratio n | 100 | Singl e | 10 | 40 |
| 53 | | | | | | 4 h | | | | | 80 |
| 54 | | | | | | 12 h | | | | | 50 |
| 55 | | | | | | 24 h | | | | | 30 |
| 56 | Fluorour acil (5-Fu) | Oral admin istrati on | 250 | Singl e | Compo und I | 0.5 h | Intra mus cula r inje ctio n | 150 | Singl e | 8 | 60 |

(continued)

| Exa mple No. | Chemoth erapeutic agent | Admi nistrat ion mode | Dosage (mg/kg) | Freq uenc y | Compo und as shown in Formul a I | Administration time (before the chemoth erapeuti c agent) | Ad mini strat ion mod e | Dosa ge (mg/k g) | Freq uenc y | Expe rimen tal cycle (days ) | Relati ve remis sion rate (%) |
|---|---|---|---|---|---|---|---|---|---|---|---|
| 57 | Fluorour acil (5-Fu) | Intram uscula r injecti on | 125 | Singl e | Compo und I | 8 h | Intra perit onea l inje ctio n | 100 | Singl e | 10 | 40 |
| 58 | Oxaliplat in | Intrap eriton eal injecti on | 20 | Singl e | Compo und I | 0.5 h | Intra perit onea l inje ctio n | 100 | Singl e | 8 | 40 |
| 59 | | | | | | 1 h | | | | | 30 |
| 60 | | | | | | 2 h | | | | | 40 |
| 61 | | | | | | 3 h | | | | | 50 |
| 62 | | | | | | 4 h | | | | | 50 |
| 63 | | | | | | 5 h | | | | | 30 |
| 64 | | | | | | 6 h | | | | | 40 |
| 65 | | | | | | 7 h | | | | | 60 |
| 66 | | | | | | 8 h | | | | | 60 |
| 67 | | | | | | 9 h | | | | | 50 |
| 68 | | | | | | 10 h | | | | | 70 |
| 69 | | | | | | 11 h | | | | | 70 |
| 70 | | | | | | 12 h | | | | | 50 |
| 71 | | | | | | 14 h | | | | | 60 |
| 72 | | | | | | 16 h | | | | | 50 |
| 73 | | | | | | 18 h | | | | | 50 |
| 74 | | | | | | 20 h | | | | | 70 |
| 75 | | | | | | 22 h | | | | | 40 |
| 76 | | | | | | 24 h | | | | | 40 |
| 77 | | | | | | 48 h | | | | | 0 |

(continued)

| Exa mple No. | Chemoth erapeutic agent | Admi nistrat ion mode | Dosage (mg/kg) | Freq uenc y | Compo und as shown in Formul a I | Administration time (before the chemoth erapeuti c agent) | Ad mini strat ion mod e | Dosa ge (mg/k g) | Freq uenc y | Expe rimen tal cycle (days ) | Relati ve remis sion rate (%) |
|---|---|---|---|---|---|---|---|---|---|---|---|
| 78 | Oxaliplat in | Intrap eriton eal injecti on | 20 | Singl e | Compo und I hydroc hloride | 0.5 h | Intra perit onea l inje ctio n | 150 | Singl e | 8 | 20 |
| 79 | | | | | | 6 h | | | | | 60 |
| 80 | | | | | | 12 h | | | | | 40 |
| 81 | Oxaliplat in | Intrav enous injecti on | 17.5 | Singl e | Compo und I | 0.5 h | Intra ven ous inje ctio n | 150 | Singl e | 8 | 20 |
| 82 | | | | | | 4 h | | | | | 30 |
| 83 | | | | | | 8 h | | | | | 30 |
| 84 | | | | | | 12 h | | | | | 40 |
| 85 | | | | | | 16 h | | | | | 40 |
| 86 | | | | | | 20 h | | | | | 40 |
| 87 | | | | | | 24 h | | | | | 50 |
| 88 | | | | | | 48 h | | | | | 0 |
| 89 | Oxaliplat in | Intrav enous injecti on | 17.5 | Singl e | Compo und III | 1 h | Intra perit onea l inje ctio n | 100 | Singl e | 8 | 40 |
| 90 | | | | | | 4 h | | | | | 70 |
| 91 | | | | | | 8 h | | | | | 40 |
| 92 | Irinoteca n (CPT-11) | Intrap eriton eal injecti on | 240 | Singl e | Compo und I | 0.5 h | Intra ven ous inje ctio n | 100 | Singl e | 7 | 50 |
| 93 | | | | | | 1 h | | | | | 50 |
| 94 | | | | | | 2 h | | | | | 50 |
| 95 | | | | | | 3 h | | | | | 40 |
| 96 | | | | | | 4 h | | | | | 50 |
| 97 | | | | | | 5 h | | | | | 40 |

(continued)

| Exa mple No. | Chemoth erapeutic agent | Admi nistrat ion mode | Dosage (mg/kg) | Freq uenc y | Compo und as shown in Formul a I | Administration time (before the chemoth erapeuti c agent) | Ad mini strat ion mod e | Dosa ge (mg/k g) | Freq uenc y | Expe rimen tal cycle (days ) | Relati ve remis sion rate (%) |
|---|---|---|---|---|---|---|---|---|---|---|---|
| 98 | | | | | | 6 h | | | | | 40 |
| 99 | | | | | | 7 h | | | | | 70 |
| 100 | | | | | | 8 h | | | | | 70 |
| 101 | | | | | | 9 h | | | | | 60 |
| 102 | | | | | | 10 h | | | | | 40 |
| 103 | | | | | | 11 h | | | | | 60 |
| 104 | | | | | | 12 h | | | | | 60 |
| 105 | | | | | | 14 h | | | | | 50 |
| 106 | | | | | | 16 h | | | | | 70 |
| 107 | | | | | | 18 h | | | | | 70 |
| 108 | | | | | | 20 h | | | | | 40 |
| 109 | | | | | | 22 h | | | | | 40 |
| 110 | | | | | | 24 h | | | | | 30 |
| 111 | | | | | | 48 h | | | | | 0 |
| 112 | Irinoteca n (CPT-11) | Intrap eriton eal injecti on | 240 | Singl e | Compo und IV (Leroci clib) | 0.5 h | Intra perit onea l inje ctio n | 100 | Singl e | 11 | 60 |
| 113 | | | | | | 4h | | | | | 50 |
| 114 | | | | | | 8h | | | | | 30 |
| 115 | Irinoteca n (CPT-11) | Intrap eriton eal injecti on | 240 | Singl e | Compo und IV hydroc hloride (Leroci clibdih ydroch loride) | 0.5 h | Intra perit onea l inje ctio n | 100 | Singl e | 11 | 40 |
| 116 | | | | | | 4 h | | | | | 60 |
| 117 | | | | | | 12 h | | | | | 20 |
| 118 | Irinoteca n (CPT-11) | Oral admin istrati on | 300 | Singl e | Compo und I | 12 h | Intra perit onea l inje ctio n | 100 | Singl e | 11 | 50 |

(continued)

| Exa mple No. | Chemoth erapeutic agent | Admi nistrat ion mode | Dosage (mg/kg) | Freq uenc y | Compo und as shown in Formul a I | Administration time (before the chemoth erapeuti c agent) | Ad mini strat ion mod e | Dosa ge (mg/k g) | Freq uenc y | Expe rimen tal cycle (days ) | Relati ve remis sion rate (%) |
|---|---|---|---|---|---|---|---|---|---|---|---|
| 119 | Irinoteca n (CPT-11) | Intram uscula r injecti on | 250 | Singl e | Compo und I | 1 h | Intra mus cula r inje ctio n | 100 | Singl e | 7 | 70 |
| 120 | Irinoteca n (CPT-11) | Intrav enous injecti on | 200 | Singl e | Compo und I | 0.h | Intra ven ous inje ctio n | 100 | Singl e | 7 | 70 |
| 121 | Fluorour acil and irinoteca n | Intrav enous injecti on | 100 (F) 200 (I) | Singl e | Compo und I | 0.5 h | Intra ven ous inje ctio n | 100 | Singl e | 10 | 70 |
| 122 | | | | | | 4h | | | | | 40 |
| 123 | | | | | | 8 h | | | | | 50 |
| 124 | | | | | | 12 h | | | | | 50 |
| 125 | | | | | | 24 h | | | | | 30 |
| 126 | | | | | | 48 h | | | | | 0 |
| 127 | Fluorour acil and irinoteca n | Intrap eriton eal injection | 100 (F) 200 (I) | Singl e | Compo und I | 1 h | Intra perit onea linje ctio n | 100 | Singl e | 8 | 40 |
| 128 | | | | | | 12 h | | | | | 30 |
| 129 | Fluorour acil and irinoteca n | Intrap eriton eal injecti on | 100 (F) 200 (I) | Singl e | Compo und I | 0.5 h | Oral adm inist ratio n | 150 | Singl e | 8 | 40 |
| 130 | | | | | | 12 h | | | | | 40 |
| 131 | | | | | | 24 h | | | | | 30 |
| 132 | Fluorour acil and irinoteca n | Intrap eriton eal injecti on | 100 (F) 200 (I) | Singl e | Compo und I hydroc hloride | 0.5 h | Intra perit onea l inje ctio n | 150 | Singl e | 8 | 30 |
| 133 | | | | | | 6 h | | | | | 60 |
| 134 | | | | | | 12 h | | | | | 50 |
| 135 | Fluorour acil and irinoteca n | Intrap eriton eal injecti on | 100 (F) 200 (I) | Singl e | Compo und IV | 0.5 h | Oral adm inist ratio n | 100 | Singl e | 8 | 30 |
| 136 | | | | | | 12 h | | | | | 50 |
| 137 | | | | | | 24 h | | | | | 40 |

(continued)

| Exa mple No. | Chemoth erapeutic agent | Admi nistrat ion mode | Dosage (mg/kg) | Freq uenc y | Compo und as shown in Formul a I | Adminis tration time (before the chemoth erapeuti c agent) | Ad mini strat ion mod e | Dosa ge (mg/k g) | Freq uenc y | Expe rimen tal cycle (days ) | Relati ve remis sion rate (%) |
|---|---|---|---|---|---|---|---|---|---|---|---|
| 138 | Fluorour acil and oxaliplati n and irinoteca n | Intrap eriton eal injecti on | 50 (F) 5 (O) 50 (I) | Singl e | Compo und I | 0.5 h | Intra perit onea l inje ctio n | 100 | Singl e | 10 | 50 |
| 139 | | | | | | 4 h | | | | | 40 |
| 140 | | | | | | 8 h | | | | | 40 |
| 141 | | | | | | 12 h | | | | | 50 |
| 142 | | | | | | 24 h | | | | | 50 |
| 143 | | | | | | 48 h | | | | | 0 |
| 144 | Fluorour acil and oxaliplati | Intrav enous injecti | 50 (F) 5 (O) 50 (I) | Singl e | Compo und I | 0.5 h | Intra ven ous | 100 | Singl e | 10 | 50 |
| 145 | | | | | | 1 h | | | | | 40 |
| 146 | | | | | | 2 h | | | | | 40 |
| 147 | n and irinoteca n and tetrahydr ofolic acid | on | 90 (L) | | | 3 h | inje ctio n | | | | 30 |
| 148 | | | | | | 4 h | | | | | 40 |
| 149 | | | | | | 6 h | | | | | 40 |
| 150 | | | | | | 8 h | | | | | 30 |
| 151 | | | | | | 10 h | | | | | 30 |
| 152 | | | | | | 12 h | | | | | 30 |
| 153 | | | | | | 14 h | | | | | 50 |
| 154 | | | | | | 16 h | | | | | 50 |
| 155 | | | | | | 18 h | | | | | 60 |
| 156 | | | | | | 20 h | | | | | 60 |
| 157 | | | | | | 24 h | | | | | 30 |
| 158 | | | | | | 48 h | | | | | 0 |

(continued)

| Example No. | Chemotherapeutic agent | Administration mode | Dosage (mg/kg) | Frequency | Compound as shown in Formula I | Administration time (before the chemotherapeutic agent) | Administration mode | Dosage (mg/kg) | Frequency | Experimental cycle (days) | Relative remission rate (%) |
|---|---|---|---|---|---|---|---|---|---|---|---|
| 159 | Fluorouracil and oxaliplatin and irinotecan and tetrahydrofolic acid | Intraperitoneal injection | 50 (F) 5 (O) 50 (I) 90 (L) | Single | Compound I | 0.5 h | Intraperitoneal injection | 100 | Single | 7 | 50 |
| 160 | | | | | | 24 h | | | | | 20 |
| 161 | Fluorouracil and oxaliplatin and irinotecan and tetrahydrofolic acid | Intraperitoneal injection | 50 (F) 5 (O) 50 (I) 90 (L) | Single | Compound I hydrochloride | 0.5 h | Intraperitoneal injection | 150 | Single | 7 | 70 |
| 162 | | | | | | 12 h | | | | | 40 |
| 163 | Fluorouracil and oxaliplatin and irinotecan and tetrahydrofolic acid | Intraperitoneal injection | 50 (F) 5 (O) 50 (I) 90 (L) | Single | Compound I | 0.5 h | Oral administration | 150 | Single | 7 | 40 |
| 164 | | | | | | 4 h | | | | | 60 |
| 165 | | | | | | 8 h | | | | | 80 |
| 166 | | | | | | 12 h | | | | | 40 |
| 167 | | | | | | 24 h | | | | | 20 |
| 168 | Fluorouracil and oxaliplatin and irinotecan and tetrahydrofolic acid | Intraperitoneal injection | 50 (F) 5 (O) 50 (I) 90 (L) | Single | Compound I | 0.5 h | Oral administration | 150 | Single | 7 | 40 |
| 169 | | | | | | 4 h | | | | | 60 |
| 170 | | | | | | 8 h | | | | | 80 |
| 171 | | | | | | 12 h | | | | | 40 |
| 172 | | | | | | 24 h | | | | | 20 |
| 173 | Fluorouracil and oxaliplatin and irinotecan and tetrahydrofolic acid | Intraperitoneal injection | 50 (F) 5 (O) 50 (I) 90 (L) | Single | Compound II | 0.5 h | Oral administration | 100 | Single | 7 | 30 |
| 174 | | | | | | 4 h | | | | | 30 |
| 175 | | | | | | 8 h | | | | | 40 |
| 176 | | | | | | 12 h | | | | | 30 |
| 177 | | | | | | 24 h | | | | | 30 |

(continued)

| Exa mple No. | Chemoth erapeutic agent | Admi nistrat ion mode | Dosage (mg/kg) | Freq uenc y | Compo und as shown in Formul a I | Administration time (before the chemoth erapeuti c agent) | Ad mini strat ion mod e | Dosa ge (mg/k g) | Freq uenc y | Expe rimen tal cycle (days ) | Relati ve remis sion rate (%) |
|---|---|---|---|---|---|---|---|---|---|---|---|
| 178 | Fluorour acil and oxaliplati | Intrav enous injecti | 50 (F) | Singl e | Compo und III | 0.5 h | Oral adm inist | 150 | Singl e | 7 | 30 |
| 179 | | | 5 (O) | | | 4 h | | | | | 50 |
| 180 | | | 50 (I) | | | 8 h | | | | | 40 |
| 181 | n and irinoteca n and tetrahydr ofolic acid | on | 90 (L) | | | 12 h | ratio n | | | | 40 |
| 182 | | | | | | 24 h | | | | | 20 |
| 183 | Fluorour acil and oxaliplati n and irinoteca n and tetrahydr ofolic acid | Intrav enous injecti on | 50 (F) | Singl e | Compo und IV | 0.5 h | Intra perit onea l inje ctio n | 100 | Singl e | 7 | 80 |
| 184 | | | 5 (O) | | | 4 h | | | | | 60 |
| 185 | | | 50 (I) | | | 8 h | | | | | 50 |
| 186 | | | 90 (L) | | | 12 h | | | | | 50 |
| 187 | | | | | | 24 h | | | | | 40 |
| 188 | Fluorour acil and oxaliplati n and irinoteca n and tetrahydr ofolic acid | Intrav enous injecti on | 50 (F) | Singl e | Compo und IV hydroc hloride | 0.5 h | Intra perit onea l inje ctio n | 100 | Singl e | 7 | 70 |
| 189 | | | 5 (O) 50 (I) 90 (L) | | | 12 h | | | | | 50 |
| 190 | Fluorour acil and oxaliplati n and irinoteca n and tetrahydr ofolic acid | Oral admin istrati on | 100 (F) | Singl e | Compo und I | 1 h | subc utan eous inje ctio n | 100 | Singl e | 7 | 60 |
| 191 | | | 10 (O) | | | 2 h | | | | | 50 |
| 192 | | | 200 (I) | | | 4 h | | | | | 30 |
| 193 | | | 100 (L) | | | 6 h | | | | | 30 |
| 194 | Topoteca n | Intrap eriton eal injecti on | 10 | Singl e | Compo und I hydroc hloride | 4h | Oral adm inist ratio n | 100 | Singl e | 14 | 50 |
| 195 | Topoteca n | Intrav enous injecti on | 20 | Singl e | Compo und IV | 0.5 h | Oral adm inist ratio n | 100 | Singl e | 14 | 60 |

(continued)

| Exa mple No. | Chemoth erapeutic agent | Admi nistrat ion mode | Dosage (mg/kg) | Freq uenc y | Compo und as shown in Formul a I | Adminis tration time (before the chemoth erapeuti c agent) | Ad mini strat ion mod e | Dosa ge (mg/k g) | Freq uenc y | Expe rimen tal cycle (days ) | Relati ve remis sion rate (%) |
|---|---|---|---|---|---|---|---|---|---|---|---|
| 196 | Gemcita bine (GEM) | Intrap eriton eal injecti on | 120 | Singl e | Compo und I | 12h | Oral adm inist ratio n | 100 | Singl e | 8 | 50 |
| 197 | Gemcita bine (GEM) | Intrav enous injecti on | 120 | Singl e | Compo und II | 24 h | Oral adm inist ratio n | 100 | Singl e | 8 | 40 |
| 198 | Etoposid e and carboplat in | Intrap eriton eal injecti on | 60 (E) 5 (C) | Singl e | Compo und II | 2h | Oral adm inist ratio n | 100 | Singl e | 10 | 50 |
| 199 | Etoposid e and carboplat in | Intrap eriton eal injecti on | 60 (E) 5 (C) | Singl e | Compo und III | 0.5 h | Oral administ ratio n | 100 | Singl e | 10 | 60 |
| 200 | Gemcita bine and carboplat in | Intrap eriton eal injecti on | 120 | Singl e | Compo und I hydroc hloride | 4 h | Oral adm inist ratio n | 100 | Singl e | 8 | 70 |
| 201 | Gemcita bine and carboplat in | Intrav enous injecti on | 120 (G) 5 (C) | Singl e | Compo und IV hydroc hloride | 24 h | Oral adm inist ratio n | 100 | Singl e | 8 | 40 |

FIG. 1 shows typical photographs showing different diarrhea gradings of mice in the chemotherapeutic agent group in Table 3. FIG. 2 shows partial diarrhea grading results of the control group, the chemotherapeutic agent group, and the group of the compound as shown in Formula I in Table 3.

It can be seen from the results in Table 3 and FIG. 2 that, the diarrhea gradings in the groups of compounds I, II, IV and III or their hydrochlorides were relieved to different degrees compared with that in the chemotherapeutic agent group. Therefore, the administration of compounds I, II, IV and III or their hydrochlorides before the chemotherapeutic agent can effectively relieve diarrhea caused by the chemotherapeutic agent.

**Examples 202-364: Relieving effect of the compound as shown in Formula I on constipation caused by a chemotherapeutic agent**

Construction of mouse animal models: Chemotherapeutic agent-caused constipation models of Balb/c mice were constructed according to the administration mode and frequency of the chemotherapeutic agent as shown in Table 4. After administration for several days, mice showed decreased stool volume (as shown in FIG. 3), which was consistent with the symptoms of constipation caused by a chemotherapeutic agent in human. Thus, chemotherapeutic agent-caused constipation models of mice are very good models to mimic constipation caused by the chemotherapeutic agent.

Balb/c mice were fed for 1 week, and then divided into groups. The experiment was divided into a control group, a chemotherapeutic agent group, and a group of the compound as shown in Formula I, each of which comprised 10 mice, to perform the administration experiment, for which the administration dosage, mode, time and frequency were shown in Table 4. The control group: being injected or intragastrically administered with the same solvent as that used in the group of the compound as shown in Formula I (the administration mode and time were shown in Table 4), and then being injected or intragastrically administered with the same solvent as that used in the chemotherapeutic agent group (the administration mode was shown in Table 4); the chemotherapeutic agent group: being injected or intragastrically administered with the same solvent as that used in the group of the compound as shown in Formula I (the administration mode and time was shown in Table 4), and then being injected or intragastrically administered with the chemotherapeutic agent (the type, administration mode and dosage were shown in Table 4); the group of the compound as shown in Formula I: being injected or intragastrically administered with the compound as shown in Formula I (the administration mode and time was shown in Table 4), and then being administered with the chemotherapeutic agent (the administration mode and dosage were shown in Table 4).

Constipation observation

The feces of mice within 3 hours were collected, which were observed for shape and weighed with an electronic balance. The feces reduction rate was calculated with reference to the method of Ji Eun Kim (Lab Anim Res. 2016 Dec; 32(4): 231-240.). Feces reduction rate

(%) = (the control group - the chemotherapeutic agent group or the group of the compound as shown in Formula I)/the control group*100%

It is regarded as effective remission when the feces reduction rate of mice in the group of the compound as shown in Formula I is reduced compared with that in the chemotherapeutic agent group.

The feces amount of mice within 3 hours was observed and recorded every day. At the end of the experiment, the number of mice in the group of the compound as shown in Formula I whose feces reduction rate was lower than that of mice in the chemotherapeutic agent group was recorded. Table 4 lists various animal experiment combinations of chemotherapeutic agents and the compound as shown in Formula I, as well as the corresponding experimental results. (Relative remission rate%= the number of mice in the group of the compound as shown in Formula I which have been effectively relieved/the total number of mice in the group of the compound as shown in Formula I * 100%. The establishment rate of constipation model in the chemotherapeutic agent group was 30%-60%, that is, about 3-6 of 10 mice showed reduced feces amount during the experiment, and there were cases of individual mice death or with no changes or an increasement in the feces amount.)

Table 4: Experimental Conditions and Results of Examples 202-364

| Exa mple No. | Chemoth erapeutic agent | Admi nistra tion mode | Dosage (mg/kg) | Fr eq ue nc y | Compou nd as shown in Formula I | Administr ation time (before the chemothe rapeutic agent) | Admi nistrat ion mode | Dos age (mg /kg) | Freq uenc y | Experi mental cycle (days) | Rel ativ e rem issi on rate (%) |
|---|---|---|---|---|---|---|---|---|---|---|---|
| 202 | Fluorour acil (5-Fu) | Intra perito neal inject ion | 175 | Si ng le | Compou nd I | 0.5 h | Intrav enous injecti on | 100 | 8 | 10 | 40 |
| 203 | | | | | | 1 h | | | | | 30 |
| 204 | | | | | | 2 h | | | | | 40 |
| 205 | | | | | | 3 h | | | | | 40 |
| 206 | | | | | | 4 h | | | | | 30 |
| 207 | | | | | | 5 h | | | | | 20 |
| 208 | | | | | | 6 h | | | | | 50 |
| 209 | | | | | | 7 h | | | | | 50 |
| 210 | | | | | | 8 h | | | | | 40 |
| 211 | | | | | | 9 h | | | | | 40 |
| 212 | | | | | | 10 h | | | | | 30 |
| 213 | | | | | | 11 h | | | | | 50 |

(continued)

| Example No. | Chemotherapeutic agent | Administration mode | Dosage (mg/kg) | Frequency | Compound as shown in Formula I | Administration time (before the chemotherapeutic agent) | Administration mode | Dosage (mg/kg) | Frequency | Experimental cycle (days) | Relative remission rate (%) |
|---|---|---|---|---|---|---|---|---|---|---|---|
| 214 | | | | | | 12 h | | 12.5 | | | 20 |
| 215 | | | | | | | | 25 | | | 30 |
| 216 | | | | | | | | 50 | | | 30 |
| 217 | | | | | | | | 100 | | | 40 |
| 218 | | | | | | | | 150 | | | 60 |
| 219 | | | | | | | | 240 | | | 60 |
| 220 | | | | | | 13 h | | 100 | | | 40 |
| 221 | | | | | | 14 h | | | | | 40 |
| 222 | | | | | | 16 h | | | | | 50 |
| 223 | | | | | | 18 h | | | | | 60 |
| 224 | | | | | | 20 h | | | | | 60 |
| 225 | | | | | | 22 h | | | | | 30 |
| 226 | | | | | | 24 h | | | | | 30 |
| 227 | | | | | | 48 h | | | | | 0 |

(continued)

| Exa mple No. | Chemoth erapeutic agent | Admi nistra tion mode | Dosage (mg/kg) | Fr eq ue nc y | Compou nd as shown in Formula I | Administr ation time (before the chemothe rapeutic agent) | Admi nistrat ion mode | Dos age (mg /kg) | Freq uenc y | Experi mental cycle (days) | Rel ativ e rem issi on rate (%) |
|---|---|---|---|---|---|---|---|---|---|---|---|
| 228 | Fluorour acil (5-Fu) | Intra veno us inject ion | 250 | Si ng le | Compou nd I | 0.5 h | Intrap eriton eal injecti on | 100 | Singl e | 10 | 30 |
| 229 | | | | | | 1 h | | | | | 40 |
| 230 | | | | | | 2 h | | | | | 40 |
| 231 | | | | | | 4 h | | | | | 60 |
| 232 | | | | | | 6 h | | | | | 60 |
| 233 | | | | | | 8 h | | | | | 50 |
| 234 | | | | | | 10 h | | | | | 50 |
| 235 | | | | | | 12 h | | | | | 70 |
| 236 | | | | | | 14 h | | | | | 40 |
| 237 | | | | | | 16 h | | | | | 40 |
| 238 | | | | | | 18 h | | | | | 50 |
| 239 | | | | | | 20 h | | | | | 50 |
| 240 | | | | | | 24 h | | | | | 50 |
| 241 | | | | | | 48 h | | | | | 0 |
| 242 | Fluorour | Intra | 250 | Si | Compou | 0.5 h | Intrap | 150 | Singl | 10 | 30 |
| 243 | acil (5-Fu) | veno us inject ion | | ng le | nd I hydroch loride | 4 h | eriton eal injecti on | | e | | 50 |
| 244 | | | | | | 8 h | | | | | 70 |
| 245 | | | | | | 12 h | | | | | 60 |
| 246 | Fluorour acil (5-Fu) | Intra veno us inject ion | 250 | Si ng le | Compou nd IV | 0.5 h | Oral admin istrati on | 100 | Singl e | 10 | 30 |
| 247 | | | | | | 4 h | | | | | 60 |
| 248 | | | | | | 12 h | | | | | 20 |
| 249 | Fluorour acil (5-Fu) | Oral admi nistra tion | 300 | Si ng le | Compou nd I | 0.5 h | Intra muse ular injecti on | 100 | Singl e | 7 | 60 |

(continued)

| Exa mple No. | Chemoth erapeutic agent | Admi nistra tion mode | Dosage (mg/kg) | Fr eq ue nc y | Compou nd as shown in Formula I | Administr ation time (before the chemothe rapeutic agent) | Admi nistrat ion mode | Dos age (mg /kg) | Freq uenc y | Experi mental cycle (days) | Rel ativ e rem issi on rate (%) |
|---|---|---|---|---|---|---|---|---|---|---|---|
| 250 | Fluorour acil (5-Fu) | Intra muse ular inject ion | 175 | Si ng le | Compou nd I | 8 h | Oral admin istrati on | 150 | Singl e | 7 | 50 |
| 251 | Oxaliplat in | Intra perito neal inject ion | 25 | Si ng le | Compou nd I | 0.5 h | Intrap eriton eal injecti on | 100 | Singl e | 10 | 20 |
| 252 | | | | | | 1 h | | | | | 20 |
| 253 | | | | | | 2 h | | | | | 40 |
| 254 | | | | | | 3 h | | | | | 40 |
| 255 | | | | | | 4 h | | | | | 50 |
| 256 | | | | | | 5 h | | | | | 50 |
| 257 | | | | | | 6 h | | | | | 60 |
| 258 | | | | | | 7 h | | | | | 60 |
| 259 | | | | | | 8 h | | | | | 30 |
| 260 | | | | | | 9 h | | | | | 40 |
| 261 | | | | | | 10 h | | | | | 40 |
| 262 | | | | | | 11 h | | | | | 50 |
| 263 | | | | | | 12 h | | | | | 50 |
| 264 | | | | | | 14 h | | | | | 30 |
| 265 | | | | | | 16 h | | | | | 40 |
| 266 | | | | | | 18 h | | | | | 60 |
| 267 | | | | | | 20 h | | | | | 30 |
| 268 | | | | | | 22 h | | | | | 30 |
| 269 | | | | | | 24 h | | | | | 40 |
| 270 | | | | | | 48 h | | | | | 0 |

(continued)

| Exa mple No. | Chemoth erapeutic agent | Admi nistra tion mode | Dosage (mg/kg) | Fr eq ue nc y | Compou nd as shown in Formula I | Administr ation time (before the chemothe rapeutic agent) | Admi nistrat ion mode | Dos age (mg /kg) | Freq uenc y | Experi mental cycle (days) | Rel ativ e rem issi on rate (%) |
|---|---|---|---|---|---|---|---|---|---|---|---|
| 271 | Oxaliplat in | Intra perito neal inject ion | 25 | Si ng le | Compou nd I hydroch loride | 0.5 h | Intrap eriton eal injecti on | 150 | Singl e | 10 | 30 |
| 272 | | | | | | 4 h | | | | | 40 |
| 273 | | | | | | 8 h | | | | | 50 |
| 274 | | | | | | 12 h | | | | | 50 |
| 275 | Oxaliplat in | Intra veno us inject ion | 25 | Si ng le | Compou nd I | 0.5 h | Intrav enous injecti on | 150 | Singl e | 7 | 30 |
| 276 | | | | | | 4 h | | | | | 50 |
| 277 | | | | | | 8 h | | | | | 50 |
| 278 | | | | | | 12 h | | | | | 40 |
| 279 | | | | | | 16 h | | | | | 40 |
| 280 | | | | | | 20 h | | | | | 60 |
| 281 | | | | | | 24 h | | | | | 60 |
| 282 | | | | | | 48 h | | | | | 0 |
| 283 | Oxaliplat in | Intra veno us inject ion | 25 | Si ng le | Compou nd II | 1 h | Intra muse ular injecti on | 100 | Singl e | 7 | 50 |
| 284 | | | | | | 6 h | | | | | 30 |
| 285 | | | | | | 12 h | | | | | 30 |
| 286 | Irinoteca n | Intra perito | 260 | Si ng | Compou nd I | 0.5 h | Intrav enous | 150 | Singl e | 8 | 40 |
| 287 | | | | | | 1 h | | | | | 40 |

(continued)

| Example No. | Chemotherapeutic agent | Administration mode | Dosage (mg/kg) | Frequency | Compound as shown in Formula I | Administration time (before the chemotherapeutic agent) | Administration mode | Dosage (mg /kg) | Frequency | Experimental cycle (days) | Relative remission rate (%) |
|---|---|---|---|---|---|---|---|---|---|---|---|
| 288 | (CPT-11) | neal injection | | le | | 2 h | injection | | | | 50 |
| 289 | | | | | | 3 h | | | | | 50 |
| 290 | | | | | | 4 h | | | | | 40 |
| 291 | | | | | | 5 h | | | | | 30 |
| 292 | | | | | | 6 h | | | | | 40 |
| 293 | | | | | | 7 h | | | | | 50 |
| 294 | | | | | | 8 h | | | | | 60 |
| 295 | | | | | | 9 h | | | | | 70 |
| 296 | | | | | | 10 h | | | | | 50 |
| 297 | | | | | | 11 h | | | | | 50 |
| 298 | | | | | | 12 h | | | | | 40 |
| 299 | | | | | | 14 h | | | | | 40 |
| 300 | | | | | | 16 h | | | | | 40 |
| 301 | | | | | | 18 h | | | | | 50 |
| 302 | | | | | | 20 h | | | | | 50 |
| 303 | | | | | | 22 h | | | | | 40 |
| 304 | | | | | | 24 h | | | | | 40 |
| 305 | | | | | | 48 h | | | | | 0 |
| 306 | Irinotecan (CPT-11) | Oral administration | 300 | Single | Compound I | 24 h | Intraperitoneal injection | 100 | Single | 10 | 60 |
| 307 | Irinotecan (CPT-11) | Intramuseular injection | 260 | Single | Compound I | 1 h | Intramuseular injection | 100 | Single | 7 | 60 |

(continued)

| Exa mple No. | Chemoth erapeutic agent | Admi nistra tion mode | Dosage (mg/kg) | Fr eq ue nc y | Compou nd as shown in Formula I | Administr ation time (before the chemothe rapeutic agent) | Admi nistrat ion mode | Dos age (mg /kg) | Freq uenc y | Experi mental cycle (days) | Rel ativ e rem issi on rate (%) |
|---|---|---|---|---|---|---|---|---|---|---|---|
| 308 | Irinotecan (CPT-11) | Intraveno us inject ion | 260 | Si ng le | Compound I | 12 h | Oraladmin istrati on | 100 | Single | 7 | 50 |
| 309 | Fluorour acil and irinoteca n | Intraveno us inject ion | 125 (F) 240 (I) | Si ng le | Compou nd I | 0.5 h | Intrav enous injecti on | 100 | Singl e | 10 | 40 |
| 310 | | | | | | 4 h | | | | | 60 |
| 311 | | | | | | 8 h | | | | | 60 |
| 312 | | | | | | 12 h | | | | | 70 |
| 313 | | | | | | 24 h | | | | | 50 |
| 314 | | | | | | 48 h | | | | | 0 |
| 315 | Fluorour acil and irinoteca n | Intra perito neal inject ion | 125 (F) 240 (I) | Si ng le | Compou nd I hydroch loride | 0.5 h | Intrap eriton eal injecti on | 150 | Singl e | 10 | 20 |
| 316 | | | | | | 4 h | | | | | 60 |
| 317 | | | | | | 12 h | | | | | 40 |
| 318 | Fluorour acil and irinoteca n | Intra perito neal inject ion | 125 (F) 240 (I) | Si ng le | Compou nd I | 1 h | Intrap eriton eal injecti on | 100 | Singl e | 7 | 40 |
| 319 | | | | | | 12 h | | | | | 50 |
| 320 | Fluorour acil and irinoteca n | Intra perito neal inject ion | 125 (F) 240 (I) | Si ng le | Compou nd III | 0.5 h | subcu taneo us injecti on | 100 | Singl e | 7 | 50 |
| 321 | | | | | | 24 h | | | | | 20 |
| 322 | Fluorour acil and oxaliplati n and irinoteca | Intra perito neal inject ion | 100 (F) 5 (O) 100 (I) | Si ng le | Compou nd I | 0.5 h | Intrap eriton eal injecti on | 300 | Singl e | 8 | 30 |
| 323 | | | | | | 4 h | | | | | 30 |
| 324 | | | | | | 8 h | | | | | 60 |
| 325 | | | | | | 12 h | | | | | 50 |
| 326 | | | | | | 24 h | | | | | 40 |

(continued)

| Exa mple No. | Chemoth erapeutic agent | Admi nistra tion mode | Dosage (mg/kg) | Fr eq ue nc y | Compou nd as shown in Formula I | Administr ation time (before the chemothe rapeutic agent) | Admi nistrat ion mode | Dos age (mg /kg) | Freq uenc y | Experi mental cycle (days) | Rel ativ e rem issi on rate (%) |
|---|---|---|---|---|---|---|---|---|---|---|---|
| 327 | n | | | | | 48 h | | | | | 0 |
| 328 | Fluorour acil and oxaliplati n and irinoteca n and tetrahydr ofolic acid | Intra veno us inject ion | 100 (F) 5 (O) 100(1) 100 (L) | Si ng le | Compou nd I | 0.5 h | Intrav enous injecti on | 100 | Singl e | 8 | 20 |
| 329 | | | | | | 1 h | | | | | 30 |
| 330 | | | | | | 2 h | | | | | 30 |
| 331 | | | | | | 3 h | | | | | 50 |
| 332 | | | | | | 4 h | | | | | 50 |
| 333 | | | | | | 6 h | | | | | 40 |
| 334 | | | | | | 8 h | | | | | 60 |
| 335 | | | | | | 10 h | | | | | 60 |
| 336 | | | | | | 12 h | | | | | 60 |
| 337 | | | | | | 14 h | | | | | 40 |
| 338 | | | | | | 16 h | | | | | 40 |
| 339 | | | | | | 18 h | | | | | 40 |
| 340 | | | | | | 20 h | | | | | 30 |
| 341 | | | | | | 24 h | | | | | 30 |
| 342 | | | | | | 48 h | | | | | 0 |
| 343 | Fluorour acil and oxaliplati n and irinoteca n and tetrahydr ofolic acid | Intra veno us inject ion | 100 (F) 5 (O) 100 (I) 100 (L) | Si ng le | Compou nd I hydroch loride | 0.5 h | Intrap eriton eal injecti on | 150 | Singl e | 8 | 30 |
| 344 | | | | | | 12 h | | | | | 70 |
| 345 | Fluorour acil and oxaliplati n and irinoteca n and tetrahydr ofolic acid | Intra perito neal injection | 100 (F) 5 (O) 100 (I) 100 (L) | Si ng le | Compou nd I | 6 h | Oral admin istrati on | 100 | Singl e | 7 | 60 |
| 346 | | | | | | 24 h | | | | | 40 |

(continued)

| Exa mple No. | Chemoth erapeutic agent | Admi nistra tion mode | Dosage (mg/kg) | Fr eq ue nc y | Compou nd as shown in Formula I | Administr ation time (before the chemothe rapeutic agent) | Admi nistrat ion mode | Dos age (mg /kg) | Freq uenc y | Experi mental cycle (days) | Rel ativ e rem issi on rate (%) |
|---|---|---|---|---|---|---|---|---|---|---|---|
| 347 | Fluorour acil and oxaliplati n and irinoteca n and tetrahydr ofolic acid | Intra perito neal inject ion | 100 (F) 5 (O) 100 (I) 100 (L) | Si ng le | Compou nd II | 0.5 h | Intrav enous injecti on | 100 | Singl e | 7 | 70 |
| 348 | | | | | | 2 h | | | | | 50 |
| 349 | Fluorour acil and oxaliplati n and irinoteca n and tetrahydr ofolic acid | Intra perito neal inject ion | 100 (F) 5 (O) 100 (I) 100 (L) | Si ng le | Compou nd III | 6 h | Oral admin istrati on | 150 | Singl e | 7 | 30 |
| 350 | | | | | | 12 h | | | | | 30 |
| 351 | Fluorour acil and oxaliplati n and irinoteca n and tetrahydr ofolic acid | Intra perito neal inject ion | 100 (F) 5 (O) 100 (I) 100 (L) | Si ng le | Compou nd IV | 4 h | Oral admin istrati on | 100 | Singl e | 7 | 60 |
| 352 | | | | | | 24 h | | | | | 30 |
| 353 | Fluorour acil and oxaliplati n and irinoteca n and tetrahydr ofolic acid | Intra perito neal inject ion | 100 (F) 5 (O) 100 (I) 100 (L) | Si ng le | Compou nd IV hydroch loride | 0.5 h | Intrap eriton eal injecti on | 100 | Singl e | 7 | 20 |
| 354 | | | | | | 12 h | | | | | 60 |
| 355 | Fluorour acil and oxaliplati n and irinoteca n and tetrahydr ofolic acid | Oral admi nistra tion | 100 (F) 5 (O) 200 (I) 100 (L) | Si ng le | Compou nd I | 0.5 h | Subcu taneo us injecti on | 100 | Singl e | 7 | 50 |
| 356 | | | | | | 4 h | | | | | 50 |
| 357 | Topoteca n | Intra perito neal inject ion | 10 | Si ng le | Compou nd IV hydroch loride | 24 h | Oral admin istrati on | 100 | Singl e | 14 | 30 |
| 358 | Topoteca n | Intra veno us inject ion | 20 | Si ng le | Compou nd IV hydroch loride | 12 h | Oral admin istrati on | 100 | Singl e | 14 | 70 |

(continued)

| Example No. | Chemotherapeutic agent | Administration mode | Dosage (mg/kg) | Frequency | Compound as shown in Formula I | Administration time (before the chemotherapeutic agent) | Administration mode | Dosage (mg/kg) | Frequency | Experimental cycle (days) | Relative remission rate (%) |
|---|---|---|---|---|---|---|---|---|---|---|---|
| 359 | Gemcitabine (GEM) | Intraperitoneal injection | 120 | Single | Compound II | 12 h | Oral administration | 100 | Single | 8 | 40 |
| 360 | Gemcitabine (GEM) | Intravenous injection | 120 | Single | Compound I | 24 h | Oral administration | 100 | Single | 8 | 30 |
| 361 | Etoposide and carboplatin | Intraperitoneal injection | 60 (E) 5 (C) | Single | Compound II | 2 h | Oral administration | 100 | Single | 10 | 60 |
| 362 | Etoposide and carboplatin | Intraperitoneal injection | 60 (E) 5 (C) | Single | Compound III | 0.5 h | Oral administration | 100 | Single | 10 | 50 |
| 363 | Gemcitabine and carboplatin | Intraperitoneal injection | 120 | Single | Compound IV | 4 h | Oral administration | 100 | Single | 8 | 60 |
| 364 | Gemcitabine and carboplatin | Intravenous injection | 120 (G) 5 (C) | Single | Compound II | 24 h | Oral administration | 100 | Single | 8 | 30 |

FIG. 3 shows typical photographs showing the feces amount within 3 hours in the control group, the chemotherapeutic agent group, and the group of the compound as shown in Formula I in Table 4. FIG. 4 shows partial results of feces reduction rate in the control group, the chemotherapeutic agent group, and the group of the compound as shown in Formula I in Table 4.

It can be seen from the results in Table 4 and FIG. 4 that, the feces reduction rates in the groups of compound I, II, IV and III, or their hydrochlorides were reduced to different degrees compared with that in the chemotherapeutic agent group. Therefore, the administration of the compound I, II, IV and III, or their hydrochlorides before the chemotherapeutic agent can effectively relieve constipation caused by the chemotherapeutic agent.

**Examples 365-384: Relieving effect of the compound as shown in Formula I on diarrhea caused by the continuous administration of a chemotherapeutic agent**

Balb/c mice were fed for 1 week, and then divided into groups. The experiment was divided into a control group, a chemotherapeutic agent group, and a group of the compound as shown in Formula I, each of which comprised 10 mice, to perform the administration experiment, for which the administration dosage, mode, time and frequency were shown in Table 5. The control group: being injected or intragastrically administered with the same solvent as that used in the group of the compound as shown in Formula I (the administration mode and time were shown in Table 5), and then being injected or intragastrically administered with the same solvent as that used in the chemotherapeutic agent group (the administration mode was shown in Table 5); the chemotherapeutic agent group: being injected or intragastrically administered with the same solvent as that used in the group of the compound as shown in Formula I (the administration mode and time were shown in Table 5), and then being injected or intragastrically administered with the chemotherapeutic agent (the type, administration mode, time and dosage were shown in Table 5); the group of the compound as shown in Formula I: being injected or intragastrically administered with the compound as shown in Formula I (the administration mode and time were shown in Table 5), and then being administered with the chemotherapeutic agent (the type, administration mode, time and dosage were shown in Table 5).

**Diarrhea observation**

The scoring of diarrhea can refer to the method of Akinobu Kurita (Cancer Chemother Pharmacol 2000; 46:211-20.), Grade 0: normal feces; Grade 1: mild diarrhea, the stools is slightly wet and soft; Grade 2: moderate diarrhea, loose stools and mild perianal contamination; Grade 3: severe diarrhea, watery stools and accompanied by severe perianal staining (see FIG. 1). It is regarded as effective remission when the diarrhea grading of mice in the group of the compound as shown in Formula I is reduced compared with the average diarrhea grading of mice in the chemotherapeutic agent group.

The diarrhea grading of mice was observed and recorded every day. At the end of the experiment, the number of mice in the group of the compound as shown in Formula I whose diarrhea grading was lower than that of mice in the chemotherapeutic agent group was recorded. Table 5 lists various animal experiment combinations of chemotherapeutic agents and the compound as shown in Formula I, as well as the corresponding experimental results. (Relative remission rate%= the number of mice in the group of the compound as shown in Formula I whose diarrhea has been effectively relieved/the total number of mice in the group of the compound as shown in Formula I * 100%. The establishment rate of diarrhea model in the chemotherapeutic agent group was 50%-80%, that is, about 5-8 of 10 mice developed diarrhea during the experiment, and there were cases of individual mice death or not developing diarrhea.)

Table 5: Experimental Conditions and Results of Examples 365-384

| Exa mpl e No. | Chemoth erapeutic agent | Admini stration mode | Dos age (mg /kg) | Freq uenc y | Compo und as shown in Formal a I | Administ ration time (before the chemoth erapeutic agent) | Admini stration mode | Dos age (mg /kg) | Freq uenc y | Experi mental cycle (days) | Relat ive remis sion rate (%) |
|---|---|---|---|---|---|---|---|---|---|---|---|
| 365 | Fluorour acil (5-Fu) | Intraper itoneal injectio n | 70 | Once a day, for 3 days | Compo und I | 2 h | Intrave nous injectio n | 100 | Once a day, for 3 days | 10 | 60 |
| 366 | | | | | | 4 h | | | | | 40 |
| 367 | | | | | | 6 h | | | | | 30 |
| 368 | | | | | | 8 h | | | | | 20 |
| 369 | Fluorour | Intraper | 70 | Once | Compo | 2 h | Intraper | 100 | Once | 9 | 50 |
| 370 | acil (5-Fu) | itoneal injectio n | | a day, for 5 days | und I | 4 h | itoneal injectio n | | a day, for 5 days | | 30 |
| 371 | | | | | | 6 h | | | | | 20 |
| 372 | | | | | | 8 h | | | | | 10 |
| 373 | Fluorour acil (5-Fu) | Intraper itoneal injectio n | 70 | Once a day, for 5 days | Compo und I hydroc hloride | 2 h | Intraper itoneal injectio n | 150 | Singl e | 9 | 60 |
| 374 | | | | | | 4 h | | | | | 30 |
| 375 | | | | | | 8 h | | | | | 10 |
| 376 | Fluorour acil (5-Fu) | Intraper itoneal injectio n | 70 | Once a day, for 7 days | Compo und I | 2 h | Intraper itoneal injectio n | 125 | Once a day, for 7 days | 10 | 10 |
| 377 | | | | | | 4 h | | | | | |
| 378 | | | | | | 6 h | | | | | |
| 379 | | | | | | 8 h | | | | | |
| 380 | Fluorour acil (5-Fu) | Intraper itoneal injectio n | 70 | Once a day, for 3 days | Compo und IV | 4 h | Oral adminis tration | 100 | Once a day, for 3 days | 8 | 30 |
| 381 | Fluorour acil (5-Fu) | Intrave nous injectio n | 70 | Once a day, for 3 days | Compo und II | 0.5 h | Intramu scular injectio n | 100 | Once a day, for 3 days | 8 | 40 |
| 382 | Fluorour acil (5-Fu) | Intrave nous injectio n | 70 | Once a day, for 3 days | Compo und III | 12 h | subcuta neous injectio n | 100 | Once a day, for 3 days | 8 | 20 |

(continued)

| Exa mpl e No. | Chemoth erapeutic agent | Admini stration mode | Dos age (mg /kg) | Freq uenc y | Compo und as shown in Formal a I | Administ ration time (before the chemoth erapeutic agent) | Admini stration mode | Dos age (mg /kg) | Freq uenc y | Experi mental cycle (days) | Relat ive remis sion rate (%) |
|---|---|---|---|---|---|---|---|---|---|---|---|
| 383 | Fluorouracil (5-Fu) | Intravenous injectio n | 70 | Once a day, for 3 days | Compound I | 6 h | Oral adminis tration | 100 | Once a day, for 3 days | 8 | 50 |
| 384 | Fluorour acil (5-Fu) | Oral adminis tration | 50 | Once a day, for 14 days | Compo und I | 8 h | Intraper itoneal injectio n | 100 | Once a day, for 14 days | 14 | Ineff ectiv e |

It can be seen from Table 5 and FIG. 5 that: the compound I, II, IV and III, and their hydrochlorides have relieving effects on diarrhea caused by the continuous administration of the chemotherapeutic agent for 7 days or less, and the relieving effects on diarrhea caused by the continuous administration of the chemotherapeutic agent for less than 7 days were significant. Compound I is ineffective to the relieving effect on diarrhea caused by the continuous administration of fluorouracil for 14 days.

**Examples 385-405: Relieving effect of oral administration of the compound as shown in Formula I on constipation caused by the continuous administration of a chemotherapeutic agent**

Balb/c mice were fed for 1 week, and then divided into groups. The experiment was divided into a control group, a chemotherapeutic agent group, and a group of the compound as shown in Formula I, each of which comprised 10 mice, to perform the administration experiment, for which the administration dosage, mode, time and frequency were shown in Table 6. The control group: being injected or intragastrically administered with the same solvent as that used in the group of the compound as shown in Formula I (the administration mode and time were shown in Table 6), and then being injected or intragastrically administered with the same solvent as that used in the group of the compound as shown in Formula I (the administration mode was shown in Table 6); the chemotherapeutic agent group: being injected or intragastrically administered with the same solvent as that used in the group of the compound as shown in Formula I (the administration mode and time were shown in Table 6), and then being injected or intragastrically administered with the chemotherapeutic agent (the type, administration mode, time and dosage were shown in Table 6); the group of the compound as shown in Formula I: being injected or intragastrically administered with the compound as shown in Formula I (the administration mode and time were shown in Table 6), and then being administered with the chemotherapeutic agent (the type, administration mode, time and dosage were shown in Table 6).

Constipation observation

The feces of mice within 3 hours were collected, which were observed for shape and weighed with an electronic balance. The feces reduction rate was calculated with reference to the method of Ji Eun Kim (Lab Anim Res. 2016 Dec; 32(4): 231-240.). Feces reduction rate (%) = (the control group - the chemotherapeutic agent group or the group of the compound as shown in Formula I)/the control group*100%

It is regarded as effective remission when the feces reduction rate of mice in the group of the compound as shown in Formula I is reduced compared with that in the chemotherapeutic agent group.

The feces amount of mice within 3 hours was observed and recorded every day. At the end of the experiment, the number of mice in the group of the compound as shown in Formula I whose feces reduction rate was lower than that of mice in the chemotherapeutic agent group was recorded. Table 6 lists various animal experiment combinations of chemotherapeutic agents and the compound as shown in Formula I, as well as the corresponding experimental results. (Relative remission rate%= the number of mice in the group of the compound as shown in Formula I which have been effectively relieved/the total number of mice in the group of the compound as shown in Formula I * 100%. The establishment rate of constipation model in the chemotherapeutic agent group was 30%-60%, that is, about 3-6 of 10 mice showed reduced feces amount during the experiment, and there were cases of individual mice death or with no changes or an increasement in the feces amount.)

Table 6: Experimental Conditions and Results of Examples 385-405

| Exa mple No. | Chemoth erapeutic agent | Admini stration mode | Dos age (mg /kg) | Freq uency | Compo und as shown in Formal a I | Administ ration time (before the chemoth erapeutic agent) | Admini stration mode | Dos age (mg /kg) | Freq uency | Experi mental cycle (days) | Rela tive remi ssion rate (%) |
|---|---|---|---|---|---|---|---|---|---|---|---|
| 385 | Fluorour acil (5-Fu) | Intraper itoneal injectio n | 100 | Once a day, for 3 days | Compo und I | 0.5 h | Intrave nous injectio n | 100 | Once a day, for 3 days | 7 | 70 |
| 386 | | | | | | 4 h | | | | | 50 |
| 387 | | | | | | 6 h | | | | | 40 |
| 388 | | | | | | 8 h | | | | | 30 |
| 389 | Fluorour acil (5-Fu) | Intraper itoneal injectio n | 100 | Once a day, for 5 days | Compo und I | 0.5 h | Intraper itoneal injectio n | 100 | Once a day, for 5 days | 9 | 60 |
| 390 | | | | | | 12 h | | | | | 30 |
| 391 | | | | | | 20 h | | | | | 10 |
| 392 | | | | | | 24 h | | | | | 10 |
| 393 | Fluorour acil (5-Fu) | Intraper itoneal injectio n | 100 | Once a day, for 5 days | Compo und I hydroc hloride | 0.5 h | Intraper itoneal injectio n | 150 | Once a day, for 5 days | 9 | 70 |
| 394 | | | | | | 4 h | | | | | 50 |
| 395 | | | | | | 12 h | | | | | 30 |
| 396 | Fluorour acil (5-Fu) | Intrave nous injectio n | 100 | Once a day, for 3 days | Compo und I | 4 h | Oral adminis tration | 100 | Once a day, for 3 days | 7 | 40 |
| 397 | Fluorour acil (5-Fu) | Intraper itoneal injectio n | 100 | Once a day, for 3 days | Compo und II | 0.5 h | Intraper itoneal injectio n | 100 | Once a day, for 3 days | 7 | 20 |
| 398 | Fluorour acil (5-Fu) | Intraper itoneal injectio n | 100 | Once a day, for 3 days | Compo und III | 2 h | Intrave nous injectio n | 100 | Once a day, for 3 days | 7 | 40 |
| 399 | Fluorour acil (5-Fu) | Intraper itoneal injectio n | 100 | Once a day, for 3 days | Compo und IV | 4 h | Oral adminis tration | 100 | Once a day, for 3 days | 7 | 50 |

(continued)

| Exa mple No. | Chemoth erapeutic agent | Admini stration mode | Dos age (mg /kg) | Freq uency | Compo und as shown in Formal a I | Administ ration time (before the chemoth erapeutic agent) | Admini stration mode | Dos age (mg /kg) | Freq uency | Experi mental cycle (days) | Rela tive remi ssion rate (%) |
|---|---|---|---|---|---|---|---|---|---|---|---|
| 400 | Fluorour acil (5-Fu) | Intraper itoneal injectio n | 100 | Once a day, for 3 days | Compo und IV hydroc hloride | 2 h | Intraper itoneal injectio n | One e a day, for 3 day s | Singl e | 7 | 50 |
| 401 | | | | | | 12 h | | | | | 30 |
| 402 | Fluorour acil (5-Fu) | Intraper itoneal injectio n | 100 | Once a day, for 7 days | Compo und I | 2 h | Intraper itoneal injectio n | 125 | Once a day, for 7 days | 10 | 10 |
| 403 | | | | | | 4 h | | | | | 10 |
| 404 | | | | | | 6 h | | | | | 10 |
| 405 | | | | | | 8 h | | | | | 0 |

It can be seen from Table 6 and FIG. 6 that: the compound I, II, IV and III, and their hydrochlorides have relieving effects on constipation caused by the continuous administration of the chemotherapeutic agent for 7 days or less, and especially have significant relieving effects on constipation caused by the continuous administration of fluorouracil for less than 7 days.

## Claims

1. A use of a compound as shown in Formula I or a pharmaceutically acceptable salt thereof in preparing a medicament, said medicament is used for preventing, relieving and/or treating chemotherapy-associated gastrointestinal side effects in a subject:

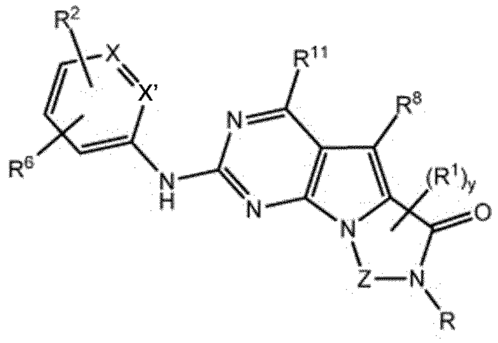

Formula I,

wherein,

Z is $-(CH_2)_X-$, wherein X is 1, 2, 3 or 4, or $-O-(CH_2)_Z-$, wherein z is 2, 3 or 4;
X and X' are each independently C or N;
R, $R^8$ and $R^{11}$ are each independently H, $C_1$-$C_3$ alkyl or haloalkyl, cycloalkyl or cycloalkyl containing one or more heteroatoms selected from N, O or S, -(alkylene)$_m$-$C_3$-$C_8$ cycloalkyl, -(alkylene)$_m$-aryl, -(alkylene)$_m$-heterocyclyl, -(alkylene)$_m$-heteroaryl, -(alkylene)$_m$-$NR^3R^4$, -(alkylene)$_m$-C(O)-$NR^3R^4$, -(alkylene)$_m$-O-$R^5$, -(alkylene)$_m$-S(O)$_n$-$R^5$, or -(alkylene)$_m$-S(O)$_n$-$NR^3R^4$, any of which may be optionally independently substituted with one or more R groups as allowed by valance, and wherein two $R^x$ groups bound to the same or adjacent atoms may optionally combine to form a ring, and wherein m is 0 or 1, n is 0, 1 or 2;
$R^1$ is independently aryl, alkyl, cycloalkyl or haloalkyl, wherein each of said alkyl, cycloalkyl and haloalkyl optionally comprises O or N heteroatoms in place of a carbon in the chain, and two $R^1$'s on adjacent ring atoms or on the same ring atom together with the ring atom(s) to which they are attached optionally form a 3-8-membered cycle;
y is 0, 1, 2, 3 or 4;
$R^2$ is -(alkylene)$_m$-heterocyclyl, -(alkylene)$_m$-heteroaryl, -(alkylene)$_m$-$NR^3R^4$, -(alkylene)$_m$-C(O)-$NR^3R^4$, -(alkylene)$_m$-C(O)-O-alkyl; -(alkylene)$_m$-O-$R^5$, -(alkylene)$_m$-S(O)$_n$-$R^5$ or -(alkylene)$_m$-S(O)$_n$-$NR^3R^4$, any of which may be optionally independently substituted with one or more $R^x$ groups as allowed by valance, and wherein two $R^x$ groups bound to the same or adjacent atoms may optionally combine to form a ring, and wherein m is 0 or 1, n is 0, 1 or 2;
$R^3$ and $R^4$ at each occurrence are independently:

(i) hydrogen or
(ii) alkyl, cycloalkyl, heterocyclyl, aryl, heteroaryl, cycloalkylalkyl, heterocycloalkyl, arylalkyl or heteroarylalkyl, any of which may be optionally independently substituted with one or more $R^x$ groups as allowed by valance, and wherein two $R^x$ groups bound to the same or adjacent atoms may optionally combine to form a ring; or $R^3$ and $R^4$ together with the nitrogen atom to which they are attached may combine to form a heterocyclic ring optionally independently substituted with one or more $R^x$ groups as allowed by valance, and wherein two $R^x$ groups bound to the same or adjacent atoms may optionally combine to form a ring;

$R^5$ and $R^{5*}$ at each occurrence are:

(i) hydrogen or

(ii) alkyl, alkenyl, alkynyl, cycloalkyl, heterocyclyl, aryl, heteroaryl, cycloalkylalkyl, heterocycloalkyl, arylalkyl or heteroarylalkyl, any of which may be optionally independently substituted with one or more $R^x$ groups as allowed by valance;

$R^x$ at each occurrence is independently halo, cyano, nitro, oxo, alkyl, haloalkyl, alkenyl, alkynyl, cycloalkyl, cycloalkenyl, heterocyclyl, aryl, heteroaryl, arylalkyl, heteroarylalkyl, cycloalkylalkyl, heterocycloalkyl, -(alkylene)$_m$-OR$^5$, -(alkylene)$_m$-O-alkylene-OR$^5$, -(alkylene)$_m$-S(O)$_n$-R$^5$, -(alkylene)$_m$-NR$^3$R$^4$, -(alkylene)$_m$-CN, -(alkylene)$_m$-C(O)-R$^5$, -(alkylene)$_m$-C(S)-R$^5$, -(alkylene)$_m$-C(O)-OR$^5$, -(alkylene)$_m$-O-C(O)-R$^5$, -(alkylene)$_m$-C(S)-OR$^5$, -(alkylene)$_m$-C(O)-(alkylene)$_m$-NR$^3$R$^4$, -(alkylene)$_m$-C(S)-NR$^3$R$^4$, -(alkylene)$_m$-N(R$^3$)-C(O)-NR$^3$R$^4$, -(alkylene)$_m$-N(R$^3$)-C(S)-NR$^3$R$^4$, -(alkylene)$_m$-N(R$^3$)-C(O)-R$^5$, -(alkylene)$_m$-N(R$^3$)-C(S)-R$^5$, -(alkylene)$_m$-O-C(O)-NR$^3$R$^4$, -(alkylene)$_m$-O-C(S)-NR$^3$R$^4$, -(alkylene)$_m$-SO$_2$-NR$^3$R$^4$, -(alkylene)$_m$-N(R$^3$)-SO$_2$-R$^5$, -(alkylene)$_m$-N(R$^3$)-SO$_2$-NR$^3$R$^4$, -(alkylene)$_m$-N(R$^3$)-C(O)-OR$^5$, -(alkylene)$_m$-N(R$^3$)-C(S)-OR$^5$ or -(alkylene)$_m$-N(R$^3$)-SO$_2$-R$^5$; wherein:
said alkyl, haloalkyl, alkenyl, alkynyl, cycloalkyl, cycloalkenyl, heterocyclyl, aryl, heteroaryl, arylalkyl, heteroarylalkyl, cycloalkylalkyl and heterocycloalkyl may be further independently substituted with one or more of the following groups:
-(alkylene)$_m$-CN, -(alkylene)$_m$-OR$^{5*}$, -(alkylene)$_m$-S(O)$_n$-R$^{5*}$, -(alkylene)$_m$-NR$^{3*}$R$^{4*}$, -(alkylene)$_m$-C(O)-R$^{5*}$, -(alkylene)$_m$-C( = S)R$^{5*}$, -(alkylene)$_m$-C( = O)OR$^{5*}$, -(alkylene)$_m$-OC( = O)R$^{5*}$, -(alkylene)$_m$-C(S)-OR$^{5*}$, -(alkylene)$_m$-C(O)-NR$^{3*}$R$^{4*}$, -(alkylene)$_m$-C(S)-NR$^{3*}$R$^{4*}$, -(alkylene)$_m$-N(R$^{3*}$)-C(O)-NR$^{3*}$R$^{4*}$, -(alkylene)$_m$-N(R$^{3*}$)-C(S)-NR$^{3*}$R$^{4*}$, -(alkylene)$_m$-N(R$^{3*}$)-C(O)-R$^{5*}$, -(alkylene)$_m$-N(R$^{3*}$)-C(S)-R5*, -(alkylene)$_m$-O-C(O)-NR$^{3*}$R$^{4*}$, -(alkylene)$_m$-O-C(S)-NR$^{3*}$R$^{4*}$, -(alkylene)$_m$-SO$_2$-NR$^{3*}$R$^{4*}$, -(alkylene)$_m$-N(R$^{3*}$)-SO$_2$-R$^{5*}$, -(alkylene)$_m$-N(R$^{3*}$)-SO$_2$-NR$^{3*}$R$^{4*}$, -(alkylene)$_m$-N(R$^{3*}$)-C(O)-OR$^{5*}$, -(alkylene)$_m$-N(R$^{3*}$)-C(S)-OR$^{5*}$ or -(alkylene)$_m$-N(R$^{3*}$)-SO$_2$-R$^{5*}$,
n is 0, 1 or 2, and m is 0 or 1;
$R^{3*}$ and $R^{4*}$ at each occurrence are independently:

(i) hydrogen or
(ii) alkyl, alkenyl, alkynyl, cycloalkyl, heterocyclyl, aryl, heteroaryl, cycloalkylalkyl, heterocycloalkyl, arylalkyl or heteroarylalkyl, any of which may be optionally independently substituted with one or more $R^x$ groups as allowed by valance; or $R^{3*}$ and $R^{4*}$ together with the nitrogen atom to which they are attached may combine to form a heterocyclic ring optionally independently substituted with one or more $R^x$ groups as allowed by valance; and

$R^6$ is H or lower alkyl, -(alkylene)$_m$-heterocyclyl, -(alkylene)$_m$-heteroaryl, -(alkylene)$_m$-NR$^3$R$^4$, -(alkylene)$_m$-C(O)-NR$^3$R$^4$; -(alkylene)$_m$-O-R$^5$, -(alkylene)$_m$-S(O)$_n$-R$^5$ or -(alkylene)$_m$-S(O)$_n$-NR$^3$R$^4$, any of which may be optionally independently substituted with one or more $R^x$ groups as allowed by valance, and wherein two $R^x$ groups bound to the same or adjacent atoms may optionally combine to form a ring.

**2.** The use of claim 1, wherein $R^6$ is absent.

**3.** The use of any one of claims 1-2, wherein said $R^x$ is not further substituted.

**4.** The use of any one of claims 1-3, wherein said $R^8$ is hydrogen or $C_1$-$C_3$ alkyl.

**5.** The use of any one of claims 1-4, wherein said $R^{11}$ is hydrogen or $C_1$-$C_3$ alkyl.

**6.** The use of any one of claims 1-5, wherein said compound comprises a compound as shown in Formula Ia:

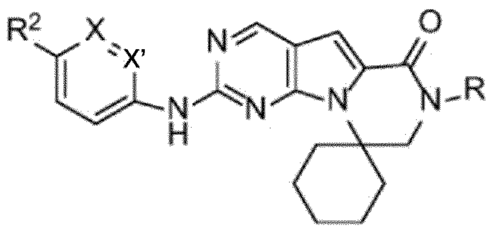

Formula Ia.

**7.** The use of any one of claims 1-6, wherein said X is C.

**8.** The use of any one of claims 1-7, wherein said X' is N.

**9.** The use of any one of claims 1-8, wherein said R is hydrogen or $C_1$-$C_3$ alkyl.

**10.** The use of any one of claims 1-9, wherein said $R^2$ is -(alkylene)$_m$-heterocyclyl, -(alkylene)$_m$-$NR^3R^4$, -(alkylene)$_m$-C(O)-$NR^3R^4$, -(alkylene)$_m$-C(O)-O-alkyl or -(alkylene)$_m$-$OR^5$, any of which may be optionally independently substituted with one or more $R^x$ groups as allowed by valance, and wherein two $R^x$ groups bound to the same or adjacent atoms may optionally combine to form a ring, and wherein m is 0 or 1.

**11.** The use of any one of claims 1-10, wherein said $R^2$ is -(alkylene)$_m$-heterocyclyl, -(alkylene)$_m$-$NR^3R^4$, -(alkylene)$_m$-C(O)-$NR^3R^4$, -(alkylene)$_m$-C(O)-O-alkyl or -(alkylene)$_m$-$OR^5$, which is not further substituted.

**12.** The use of any one of claims 1-11, wherein m in said $R^2$ is 1.

**13.** The use of any one of claims 1-12, wherein alkylene in said $R^2$ is methylene.

**14.** The use of any one of claims 1-13, wherein said $R^2$ is

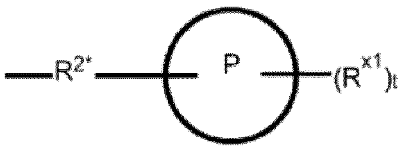

,

wherein, $R^{2*}$ is a bond, alkylene, -(alkylene)$_m$-O-(alkylene)$_m$-, -(alkylene)$_m$-C(O)-(alkylene)$_m$-, -(alkylene)$_m$-S(O)$_2$-(alkylene)$_m$- and -(alkylene)$_m$-NH-(alkylene)$_m$-, wherein each m is independently 0 or 1;

P is a 4- to 8-membered mono- or bicyclic saturated heterocyclyl group;
each $R^{x1}$ is independently -(alkylene)$_m$-(C(O))$_m$-(alkylene)$_m$-(N($R^N$))$_m$-(alkyl)$_m$, wherein each m is independently 0 or 1 provided at least one m is 1; -(C(O))-O-alkyl; -(alkylene)$_m$-cycloalkyl wherein m is 0 or 1; -N($R^N$)-cycloalkyl; -C(O)-cycloalkyl; -(alkylene)$_m$-heterocyclyl wherein m is 0 or 1; or -N($R^N$)-heterocyclyl; -C(O)-heterocyclyl; -S(O)$_2$-(alkylene)$_m$ wherein m is 1 or 2, wherein:

$R^N$ is H, $C_1$ to $C_4$ alkyl or $C_1$ to $C_6$ heteroalkyl, and
wherein two $R^{x1}$ can, together with the atoms to which they attach on P, which may be the same atom, form a ring; and
t is 0, 1 or 2.

**15.** The use of claim 14, wherein said each $R^{x1}$ is optionally substituted by unsubstituted alkyl, halogen or hydroxyl.

**16.** The use of any one of claims 14-15, wherein $R^{x1}$ is hydrogen or unsubstituted $C_1$-$C_4$ alkyl.

**17.** The use of any one of claims 14-16, wherein at least one of said $R^{x1}$ is -(alkylene)$_m$-heterocyclyl, wherein m is 0 or 1.

**18.** The use of any one of claims 1-17, wherein said $R^2$ is

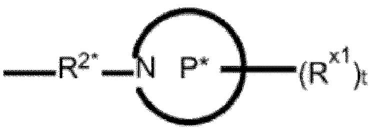

,

wherein P* is a 4- to 8-membered mono- or bicyclic saturated heterocyclyl group.

**19.** The use of any one of claims 1-18, wherein said $R^2$ is

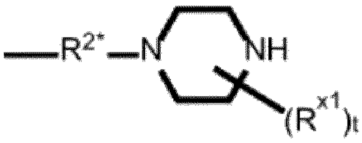
.

**20.** The use of any one of claims 1-19, wherein said $R^2$ is

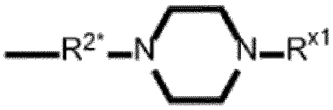
.

**21.** The use of any one of claims 1-20, wherein said $R^2$ is

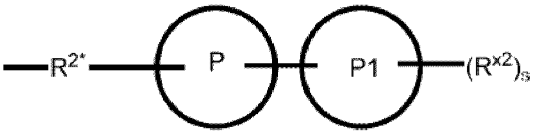
,

wherein, $R^{2*}$ is a bond, alkylene, -(alkylene)$_m$-O-(alkylene)$_m$-, -(alkylene)$_m$-C(O)-(alkylene)$_m$-, -(alkylene)$_m$-S(O)$_2$-(alkylene)$_m$- and -(alkylene)$_m$-NH-(alkylene)$_m$-, wherein each m is independently 0 or 1;

P is a 4- to 8-membered mono- or bicyclic saturated heterocyclyl group;
P1 is a 4- to 6-membered monocyclic saturated heterocyclyl group; and
each $R^{x2}$ is independently hydrogen or alkyl; and s is 0, 1 or 2.

**22.** The use of any one of claims 1-21, wherein said $R^2$ is

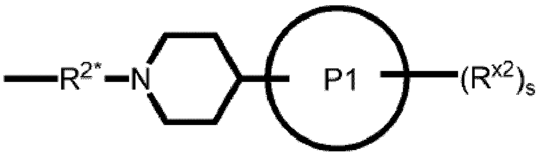
.

**23.** The use of any one of claims 21-22, wherein P1 in said $R^2$ comprises at least one nitrogen.

**24.** The use of any one of claims 14-23, wherein any alkylene in said $R^{2*}$ is not further substituted.

**25.** The use of any one of claims 14-24, wherein said $R^{2*}$ is a bond.

**26.** The use of any one of claims 14-25, wherein said $R^{x1}$ is methyl or propyl.

**27.** The use of any one of claims 1-26, wherein said $R^2$ is

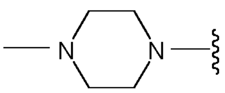
,

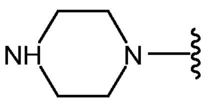
,
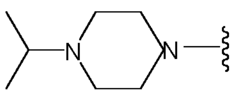
or
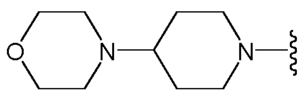
.

**28.** The use of any one of claims 1-27, wherein said compound is selected from the following group:

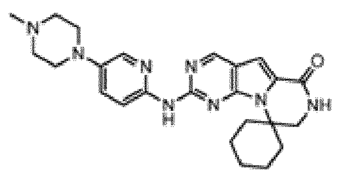

Compound I

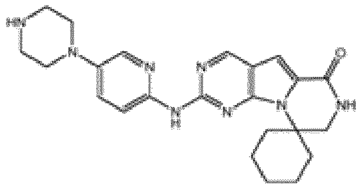

Compound II

,

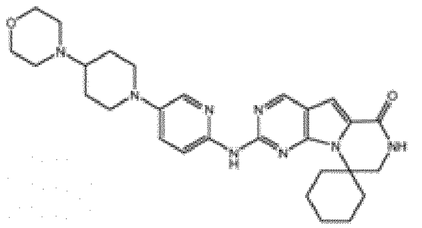

Compound III

and

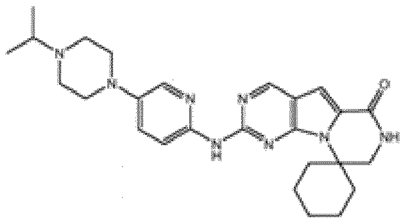

Compound IV

.

**29.** The use of any one of claims 1-28, wherein said compound is

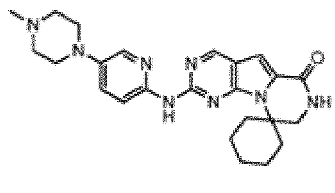

Compound I

,

and its hydrochloride form.

**30.** The use of any one of claims 1-29, wherein said compound is

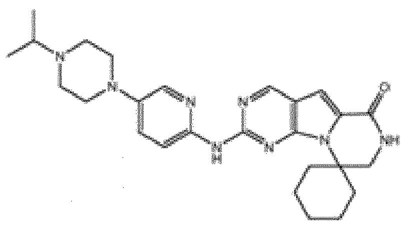

Compound IV

,

and its hydrochloride form.

**31.** The use of any one of claims 1-30, wherein said chemotherapy comprises administration of a chemotherapeutic agent.

**32.** The use of any one of claims 1-31, wherein said chemotherapeutic agent is a cytotoxic agent.

**33.** The use of any one of claims 1-32, wherein said chemotherapeutic agent is selected from one or more of the following group: a DNA synthesis inhibitor, a RNA synthesis inhibitor, a protein synthesis inhibitor, a cell division inhibitor, a DNA base analogue, a topoisomerase inhibitor and/or a telomerase synthesis inhibitor.

**34.** The use of any one of claims 1-33, wherein said chemotherapy is administered in combination with one or more other therapies.

**35.** The use of any one of claims 1-34, wherein said chemotherapeutic agent is selected from fluorouracil, oxaliplatin, irinotecan, topotecan, etoposide, cisplatin, docetaxel, gemcitabine, carboplatin, methotrexate, doxorubicin, cytarabine, vinorelbine and capecitabine, and a combination thereof.

**36.** The use of any one of claims 1-35, wherein said chemotherapeutic agent is selected from 5-fluorouracil, irinotecan and oxaliplatin, and a combination thereof.

**37.** The use of any one of claims 1-36, wherein said chemotherapy-associated gastrointestinal side effects comprise gastrointestinal side effects caused by said chemotherapeutic agent.

**38.** The use of any one of claims 1-37, wherein said gastrointestinal side effects occur or deteriorate after the administration of said chemotherapeutic agent.

**39.** The use of any one of claims 1-38, wherein said gastrointestinal side effects occur or deteriorate about 1 hr, about 2 hrs, about 3 hrs, about 4 hrs, about 5 hrs, about 6 hrs, about 7 hrs, about 8 hrs, about 9 hrs, about 10 hrs, about 11 hrs, about 12 hrs, about 1 day, about 2 days, about 4 days, about 7 days, about 2 weeks, about 3 weeks, about 1 month, about 2 months or longer after the administration of said chemotherapeutic agent.

**40.** The use of any one of claims 1-39, wherein said gastrointestinal side effects comprise gastric mucosal injury diseases and/or intestinal mucosal injury diseases.

**41.** The use of any one of claims 1-40, wherein said gastrointestinal side effects comprise diarrhea, abdominal pain, nausea, vomiting, mucositis, loss of appetite, gastric ulcer, gastritis, constipation, enteritis, intestinal perforation, intestinal bleeding, ulcer and/or intestinal necrosis.

**42.** The use of any one of claims 1-41, wherein said gastrointestinal side effects comprise diarrhea, constipation and/or enteritis.

**43.** The use of any one of claims 1-42, wherein the severity of said gastrointestinal side effects is Grade 1 or above, Grade 2 or above, Grade 3 or above, Grade 4 or above, or Grade 5, as evaluated in accordance with NCI-CTCAE.

**44.** The use of any one of claims 1-43, wherein said subject comprises a cancer patient.

**45.** The use of any one of claims 1-44, wherein said medicament does not substantially affect the therapeutic effect of said chemotherapeutic agent.

**46.** The use of any one of claims 1-45, wherein the medicament is administered about 0.5 hrs, about 1 hr, about 2 hrs, about 3 hrs, about 4 hrs, about 5 hrs, about 6 hrs, about 7 hrs, about 8 hrs, about 9 hrs, about 10 hrs, about 11 hrs, about 12 hrs, about 13 hrs, about 14 hrs, about 15 hrs, about 16 hrs, about 17 hrs, about 18 hrs, about 19 hrs, about 20 hrs or longer before the administration of said chemotherapy.

**47.** The use of any one of claims 1-46, wherein the medicament is administered about 0.5~12 hrs before the administration of said chemotherapy.

**48.** The use of any one of claims 1-47, wherein said medicament is prepared for oral administration.

**49.** The use of any one of claims 1-48, wherein said medicament is prepared for injection administration.

**50.** The use of claim 49, wherein said medicament is prepared for administration through subcutaneous injection, intramuscular injection, intraperitoneal injection and/or intravenous injection.

**51.** The use of any one of claims 1-50, wherein said medicament further comprises one or more other active ingredients.

**52.** A method of preventing, relieving and/or treating chemotherapy-associated gastrointestinal side effects, comprising administering to a subject in need thereof said compound as shown in Formula I as claimed in the use of any one

of claims 1-51 or a pharmaceutically acceptable salt thereof.

**53.** The compound as shown in Formula I as claimed in the use of any one of claims 1-51 or a pharmaceutically acceptable salt thereof, which is used for preventing, relieving and/or treating chemotherapy-associated gastrointestinal side effects in a subject.

**54.** A pharmaceutical composition, comprising said compound as shown in Formula I as claimed in the use of any one of claims 1-51 or a pharmaceutically acceptable salt thereof and a chemotherapeutic agent.

**55.** A kit, comprising said compound as shown in Formula I as claimed in the use of any one of claims 1-51 or a pharmaceutically acceptable salt thereof and a chemotherapeutic agent.

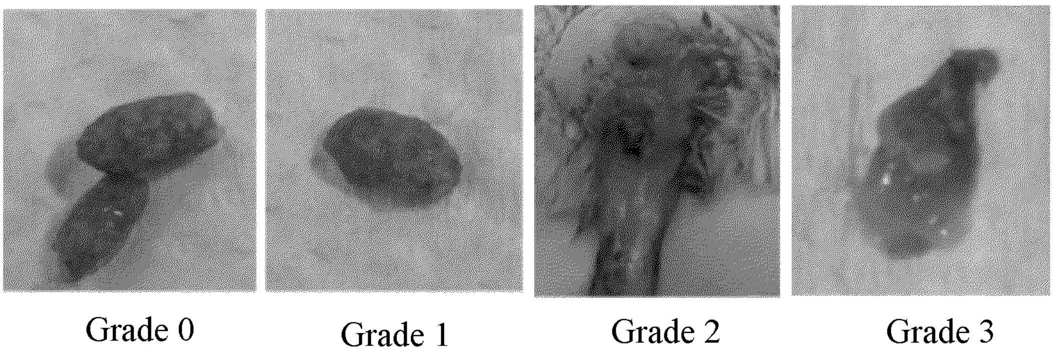


FIG. 1

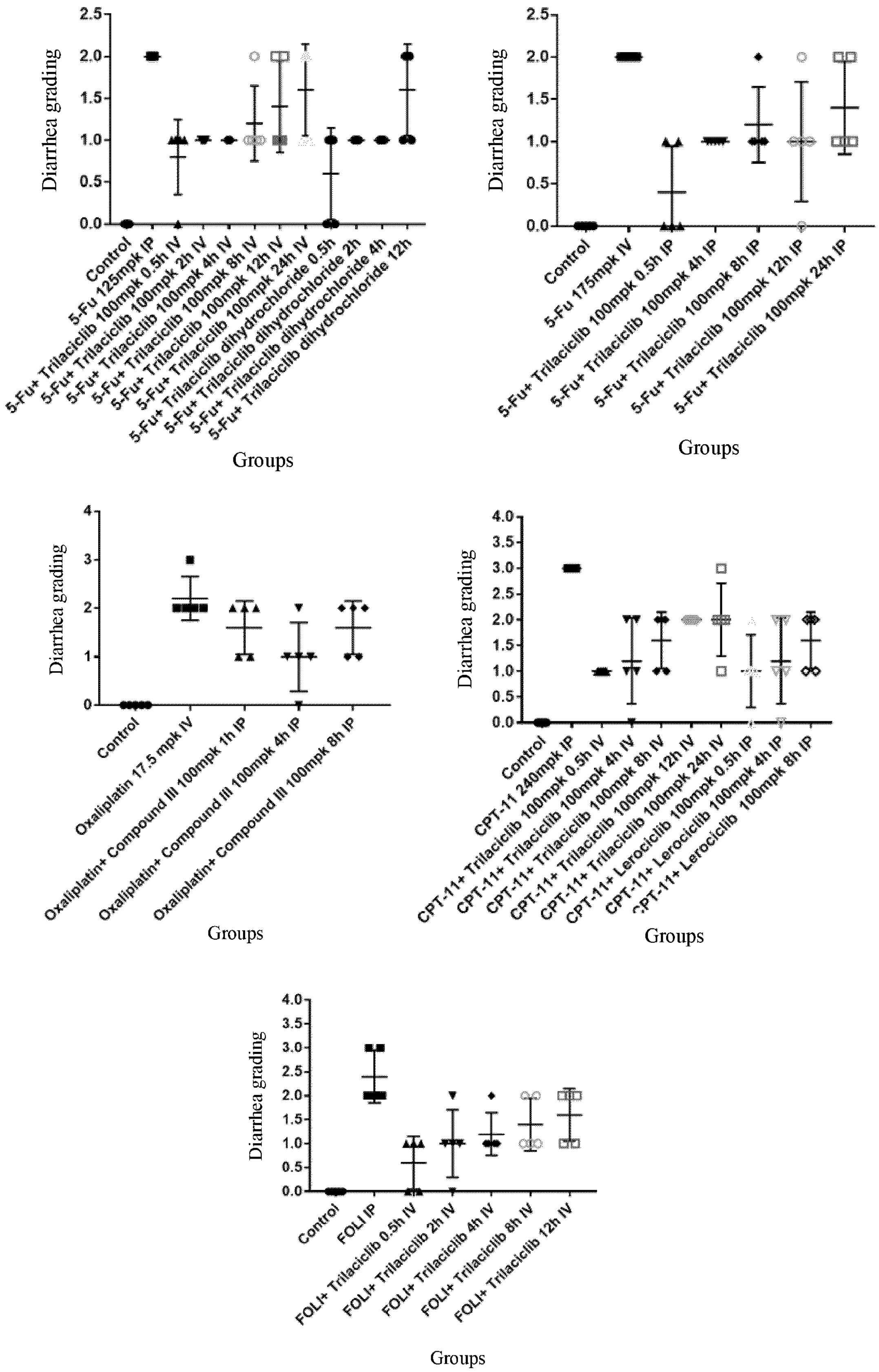
Diarrhea grading
Groups
Control
5-Fu 125mpk IP
5-Fu+ Trilaciclib 100mpk 0.5h IV
5-Fu+ Trilaciclib 100mpk 2h IV
5-Fu+ Trilaciclib 100mpk 4h IV
5-Fu+ Trilaciclib 100mpk 8h IV
5-Fu+ Trilaciclib 100mpk 12h IV
5-Fu+ Trilaciclib 100mpk 24h IV
5-Fu+ Trilaciclib dihydrochloride 0.5h
5-Fu+ Trilaciclib dihydrochloride 2h
5-Fu+ Trilaciclib dihydrochloride 4h
5-Fu+ Trilaciclib dihydrochloride 12h
Diarrhea grading
Groups
Control
5-Fu 175mpk IV
5-Fu+ Trilaciclib 100mpk 0.5h IP
5-Fu+ Trilaciclib 100mpk 4h IP
5-Fu+ Trilaciclib 100mpk 8h IP
5-Fu+ Trilaciclib 100mpk 12h IP
5-Fu+ Trilaciclib 100mpk 24h IP
Diarrhea grading
Groups
Control
Oxaliplatin 17.5 mpk IV
Oxaliplatin+ Compound III 100mpk 1h IP
Oxaliplatin+ Compound III 100mpk 4h IP
Oxaliplatin+ Compound III 100mpk 8h IP
Diarrhea grading
Groups
Control
CPT-11 240mpk IP
CPT-11+ Trilaciclib 100mpk 0.5h IV
CPT-11+ Trilaciclib 100mpk 4h IV
CPT-11+ Trilaciclib 100mpk 8h IV
CPT-11+ Trilaciclib 100mpk 12h IV
CPT-11+ Trilaciclib 100mpk 24h IV
CPT-11+ Lerociclib 100mpk 0.5h IP
CPT-11+ Lerociclib 100mpk 4h IP
CPT-11+ Lerociclib 100mpk 8h IP
Diarrhea grading
Groups
Control
FOLI IP
FOLI+ Trilaciclib 0.5h IV
FOLI+ Trilaciclib 2h IV
FOLI+ Trilaciclib 4h IV
FOLI+ Trilaciclib 8h IV
FOLI+ Trilaciclib 12h IV

**FIG. 2**

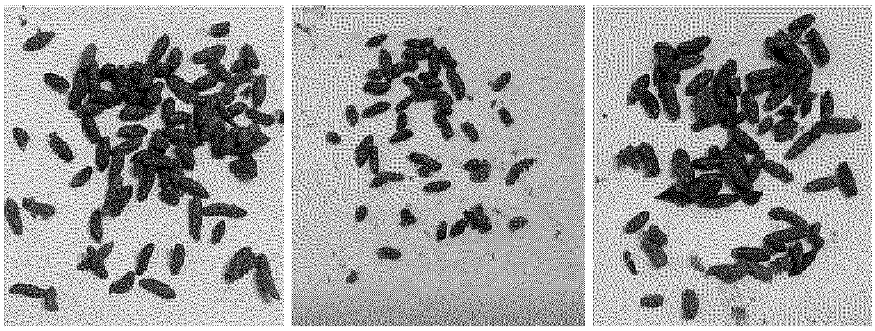
Control group
Chemotherapeutic agent group
CDK inhibitor group

FIG. 3

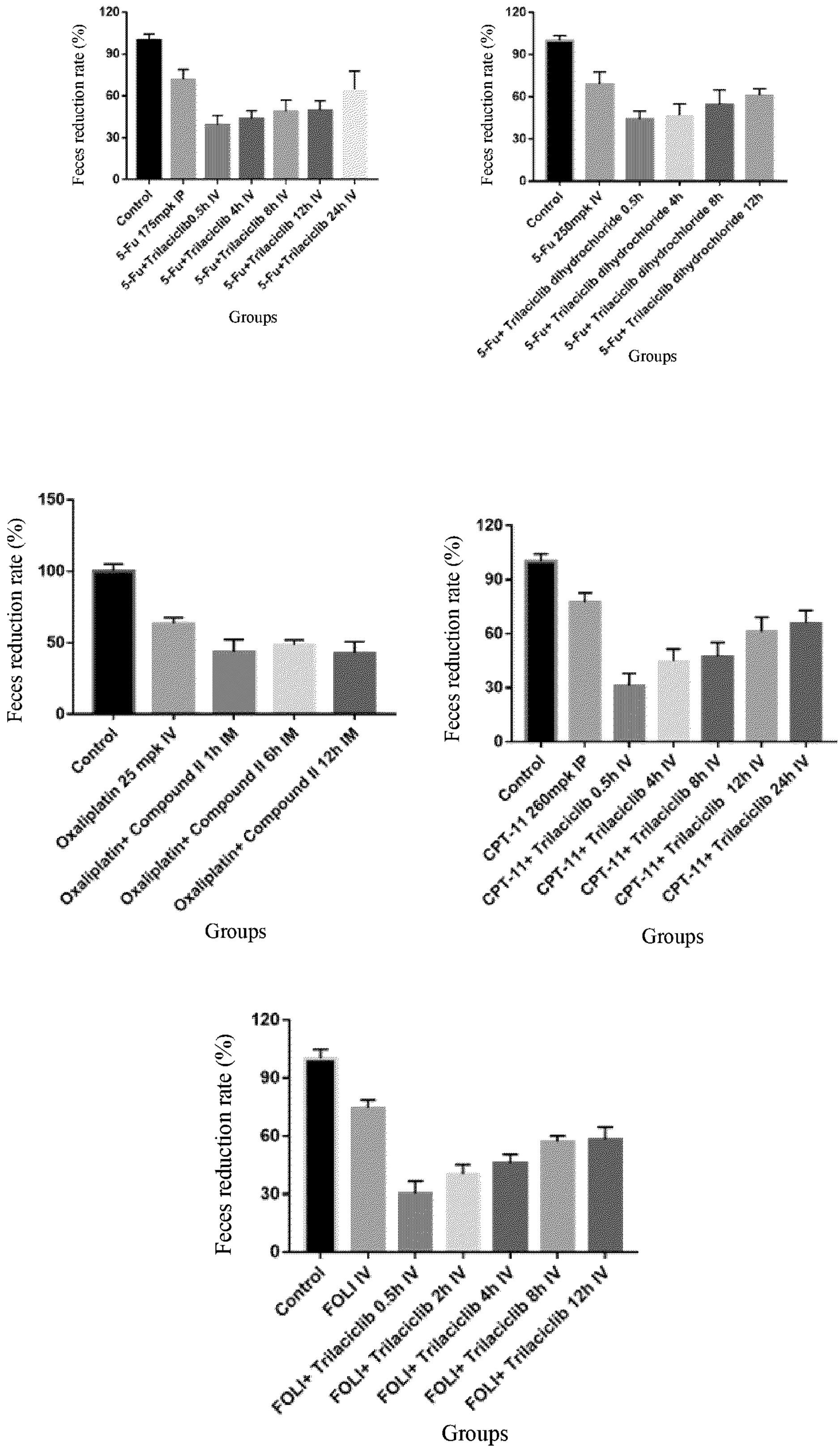
Feces reduction rate (%)
Groups
Control
5-Fu 175mpk IP
5-Fu+Trilaciclib0.5h IV
5-Fu+Trilaciclib 4h IV
5-Fu+Trilaciclib 8h IV
5-Fu+Trilaciclib 12h IV
5-Fu+Trilaciclib 24h IV
5-Fu 250mpk IV
5-Fu+ Trilaciclib dihydrochloride 0.5h
5-Fu+ Trilaciclib dihydrochloride 4h
5-Fu+ Trilaciclib dihydrochloride 8h
5-Fu+ Trilaciclib dihydrochloride 12h
Oxaliplatin 25 mpk IV
Oxaliplatin+ Compound II 1h IM
Oxaliplatin+ Compound II 6h IM
Oxaliplatin+ Compound II 12h IM
CPT-11 260mpk IP
CPT-11+ Trilaciclib 0.5h IV
CPT-11+ Trilaciclib 4h IV
CPT-11+ Trilaciclib 8h IV
CPT-11+ Trilaciclib 12h IV
CPT-11+ Trilaciclib 24h IV
FOLI IV
FOLI+ Trilaciclib 0.5h IV
FOLI+ Trilaciclib 2h IV
FOLI+ Trilaciclib 4h IV
FOLI+ Trilaciclib 8h IV
FOLI+ Trilaciclib 12h IV

FIG. 4

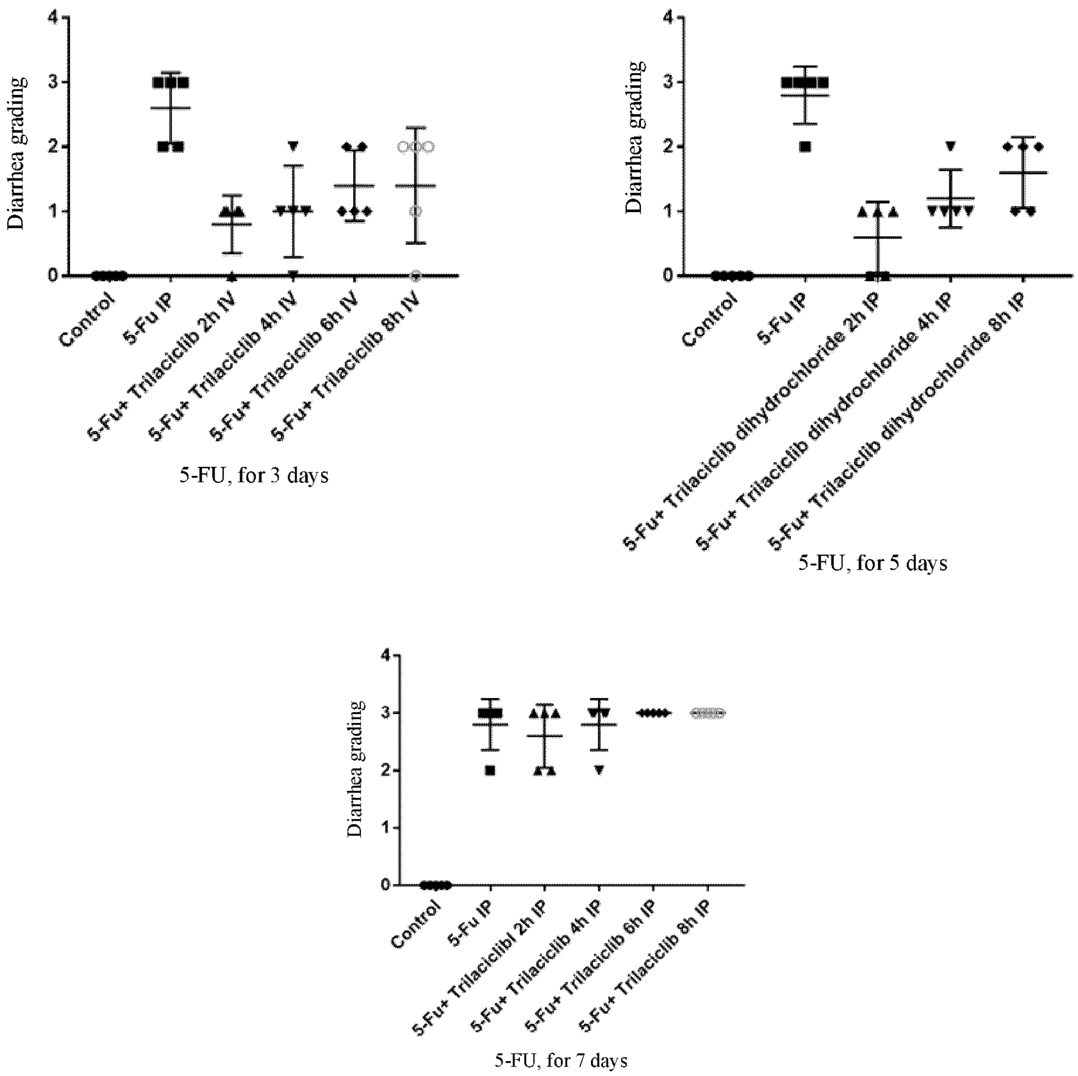
Diarrhea grading
4
3
2
1
0
Control
5-Fu IP
5-Fu+ Trilaciclib 2h IV
5-Fu+ Trilaciclib 4h IV
5-Fu+ Trilaciclib 6h IV
5-Fu+ Trilaciclib 8h IV
5-FU, for 3 days
Diarrhea grading
4
3
2
1
0
Control
5-Fu IP
5-Fu+ Trilaciclib dihydrochloride 2h IP
5-Fu+ Trilaciclib dihydrochloride 4h IP
5-Fu+ Trilaciclib dihydrochloride 8h IP
5-FU, for 5 days
Diarrhea grading
4
3
2
1
0
Control
5-Fu IP
5-Fu+ Trilaciclibl 2h IP
5-Fu+ Trilaciclib 4h IP
5-Fu+ Trilaciclib 6h IP
5-Fu+ Trilaciclib 8h IP
5-FU, for 7 days

FIG. 5

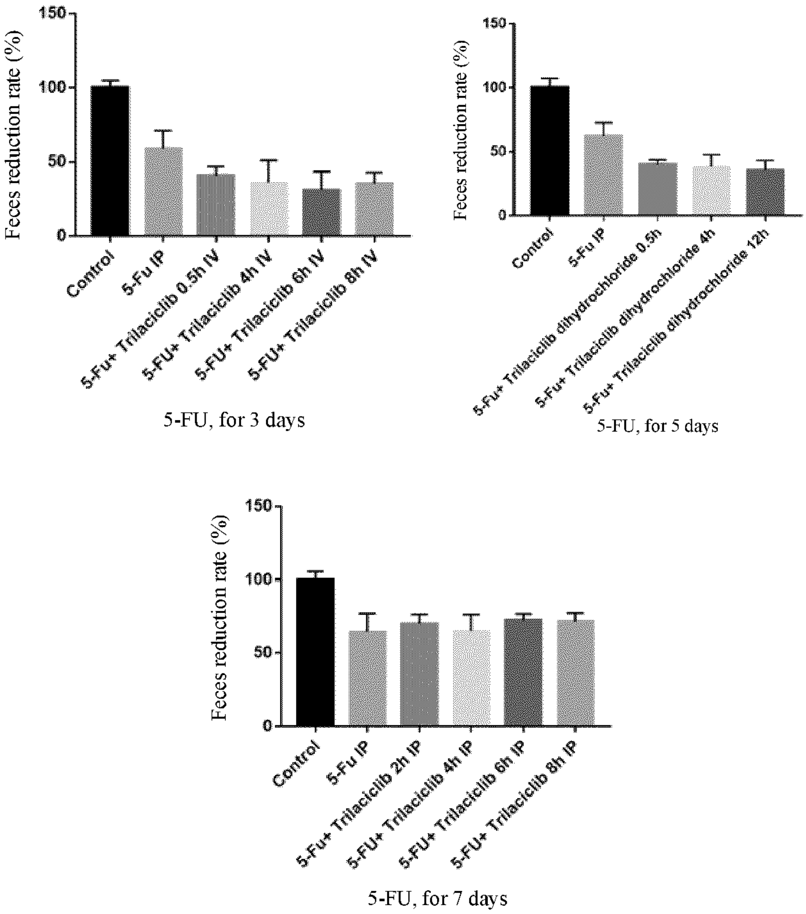
Feces reduction rate (%)
150
100
50
0
Control
5-Fu IP
5-Fu+ Trilaciclib 0.5h IV
5-FU+ Trilaciclib 4h IV
5-FU+ Trilaciclib 6h IV
5-FU+ Trilaciclib 8h IV
5-FU, for 3 days
Feces reduction rate (%)
150
100
50
0
Control
5-Fu IP
5-Fu+ Trilaciclib dihydrochloride 0.5h
5-Fu+ Trilaciclib dihydrochloride 4h
5-Fu+ Trilaciclib dihydrochloride 12h
5-FU, for 5 days
Feces reduction rate (%)
150
100
50
0
Control
5-Fu IP
5-Fu+ Trilaciclib 2h IP
5-FU+ Trilaciclib 4h IP
5-FU+ Trilaciclib 6h IP
5-FU+ Trilaciclib 8h IP
5-FU, for 7 days

**FIG. 6**

**INTERNATIONAL SEARCH REPORT**

International application No.
**PCT/CN2022/097793**

**A. CLASSIFICATION OF SUBJECT MATTER**

A61K 31/495(2006.01)i; A61K 31/519(2006.01)i; C07D 487/14(2006.01)i; A61P 1/00(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; C07D; A61P

Documentation searched other than minimum documentation to the extent that such documents are included in the fields searched

Electronic data base consulted during the international search (name of data base and, where practicable, search terms used)

CNABS; CNTXT; DWPI; SIPOABS; STNext; CNKI: 上海岸阔医药科技; 李文晰; 化疗; 胃肠道; 呕吐; 腹泻; 恶心; 结构检索; structural search; CD4/6; Trilaciclib; Chemotherapy; Gastrointestinal; Vomiting; Diarrhea; Nausea

**C. DOCUMENTS CONSIDERED TO BE RELEVANT**

| Category* | Citation of document, with indication, where appropriate, of the relevant passages | Relevant to claim No. |
|---|---|---|
| X | WEISS, J. et al. "Effects of Trilaciclib on Chemotherapy-Induced Myelosuppression and Patient-Reported Outcomes in Patients with Extensive-Stage Small Cell Lung Cancer: Pooled Results from Three Phase II Randomized, Double-Blind, Placebo-Controlled Studies;" *Clinical Lung Cancer;,* Vol. 22, No. 5, 26 March 2021 (2021-03-26), pages 449-460, abstract, and table 3 | 1-20, 27-29, 31-35, 37-38, 40-45, 47, 49-50, 52-53 |
| Y | WEISS, J. et al. "Effects of Trilaciclib on Chemotherapy-Induced Myelosuppression and Patient-Reported Outcomes in Patients with Extensive-Stage Small Cell Lung Cancer: Pooled Results from Three Phase II Randomized, Double-Blind, Placebo-Controlled Studies;" *Clinical Lung Cancer;,* Vol. 22, No. 5, 26 March 2021 (2021-03-26), pages 449-460, abstract, and table 3 | 1-55 |
| Y | WO 2016040858 A1 (G1 THERAPEUTICS, INC.) 17 March 2016 (2016-03-17) claims 1 and 2 | 1-55 |
| Y | CN 110325191 A (G1 THERAPEUTICS, INC.) 11 October 2019 (2019-10-11) claims 1-29, and description paragraph 0024 | 1-55 |
| A | WO 2020257536 A1 (G1 THERAPEUTICS, INC.) 24 December 2020 (2020-12-24) entire document | 1-55 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 August 2022** | **29 August 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/097793**

**C. DOCUMENTS CONSIDERED TO BE RELEVANT**

| Category* | Citation of document, with indication, where appropriate, of the relevant passages | Relevant to claim No. |
|---|---|---|
| A | TAN, A. R. et al. "Trilaciclib plus chemotherapy versus chemotherapy alone in patients with metastatic triple-negative breast cancer: a multicentre, randomised, open-label, phase 2 trial;" *THE LANCET Oncology;*, Vol. 20, No. 11, 28 September 2019 (2019-09-28), pages 1587-1601, and abstract | 1-55 |

Form PCT/ISA/210 (second sheet) (January 2015)

**INTERNATIONAL SEARCH REPORT**

International application No.
**PCT/CN2022/097793**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **52**
because they relate to subject matter not required to be searched by this Authority, namely:

[1] Claim 52 relates to a method for preventing, alleviating and/or treating chemotherapy-related gastrointestinal side effects, and does not comply with PCT Rule 39.1(iv). A search was conducted on the basis that the title of the subject matter is a use of a compound represented by formula I or a pharmaceutically acceptable salt thereof in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/097793**

| Patent document cited in search report | | | Publication date (day/month/year) | Patent family member(s) | | | Publication date (day/month/year) |
|---|---|---|---|---|---|---|---|
| WO | 2016040858 | A1 | 17 March 2016 | US | 2017182043 | A1 | 29 June 2017 |
| | | | | EP | 3191098 | A1 | 19 July 2017 |
| | | | | US | 2019151311 | A1 | 23 May 2019 |
| CN | 110325191 | A | 11 October 2019 | WO | 2018156812 | A1 | 30 August 2018 |
| | | | | EP | 3585389 | A1 | 01 January 2020 |
| WO | 2020257536 | A1 | 24 December 2020 | EP | 3986410 | A1 | 27 April 2022 |
| | | | | AU | 2020296087 | A1 | 27 January 2022 |
| | | | | CN | 114222577 | A | 22 March 2022 |
| | | | | CA | 3143339 | A1 | 24 December 2020 |
| | | | | TW | 202114684 | A | 16 April 2021 |
| | | | | KR | 20220024540 | A | 03 March 2022 |
| | | | | US | 2022175787 | A1 | 09 June 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **AKINOBU KURITA.** *Cancer Chemother Pharmacol,* 2000, vol. 46, 211-20
- **JI EUN KIM.** *Lab Anim Res.,* December 2016, vol. 32 (4), 231-240